# EUROPÄISCHE PATENTSCHRIFT

(11) **EP 2 066 705 B1**
(45) Veröffentlichungstag und Bekanntmachung des Hinweises auf die Patenterteilung: **25.05.2011**
(21) Anmeldenummer: 07803492.3
(22) Anmeldetag: 14.09.2007
(51) Int. Cl.: C08F 4/38, C08F 2/00, C08F 8/44, C08F 226/00, A61K 9/06, A61K 47/32, A61K 8/04, A61K 8/81, A61Q 5/00, A61Q 5/06

(54) **KATIONISCHE POLYMERE ALS VERDICKER FÜR WÄSSRIGE UND ALKOHOLISCHE ZUSAMMENSETZUNGEN**
CATIONIC POLYMERS AS THICKENERS FOR AQUEOUS AND ALCOHOLIC COMPOSITIONS
POLYMÈRES CATIONIQUES SERVANT D'ÉPAISSISSANTS POUR DES COMPOSITIONS AQUEUSES ET ALCOOLIQUES

(30) Priorität: 21.09.2006 EP 06121075
(43) Veröffentlichungstag der Anmeldung: 10.06.2009
(73) Patentinhaber: BASF SE, 67056 Ludwigshafen (DE)
(72) Erfinder: GARCIA CASTRO, Ivette, 67067 Ludwigshafen (DE); NGUYEN KIM, Son, 69502 Hemsbach (DE); JAHNEL, Wolfgang, 76756 Bellheim (DE); LAUBENDER, Matthias, 67105 Schifferstadt (DE); WERNER, Rolf, 67063 Ludwigshafen (DE)
(86) Internationale Anmeldenummer: PCT/EP2007/059692
(87) Internationale Veröffentlichungsnummer: WO 2008/034767

(56) Entgegenhaltungen:
- US-A- 4 058 491
- HF MARK, NM BIKALES, CG OVERBERGER, G MENGES: ENCYCLOPEDIA OF POLYMER SCIENCE AND ENGINEERING, EDITION 2, Bd. 11, 1988, Seiten 1-21, XP008088395
- GEZONDHEIDSRAAD HEALTH COUNCIL OF THE NETHERLANDS: "Azobisisobutyronitrile. Health-based recommended occupational exposure limit" INTERNET CITATION, [Online] 12. März 2002 (2002-03-12), XP007903981 Gefunden im Internet: URL:http://www.gr.nl/pdf.php?ID=514> [gefunden am 2008-02-01]

## Beschreibung

Die vorliegende Erfindung betrifft ein Verfahren zur Herstellung von Polymeren durch radikalische Polymerisation eines Gemisches umfassend 99,99 bis 10 Gew.-% wenigstens einer α,β-ethylenisch ungesättigten Verbindung mit wenigstens einer kationogenen und/oder kationischen Gruppe pro Molekül, 0 bis weniger als 25 Gew.-% wenigstens einer von a) verschiedenen monoethylenisch ungesättigten amidgruppenhaltigen Verbindung sowie 0,01 bis 5 Gew.-% eines Vernetzers unter Verwendung von wenigstens zwei verschiedenen wasserunlöslichen Initiatoren.

Polymere finden in der Haarkosmetik vielfache Anwendung. Ihre Aufgabe in der Haarkosmetik besteht darin, die Eigenschaften des Haares zu beeinflussen, insbesondere dem Haar Festigung zu geben, die Kämmbarkeit zu verbessern und ein angenehmes Griffgefühl zu vermitteln.

So werden Conditioner dazu eingesetzt, um die Trocken- und Nasskämmbarkeit, Gefühl, Glanz und Erscheinungsform zu verbessern sowie dem Haar antistatische Eigenschaften zu verleihen. Bevorzugt werden wasserlösliche Polymere mit polaren, häufig kationischen Funktionalitäten eingesetzt, die eine größere Affinität zur strukturell bedingten negativen Oberfläche des Haares aufweisen. Struktur und Wirkungsweise verschiedener Haarbehandlungspolymere sind in Cosmetic & Toiletries 103 (1988) 23 beschrieben. Handelsübliche Conditionerpolymere sind z.B. kationische Hydroxyethylcellulose, kationische Polymere auf der Basis von N-Vinylpyrrolidon, z.B. Copolymere aus N-Vinylpyrrolidon und quarterniertem N-Vinylimidazol, Acrylamid und Diallyldimethylammoniumchlorid oder Silikone.

Zur Festigung von Haarfrisuren werden Vinyllactam-Homo- und Copolymere und Carboxylatgruppenhaltige Polymere eingesetzt. Anforderungen an Haarfestigerharze sind zum Beispiel eine starke Festigung bei hoher Luftfeuchtigkeit, Elastizität, Auswaschbarkeit vom Haar, Verträglichkeit in der Formulierung und ein angenehmer Griff des Haares.

Schwierigkeiten bereitet oft die Kombination verschiedener Eigenschaften wie zum Beispiel angenehmer Griff des Haares, Festigung und gleichzeitige verdickende Wirkung der Polymere in den haarkosmetischen Zubereitungen.

Dies ist insbesondere in Gelformulierungen von Bedeutung. Darüberhinaus zeigen übliche Festigerpolymere häufig Unverträglichkeiten mit Verdickerpolymeren, es kommt zu Trübungen und Ausfällungen in den kosmetischen Formulierungen. Klassische Verdicker, die entweder aus vernetzter Polyacrylsäure (Carbopol) oder Copolymeren bestehen, haben den Nachteil, daß sie aufgrund der Vernetzung keine für die Haarfestigung geeigneten Filme bilden. Sie sorgen für die Konsistenz des Gels, werden aber nach dem Trocknen des Gels auf dem Haar nicht mehr benötigt und stören daher bisweilen die anwendungstechnischen und ästhetischen Eigenschaften der Formulierung (Festigungswirkung, Feuchtigkeitsempfindlichkeit, Klarheit, Gelstruktur).

Weiterhin kommen rheologiemodifizierende Mittel auch in pharmazeutischen Zubereitungen zur Anwendung. So enthalten insbesondere Zubereitungen für die topische Anwendung wie Salben, Cremes, Gele, Emulsionen oder Tropfen die Viskosität beeinflussende Mittel. Auch in Sirupen sind entsprechende Mittel zu finden.

Häufig in pharmazeutischen Zubereitungen für diesen Zweck verwendete Mittel sind die bereits erwähnten Carbopole (CTFA-Bezeichnung "Carbomer") . Diese müssen für die Anwendung mit anorganischen oder organischen Basen neutralisiert werden, wodurch der pH-Wert ansteigt. Die verdickende Wirkung ist somit pH-abhängig. Im Sauren ist keine ausreichende verdickende Wirkung gegeben.

Die notwendige Neutralisierung der Carbomere bereitet ausserdem Probleme beim Einsatz von Wirkstoffsäuren, vor allem bei topischen Anwendungen. Die Wirkstoffe müssen dann in der Zubereitung in ihre Salze überführt werden, was bei Aufnahme über die Haut die Resorption erschwert.

Hinzu kommt, dass alkalisch instabile Wirkstoffe wie beispielsweise die Ascorbinsäure in Gegenwart von neutralisierten Carbomeren zur Zersetzung neigen.

### Stand der Technik

WO 04/100910 (BASF) beschreibt kosmetische Mittel, welche wenigstens ein Polymer enthalten, das durch radikalische Polymerisation α,β-ethylenisch ungesättigter Verbindungen, die jeweils mindestens einen stickstoffhaltigen Heterocyclus enthalten, in Gegenwart einer polymeren Pfropfgrundlage, erhältlich ist. Insbesondere werden Vinylpyrrolidon-Vinylimidazol-Copolymere, die in Gegenwart von Polyethylenglykol hergestellt werden, beschrieben. Die beschriebenen Polymere sind nicht vernetzt.

WO 93/22380 (ISP) offenbart Hydrogele, Klebstoffe und Beschichtungen enthaltend vernetzte Copolymere aus 80-99 Gew.-% N-Vinylpyrrolidon und 1 bis 20 Ges.-% N-Vinylimidazol oder 4-Vinylpyridin, die durch Lösungspolymerisation in Wasser hergestellt wurden. Die Verwendung von wenigstens 2 verschiedenen Initiatoren bei der Herstellung der kationischen Polymere wird nicht beschrieben.

DE 198 33 287 (BASF) beschreibt die Herstellung von Polymerisaten aus (a1) 5 bis 99,99 Gew.-% eines radikalisch polymerisierbaren Monomeren, welches ein quatemiertes oder ein quaternierbares Stickstoffatom enthält, oder Gemischen solcher Monomeren, (a2) 5 bis 95 Gew.-% eines N-Vinyllactams, (b) 0,01 bis 20 Gew.-% eines vernetzend wirkenden Monomeren mit mindestens zwei ethylenisch ungesättigten Gruppen, und (c) 0 bis 50 Gew.-% eines weiteren radikalisch polymerisierbaren Monomeren in überkritischem Kohlendioxid als inertem Verdünnungsmittel unter Durchmischung bei Temperaturen von über 31°C bis 150°C und Drücken oberhalb von 73 bar. Die Verwendung von wenigstens 2 verschiedenen wasserunlöslichen Initiatoren bei der Herstellung der kationischen Polymere wird nicht beschrieben.

DE 197 31 907 (BASF) beschreibt vernetzte kationische Polymere aus Vinylimidazol und N-Vinylpyrrolidon, die durch Gel-, umgekehrte Suspensions- oder Inverse Emulsionspolymerisation in wässrigen Lösungsmittelgemischen hergestellt werden. Die Verwendung von wenigstens 2 verschiedenen wasserunlöslichen Initiatoren bei der Herstellung der kationischen Polymere wird nicht beschrieben.

WO 93/25595 beschreibt vernetzte kationische Copolymere auf Basis quaternisierter Dialkylaminoalkylacrylate oder Dialkylaminoalkylacrylamide. Die Verwendung von wenigstens 2 verschiedenen wasserunlöslichen Initiatoren bei der Herstellung der kationischen Polymere wird nicht beschrieben.

EP-A 687694 beschreibt ein Verfahren zur Herstellung von Polymerisaten auf Basis von Vinylimidazolen durch radikalische Fällungspolymerisation in einem organischen Lösungsmittel oder Lösungsmittelgemisch, welches keine aromatischen Gruppen und außer Sauerstoff kein Heteroatom enthält. Die Verwendung von wenigstens 2 verschiedenen wasserunlöslichen Initiatoren wird nicht beschrieben.

EP A 0 893 117 und EP 1 064 924 beschreiben die Verwendung von hochmolekularen vernetzten kationischen Polymeren als Lösungspolymerisate. Diese weisen eine gute konditionierende Wirkung in Shampoos aus. Die Verwendung von wenigstens 2 verschiedenen wasserunlöslichen Initiatoren bei der Herstellung der kationischen Polymere wird nicht beschrieben.

In der DE C 33 36 047 sind topisch applizierbare Zusammensetzungen des Wirkstoffs Diclofenac beschrieben, die als Verdicker mit sekundären organischen Aminen neutralisiertes Carbomer enthalten.

In der EP-A 450 123 werden Diclofenac-Natrium enthaltende Zusammensetzungen für die topische Anwendung beschrieben, die zur Vermeidung der mit einer Neutralisierung mit sekundären organischen Aminen verbundenen Nachteile als Verdicker Cellulosederivate enthalten.

### Aufgabe der Erfindung

Eine Aufgabe der vorliegenden Erfindung war ein Verfahren zur Herstellung von Polymeren, die als alleiniger Inhaltsstoff neben Wasser und/oder Alkohol einer klaren wässrigen, alkoholischen oder wässrig/alkoholischen Zubereitung in möglichst geringen Konzentrationen eine Verdickung der Zubereitung ermöglichen.

Eine weitere Aufgabe der vorliegenden Erfindung war es, Polymere zu finden, die für kosmetische Anwendungen gut geeignet sind und insbesondere auf dem Gebiet der Haarkosmetik gute anwendungstechnische Eigenschaften wie angenehmen Griff, gute Konditionier- und Festigungswirkungswirkung und dabei gleichzeitig verdickende Eigenschaften aufweisen. Daneben sind auch die visuelle Klarheit der die Polymere enthaltenden Zubereitungen, insbesondere der Gele, sowie eine gute Gelstruktur von Bedeutung.

Die bereitzustellenden Polymere sollen weiterhin eine hohe Stabilität der resultierenden Zusammensetzungen gegenüber Salzen gewährleisten und auch im pH-Wert-Bereich von 5 bis 8 mit den kosmetisch üblichen Polymeren verträglich sein.

Ziel war es weiterhin, Polymere bereitzustellen, die eine effektive Rheologiemodifizierung auch über einen möglichst breiten pH-Wert-Bereich ermöglichen. Die Polymere sollten natürlich kostengünstig zugänglich sein, und nach ihrer Herstellung möglichst wenige Aufarbeitungsmaßnahmen erfordern.

Eine weitere Aufgabe der Erfindung war die Bereitstellung von Polymeren als Verdicker für pharmazeutische Zubereitungen, insbesondere für Zubereitungen zur topischen Anwendung. Die Aufgabe betraf insbesondere die Bereitstellung von Polymeren, die über einen weiten pH-Bereich von 1 bis 10 eine verdickende Wirkung aufweisen. Weiterhin bestand die Aufgabe auch darin, verdickende Mittel für pharmazeutische Zubereitungen zu finden, die schon in kleinen Konzentrationen eine ausreichende Wirkung entfalten. Die bereitzustellenden Polymere sollen weiterhin das Problem der Salzbildung bei Wirkstoffsäuren vermeiden helfen. Ziel der vorliegenden Erfindung war es auch Gelformulierungen für im basichen oder neutralen Milieu empfindliche Wirkstoffe zu finden.
1. Die vorgenannten Aufgaben wurden gelöst durch Polymere, die erhältlich sind durch ein Verfahren zur Herstellung von Polymeren durch radikalische Polymerisation von
   a) 99,99 bis 10 Gew.-% wenigstens einer α,β-ethylenisch ungesättigten Verbindung mit wenigstens einer kationogenen und/oder kationischen Gruppe pro Molekül,
   b) 0 bis weniger als 25 Gel.-% wenigstens einer von a) verschiedenen monoethylenisch ungesättigten amidgruppenhaltigen Verbindung,
   c) 0,01 bis 5 Gew.-% eines Vernetzers,
   d) 0 bis 15 Gel.-% wenigstens einer monoethylenisch ungesättigten Verbindung d1) enthaltend wenigstens eine Gruppe ausgewählt aus der Gruppe bestehend aus, gegebenenfalls substituierten, C₅-C₃₀-Alkyl, C₅-C₃₀-Alkenyl, C₅-C₈-Cycloalkyl, Aryl, Arylalkyl und Hetaryl und/oder eines reaktiven Vorproduktes d2) der Komponente d),
   e) 0 bis 30 Gel.-% weiterer, von a) bis d) verschiedener, monoethylenisch ungesättigter Verbindungen,
      mit der Maßgabe, dass sich die Mengen der Komponenten a) bis e) zu 100 Gew.-% addieren,
      in Gegenwart von
   f) 0 bis 70 Gew.-%, bezogen auf die Summe der Komponenten a) bis e) einer polyetherhaltigen Verbindung,
      dadurch gekennzeichnet,
      - dass im Verlauf des Verfahrens wenigstens zwei unterschiedliche wasserunlösliche Initiatoren A und B eingesetzt werden und
      - dass die Polymerisation eine Füllungspolymerisation ist.

Eine bevorzugte Ausführungsform der Erfindung sind Polymere, die erhältlich sind durch das vorgenannte Verfahren, wobei die α,β-ethylenisch ungesättigte Verbindung a) ausgewählt ist aus
ai) Estern α,β-ethylenisch ungesättigter Mono- und Dicarbonsäuren mit Aminoalkoholen, welche am Aminstickstoff mono- oder dialkyliert sein können,
aii) Amiden α,β-ethylenisch ungesättigter Mono- und Dicarbonsäuren mit Diaminen, welche mindestens eine primäre oder sekundäre Aminogruppe aufweisen,
aiii) N,N-Diallylaminen,
aiv) vinyl- und allylsubstituierten Stickstoffheterocyclen,
av) vinyl- und allylsubstituierten heteroaromatischen Verbindungen und
avi) Mischungen davon.

Im Rahmen der vorliegenden Erfindung umfasst der Ausdruck "Alkyl" geradkettige und verzweigte Alkylgruppen. Geeignete kurzkettige Alkylgruppen sind z.B. geradkettige oder verzweigte C₁-C₇-Alkyl-, bevorzugt C₁-C₈-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl; n-Heptyl, 2-Heptyl, 3-Heptyl, 2-Ethylpentyl, 1-Propylbutyl, Octyl etc. Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z.B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en) etc..

"Cycloalkyl" steht vorzugsweise für C₅-C₈-Cycloalkyl, wie Cyclopentyl, Cyclohexyl, Cycloheptyl oder Cyclooctyl.

Der Ausdruck "Heterocycloalkyl" im Sinne der vorliegenden Erfindung umfasst gesättigte, cycloaliphatische Gruppen mit im Allgemeinen 4 bis 7, vorzugsweise 5 oder 6 Ringatomen, in denen 1 oder 2 der Ringkohlenstoffatome durch Heteroatome, ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, COOR, COO⁻M⁺ und NE2¹E², bevorzugt Alkyl, tragen können. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

"Aryl" umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, - SO₃H, Sulfonat, NE¹E², Alkylen-NE¹E², Nitro, Cyano oder Halogen auf.

"Hetaryl" steht vorzugsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.

"Arylalkyl" steht für Gruppen, die sowohl Alkyl- als auch Arylreste enthalten, wobei diese Arylalkyl-Gruppen entweder über den Aryl- oder über den Alkylrest mit der sie tragenden Verbindung verknüpft sind.

### Monomer a)

Für das erfindungsgemäße Verfahren geeignete Monomere a) sind beispielsweise generell die in der WO 03/080001 auf S.18, Z.27 bis S.22, Z.38 als "direkte Vorprodukte a2" bezeichneten Verbindungen, worauf hiermit in vollem Umfang Bezug genommen wird. Unter "kationogen" wird die Fähigkeit eines Moleküls verstanden, durch Protonierung/Quatemisierung in den kationischen Ladungszustand überführt werden zu können.

Geeignete kationogene Monomere a) sind ai) die Ester von α,β-ethylenisch ungesättigten Mono- und Dicarbonsäuren mit Aminoalkoholen. Bevorzugte Aminoalkohole sind C₂C₁₂-Aminoalkohole, welche am Aminstickstoff C₁-C₈-mono- oder -dialkyliert sind. Als Säurekomponente dieser Ester eignen sich z.B. Acrylsäure, Methacrylsäure, Fumarsäure, Maleinsäure, Itaconsäure, Crotonsäure, Maleinsäureanhydrid, Monobutylmaleat und Gemische davon.
Geeignete Monomere a) sind weiterhin aii) die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen.

Bevorzugte Säurekomponenten sind Acrylsäure, Methacrylsäure und deren Gemische.

Bevorzugte Ester ai) bzw. Amide aii) von α,β-ethylenisch ungesättigten Mono- und Dicarbonsäuren mit Aminoalkoholen bzw. Diaminen sind also Aminoalkyl(meth)acrylate und Aminoalkyl(meth)acrylamide der allgemeinen Formel I: 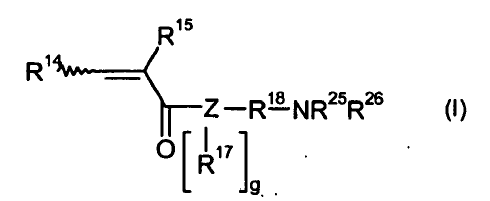 wobei
- R¹⁴ und R¹⁵: unabhängig voneinander ausgewählt sind aus der Gruppe bestehend aus Wasserstoff, C₁-C₈ linear- oder verzweigtkettige Alkyl, Methoxy, E- thoxy, 2-Hydroxyethoxy, 2-Methoxyethoxy und 2-Ethoxyethyl. Bevorzugt sind Wasserstoff, Methyl oder Ethyl,
- R¹⁷: Wasserstoff oder Methyl ist,
- R¹⁸: Alkylen oder Hydroxyalkylen mit 1 bis 24 C-Atomen, optional substituiert durch Alkyl, bevorzugt C₂H₄, C₃H₆, C₄H₈, CH₂-CH(OH)-CH₂ ist,
- g: 0 oder 1 ist,
- Z: Stickstoff für g = 1 oder Sauerstoff für g = 0 ist,
- R²⁵ bzw.R²⁶: jeweils und unabhängig voneinander ausgewählt sind aus der Gruppe bestehend aus Wasserstoff, C₁-C₄₀ linear- oder verzweigtkettige Alkyl, Formyl, C₁-C₁₀ linear- oder verzweigtkettige Acyl, N,N-Dimethylaminoethyl, 2-Hydroxyethyl, 2-Methoxyethyl, 2-Ethoxyethyl, Hydroxypropyl, Methoxypropyl, Ethoxypropyl oder Benzyl. Bevorzugt sind Wasserstoff, Methyl, Ethyl, n-Propyl und Benzyl.

Bevorzugt als Monomere ai) sind insbesondere
N-Methylaminoethyl(meth)acrylat, N-Ethylaminoethyl(meth)acrylat,
N-(n-Propyl)aminoethyl(meth)acrylat, N-(n-Butyl)aminoethyl(meth)acrylat,
N-(tert.-Butyl)aminoethyl(meth)acrylat, N,N-Dimethylaminomethyl(meth)acrylat,
N,N-Dimethylaminoethyl(meth)acrylat, N,N-Diethylaminoethyl(meth)acrylat,
N,N-Dimethylaminopropyl(meth)acrylat, N,N-Diethylaminopropyl(meth)acrylat und
N,N-Dimethylaminocyclohexyl(meth)acrylat. Insbesondere werden als Monomere ai)
N-(tert.-Butyl)aminoethylacrylat und N-(tert.-Butyl)aminoethylmethacrylat eingesetzt.

Die Amide können unsubstituiert, N-Alkyl oder N-Alkylamino monosubstituiert oder N,N-dialkylsubstituiert oder N,N-dialkylaminodisubstituiert vorliegen, worin die Alkyl- oder Alkylaminogruppen von C₁-C₄₀ linearen, C₃-C₄₀ verzweigtkettigen, oder C₃-C₄₀ carbocyclischen Einheiten abgeleitet sind.

Bevorzugte Monomere aii) sind
N-[2-(dimethylamino)ethyl]acrylamid,
N-[2-(dimethylamino)ethyl]methacrylamid, N-[3-(dimethylamino)propyl]acrylamid,
N-[3-(dimethylamino)propyl]methacrylamid, N-[4-(dimethylamino)butyl]acrylamid,
N-[4-(dimethylamino)- butyl]methacrylamid, N-[2-(diethylamino)ethyl]acrylamid,
N-[4-(dimethylamino)cyclohexyl]acrylamid, N-[4-(dimethylamino)cyclohexyl]methacrylamid, N-[8-(Dimethylamino)octyl]methacrylamid, N-[12-(Dimethylamino)dodecyl]-methacrylamid,
N-[3-(Diethylamino)propyl]methacrylamid und N-[3-(Diethylamino)propyl]acrylamid.

Weiterhin kann Monomer a) auch ausgewählt sein aus aiii) N,N-Diallylaminen der allgemeinen Formel **II** 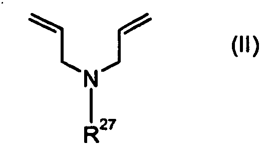 wobei R²⁷ Wasserstoff oder C₁ bis C₂₄ Alkyl bedeutet. Besonders bevorzugt sind N,N-Diallylamin und N,N-Diallyl-N-Methylamin, insbesondere N,N-Diallyl-N-Methylamin. Besonders bevorzugt ist N,N,-Diallyl-N-Methylamin, das in einer quatemisierten Form beispielsweise unter der Bezeichnung DADMAC (Diallyldimethylammoniumchlorid) kommerziell erhältlich ist.

Besonders bevorzugte kationogene Monomere a) sind weiterhin aiv) vinyl- und allylsubstituierte Stickstoffheterocyclen, wie N-Vinylimidazol, N-Vinylimidazol-Derivate, z.B. N-Vinyl-2-methylimidazol, vinyl- und allylsubstituierte heteroaromatische Verbindungen, wie 2- und 4-Vinylpyridin, 2- und 4-Allylpyridin.

Ganz besonders bevorzugt sind N-Vinylimidazole der allgemeinen Formel (III), worin R¹ bis R³ für Wasserstoff, C₁-C₄-Alkyl oder Phenyl steht

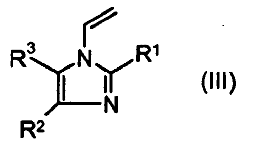

Beispiele für Verbindungen der allgemeinen Formel (III) sind folgender Tabelle 1 zu entnehmen:

**Tabelle 1**

| R¹ | R² | R³ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Am meisten bevorzugt als Monomer a) ist aiv) N-Vinylimidazol, also die Verbindung der Formel III, wobei alle Reste R¹ bis R³ Wasserstoff bedeuten.

Das nach dem erfindungsgemäßen Verfahren hergestellte Polymer enthält höchstens 99,99 und wenigstens 10, bevorzugt wenigstens 30, weiter bevorzugt wenigstens 60 und besonders bevorzugt wenigstens 70 Gew.-% einpolymerisierte Monomere a).

Insbesondere für die bevorzugte erfindungsgemäße Verwendung als Verdicker in haarkosmetischen Zubereitungen sind Polymere mit einem Anteil von wenigstens 60, bevorzugt wenigstens 70 Gew.-% einpolymerisierter Monomere a) vorteilhaft.

Sofern nicht ausdrücklich anderweitig vermerkt, beziehen sich die Angaben "Gew.-%" für die Komponenten a) bis e) auf die Summe von 100 Gew.-% der Komponenten a) bis e).

Die Umsetzung von a) zu quaternären Verbindungen erfolgt während oder bevorzugt nach der Reaktion. Bei einer nachfolgenden Umsetzung kann das intermediäre Polymerisat zunächst isoliert und gereinigt oder bevorzugt im Wesentlichen unmittelbar umgesetzt werden. Die Quaternisierung kann vollständig oder teilweise erfolgen. Dabei werden bevorzugt mindestens 10 %, weiter bevorzugt mindestens 20 %, besonders bevorzugt mindestens 30 % und insbesondere wenigstens 50 mol-% der eingebauten Monomere a) in die entsprechende quaternäre Form überführt. Der Anteil der Umsetzung zu quaternären Verbindungen ist bevorzugt um so höher, je geringer die Wasserlöslichkeit von a) ist.

Bevorzugt ist es, die Monomere a) in überwiegend, d.h. zu mehr als 70, bevorzugt zu mehr als 90, besonders bevorzugt zumehr als 95, am meisten bevorzugt zu mehr als 99 Mol-% in kationogener, also nicht quaternisierter oder protonierter Form zur Polymerisation einzusetzen und erst während oder besonders bevorzugt nach der Polymerisation durch Quaternisierung in die quatemisierte oder protonierte Form zu überführen.
Nach der Polymerisation bedeutet, dass 90, bevorzugt 95, weiter bevorzugt 99 und insbesondere 99,9 Gew.-% der Monomere a) bis e) einpolymerisiert sind und nicht mehr in polymerisierbarer Form vorliegen.

Die Quaternisierung wird bevorzugt unmittelbar nach der Polymerisation in der Polymerisationsmischung durchgeführt. Es ist bevorzugt, nach der Polymerisation und vor der Quaternisierung keine weiteren Verfahrensschritte wie Destillation, Waschen, Ausfällung oder sonstige Isolierungs- oder Reinigungsschritte vorzunehmen.

### Protonierung/Quaternisierung

In einer bevorzugten Ausführungsform der Erfindung wird das Polymer nach der Polymerisation teilweise oder vollständig protoniert oder quaternisiert, da als Monomer a) bevorzugt ein nicht oder nur teilweise quaternisiertes oder protoniertes Monomer zur Polymerisation eingesetzt wird.

Die Monomere (a) werden bevorzugt in nicht-quatemisierter bzw. nicht-protonierter Form polymerisiert, wobei man im letzteren Fall das erhaltene Polymer bevorzugt nach der Polymerisation quaternisiert/protoniert wird.

Für den Fall, dass die Monomeren in quaternisierter Form eingesetzt werden, kann man sie entweder als getrocknete Substanz oder in Form konzentrierter Lösungen in für die Monomeren geeigneten Lösungsmitteln, beispielsweise in polaren Lösungsmitteln wie Wasser, Methanol, Ethanol, Aceton, in den weiteren Komponenten a) bis f), sofern diese als Lösungsmittel geeignet sind oder in Elektrolytlösungen einsetzen.

Zur Protonierung eignen sich beispielsweise Mineralsäuren wie HCl, H₂S0₄, sowie Monocarbonsäuren z.B. Ameisensäure und Essigsäure, Dicarbonsäuren und mehrfunktionelle Carbonsäuren, z.B. Oxalsäure und Zitronensäure sowie alle anderen protonenabgebenden Verbindungen und Substanzen, die in der Lage sind, das entsprechende Stickstoffatom zu protonieren. Insbesondere eignen sich wasserlösliche Säuren zur Protonierung.
Als bevorzugte anorganische Säuren seien Phosphorsäure, phosphorige Säure, Schwefelsäure, schweflige Säure und Salzsäure genannt, wobei Phosphorsäure besonders bevorzugt ist. ,
Als mögliche organische Säuren seien ein- und mehrwertige, gegebenenfalls substituierte aliphatische und aromatische Carbonsäuren, ein- und mehrwertige, gegebenenfalls substituierte aliphatische und aromatische Sulfonsäuren oder ein- und mehrwertige, gegebenenfalls substituierte aliphatische und aromatische Phosphonsäuren. Bevorzugte organische Säuren sind Hydroxycarbonsäuren wie beispielsweise Glykolsäure, Milchsäure, Weinsäure und Citronensäure genannt, wobei Milchsäure besonders bevorzugt ist.
Die Protonierung des Polymers erfolgt entweder während, unmittelbar im Anschluss an die Polymerisation oder erst bei der Formulierung der kosmetischen Zubereitung, bei der in der Regel ein physiologisch verträglicher pH-Wert eingestellt wird.
Unter Protonierung ist zu verstehen, dass mindestens ein Teil der protonierbaren Gruppen des Polymers, bevorzugt wenigstens 20, bevorzugt mehr als 50, besonders bevorzugt mehr als 70, am meisten bevorzugt mehr als 90 Mol-% protoniert wird, so dass eine kationische Gesamtladung des Polymers resultiert.

Zur Quaternisierung der Verbindungen a) eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid, Propylbromid, Hexylbromid, Dodecylbromid, Laurylbromid und Benzylhalogenide, insbesondere Benzylchlorid und Benzylbromid. Bevorzugtes Quaternisierungsmittel ist Methylchlorid. Zur Quaternisierung mit langkettigen Alkylresten sind die entsprechenden Alkylbromide wie Hexylbromid, Dodecylbromid oder Laurylbromid bevorzugt. Weitere geeignete Quaternsierungsmittel sind Dialkylsulfate, insbesondere Dimethylsulfat oder Diethylsulfat.
Die Quaternisierung der basischen Monomere a) kann auch mit Alkylenoxiden wie Ethylenoxid oder Propylenoxid in Gegenwart von Säuren durchgeführt werden. Bevorzugte Quaternisierungsmittel sind Methylchlorid, Dimethylsulfat oder Diethylsulfat, wobei Methylchlorid besonders bevorzugt ist.
Die Quaternisierung der Polymere mit einem der genannten Quaternisierungsmittel erfolgt nach allgemein bekannten Methoden.

Die nach dem erfindungsgemäßen Verfahren hergestellten Polymere werden bevor zugt zur Modifizierung der Rheologie wässriger Zusammensetzungen, bevorzugt im pH-Wert-Bereich von 1 bis 12, besonders bevorzugt von 2 bis 10 verwendet.

Im Bereich von pH 1 bis pH 5 ist es vorteilhaft, wenn die quatemisierbaren Gruppen der Polymere zu weniger als 20%, bevorzugt zu weniger als 10%, besonders bevorzugt zu weniger als 1% quaternisiert vorliegen.

Im Bereich von pH 6 bis pH 10 ist es vorteilhaft, wenn die quaternisierbaren Gruppen der Polymere zu wenigstens 10%, bevorzugt zu wenigstens 20% und höchstens zu 99, bevorzugt zu höchstens 90 quaternisiert vorliegen.

In einer bevorzugten Ausführungsform der Erfindung werden die quatemisierbaren Gruppen der durch das erfindungsgemäße Verfahren erhältlichen Polymere im Bereiich von 40 bis 80, bevorzugt im Bereich von 50 bis 70 mol% quatemisiert.

Zwischen pH 5 und pH 6 können die Polymere, abhängig von ihrer quantitativen und/oder qualitativen Monomerzusammensetzung vorteilhaft entweder teilweise quaternisiert oder nicht quaternisiert vorliegen.
Der Fachmann findet durch Routine-Versuche heraus, ob die quaternisierbaren Gruppen eines erfindungsgemäß hergestellten Polymers in einem bestimmten pH-Wert-Bereich vorteilhaft zu mehr als 50 mol-%, zu 50 mol% oder zu weniger als 50 mol-% quaternisiert vorliegen müssen, um die gewünschten Effekte wie die Rheologiemodifizierung zu erzielen. Durch weitere Routine-Versuche lässt sich auch der für die gewünschte Wirkung am besten geeignete Quatemisierungsgrad (= Quotient aus Menge der quaternisierten Gruppen und Summe der Mengen von quatemisierten Gruppen und nicht quaternisierten quatemisierbaren Gruppen) ermitteln.

In einer Ausführungsform der Erfindung enthält das Polymer nur Monomere a) und c) einpolymerisiert.

### Monomer b).

Das nach dem erfindungsgemäßen Verfahren hergestellte Polymer enthält 0 bis weniger als 25, bevorzugt 1 bis 20 und besonders bevorzugt 5 bis 20 Gew.-% wenigstens einer von a) verschiedenen, monoethylenisch ungesättigten amidgruppenhaltigen Verbindung b) einpolymerisiert.
Monomer b) ist bevorzugt ausgewählt aus Verbindungen der allgemeinen Formel IV 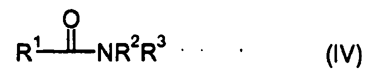 wobei
R¹ für eine Gruppe der Formel CH₂=CR⁴- mit R⁴ = H oder C₁-C₄-Alkyl steht und R² und R³ unabhängig voneinander für H, Alkyl, Cycloalkyl, Heterocycloalkyl, Aryl oder Hetaryl stehen oder R² und R³ gemeinsam mit dem Stickstoffatom, an das sie gebunden sind, für einen fünf- bis achtgliedrigen Stickstoff-Heterocyclus stehen oder
R² für eine Gruppe der Formel CH₂=CR⁴- steht und R¹ und R³ unabhängig voneinander für H, Alkyl, Cycloalkyl, Heterocycloalkyl, Aryl oder Hetaryl stehen oder R¹ und R³ gemeinsam mit der Amidgruppe, an die sie gebunden sind für ein Lactam mit 5 bis 8 Ringatomen stehen.

Bevorzugt enthält das nach dem erfindungsgemäßen Verfahren hergestellte Polymer als Monomer b) wenigstens ein N-Vinyllactam einpolymerisiert. Als N-Vinyllactam b) eignen sich unsubstituierte N-Vinyllactame und N-Vinyllactamderivate, die z.B. einen oder mehrere C₁-C₆-Alkylsubstituenten, wie Methyl, Ethyl, n-Propyl, Isopropyl, n-Butyl, sec.-Butyl, tert.-Butyl etc., aufweisen können. Dazu zählen z.B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. sowie deren Mischungen.

Bevorzugt enthält das nach dem erfindungsgemäßen Verfahren hergestellte Polymer Monomere b) eingebaut, wobei in Formel IV R² für CH₂=CH- und R¹ und R³ gemeinsam mit der Amidgruppe, an die sie gebunden sind für ein Lactam mit 5 Ringatomen stehen.

Besonders bevorzugt werden als Monomere b) N-Vinylpyrrolidon, N-Vinylcaprolactam, N-Vinylformamid, (Meth)Acrylamid oder deren Mischungen eingesetzt, wobei N-Vinylpyrrolidon und Methacrylamid am meisten bevorzugt sind.

In einer Ausführungsform enthält das das nach dem erfindungsgemäßen Verfahren hergestellte Polymer lediglich Monomere a) und b) eingebaut, wobei N-Vinylimidazol als a) und N-Vinylpyrrolidon als b) bevorzugt sind.

Das das nach dem erfindungsgemäßen Verfahren hergestellte Polymer enthält weniger als 25, bevorzugt höchstens 23 und insbesondere höchstens 20 Gew.-% von b) einpolymerisiert.
In einer Ausführungsform der Erfindung enthält das nach dem erfindungsgemäßen Verfahren hergestellte Polymer wenigstens 1, besonders bevorzugt wenigstens 2 und insbesondere wenigstens 5 und weniger als 25, bevorzugt höchstens 20 Gew.-% von b) einpolymerisiert.

### Vernetzer c)

In einer weiteren bevorzugten Ausführungsform der Erfindung ist der zur Herstellung der für die erfindungsgemäße Verwendung geeigneten Polymere verwendete Vernetzer c) ausgewählt aus Verbindungen mit wenigstens 2 ethylenisch ungesättigten, nichtkonjugierten, radikalisch polymerisierbaren Doppelbindungen pro Molekül.

Geeignete Vernetzer c) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000.

Außer den Homopolymeren des Ethylenoxids bzw. Propylenoxids können auch Blockcopolymere aus Ethylenoxid oder Propylenoxid oder Copolymere, die Ethylenoxid- und Propylenoxid-Gruppen eingebaut enthalten, eingesetzt werden.

Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Bevorzugte mehrwertige Alkohole in diesem Zusammenhang sind auch Di- und Trisaccaride.

Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer c) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer sind ferner geeignet die Amide von (Meth)Acrylsäure, ltaconsäure und Maleinsäure sowie N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder lsophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Vernetzer geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenhamstoff oder Weinsäurediamid, z.B. N,N'-Divinylethylenharnstoff oder N,N'-Divinylpropylenharnstoff.

Geeignet sind auch Alkylenbisacrylamide wie Methylenbisacrylamid und N,N'-(2,2)butan und 1,1'-bis-(3,3'-vinylbenzimidazolith-2-on)-1,4-butan.

Andere geeignete Vernetzer sind beispielsweise Alkylenglykoldi(meth)acrylate wie Ethylenglykoldiacrylat, Ethylenglykoldimethacrylat, Tetraethlyenglykolacrylat, Tetraethylenglykoldimethacrylat, Diethylenglykolacrylat, Diethylenglykolmethacrylat, Vinylacrylat, Allylacrylat, Allylmethacrylat, Divinyldioxan, Pentaerythritallylether sowie Gemische dieser Vernetzer.

Weitere geeignete Vernetzer sind Divinyldioxan, Tetraallylsilan oder Tetravinylsilan.

Besonders bevorzugt eingesetzte Vernetzer sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Pentaerythrittriallylether, Methylenbisacrylamid, N,N'-Divinylethylenharnstoff, Triallylamin und Triallylmonoalkylammoniumsalze, und Acrylsäureester von Ethylenglykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin. Am meisten bevorzugt ist Pentaerythrittriallylether.

Selbstverständlich können auch Mischungen der vorgenannten Verbindungen eingesetzt werden. Der Vernetzer ist vorzugsweise im Reaktionsmedium löslich. Ist die Löslichkeit des Vernetzers im Reaktionsmedium gering, so kann er in einem Monomeren oder in einer Monomerenmischung gelöst werden oder aber in einem Lösungsmittel gelöst zudosiert werden, das sich mit dem Reaktionsmedium mischt. Besonders bevorzugt sind solche Vernetzer, die in der Monomermischung löslich sind.

Die Vernetzer c) werden für die erfindungsgemäße Verwendung in Mengen von wenigstens 0,01, bevorzugt wenigstens 0,02, weiter bevorzugt wenigstens 0,05 und besonders bevorzugt wenigstens 0,1 und höchstens 10, bevorzugt höchstens 5, weiter bevorzugt höchstens 2 und besonders bevorzugt höchstens 1 Gew.-%, bezogen auf die Gesamtmenge der Monomere a) bis e), eingesetzt.

In einer besonders bevorzugten Ausführungsform der Erfindung wird Pentaerythrittriallylether in einer Menge von 0,1 Gew.-% bis 0,7 Gew.%, am meisten bevorzugt in einer Menge von 0,3 Gew.-% bis 0,6 Gew.-% eingesetzt.

Die Gew.-%-Menge von Vernetzer c) bezieht sich auf die zur Herstellung des Polymers verwendete Menge des Gemisches der Komponenten a) bis e).

Weitere monoethylenisch ungesättigte Verbindung d)

Erfindungsgemäß umfasst das zu polymerisierende Monomerengemisch weiterhin 0 bis 15 Gew.-% wenigstens einer monoethylenisch ungesättigten Verbindung d1) enthaltend wenigstens eine Gruppe ausgewählt aus der Gruppe bestehend aus gegebenenfalls substituierten C₅-C₃₀-Alkyl, C₅-C₃₀-Alkenyl, C₅-C₈-Cycloalkyl, Aryl, Arylalkyl und Hetaryl und/oder eines reaktiven Vorproduktes (d2) der Komponente d).

Verbindung d) trägt im für die erfindungsgemäße Verwendung geeigneten Polymer eine hydrophobe Gruppe.

Bei den Verbindungen d1) kann es sich um an sich hydrophobe Monomere handeln wie beispielsweise Ester bzw. Amide der (Meth)acrylsäure mit aliphatischen C₅- C₃₀-Alkoholen bzw. bzw. Aminen wie z.B. Hexyl(meth)acrylat bzw. -(meth)acrylamid, n-Heptyl(meth)acrylat bzw. -(meth)acrylamid, n-Octyl(meth)acrylat bzw. - (meth)acrylamid, n-Nonyl(meth)acrylat bzw. -(meth)acrylamid, n-Decyl(meth)acrylat bzw. -(meth)acrylamid, n-Undecyl(meth)acrylat bzw. -(meth)acrylamid, n-Dodecyl(meth)acrylat bzw. -(meth)acrylamid, n-Tridecyl(meth)acrylat bzw. - (meth)acrylamid, n-Tetradecyl(meth)acrylat bzw. -(meth)acrylamid, n-Pentadecyl(meth)acrylat bzw. -(meth)acrylamid, n-Hexadecyl(meth)acrylat bzw. - (meth)acrylamid, n-Heptadecyl(meth)acrylat bzw. -(meth)acrylamid, n-Octadecyl(meth)acrylat bzw. -(meth)acrylamid und n-Nonadecyl(meth)acrylat bzw. - (meth)acrylamid.

Bei den Verbindungen d1) kann es sich beispielsweise auch um mit hydrophoben Resten substituierte (Meth)Acrylsäureester von Polyalkylenglykolen handeln wie beispielsweise alkylsubstituierte (Meth)Acrylsäure-Polyethylenglykolester.

Geeignet als d1) sind auch langkettige Allyl- oder Vinylether wie C₅-C₃₀-Alkyl-Vinylether oder C₅-C₃₀-Alkenyl-Vinylether.

Geeignet als d1) sind auch radikalisch polymerisierbare, olefinisch ungesättigte Gruppen enthaltende Derivate von Polyisobuten. Davon bevorzugte Verbindungen d1) sind beispielsweise Umsetzungsprodukte von Polyisobutensuccinanhydrid (PIBSA) mit Hydroxyalkyl(meth)acrylaten und Polyisobutensuccinimid (PIBSA) mit Hydroxyalkyl(meth)acrylaten

WO 04/035635, S.12, Z.26 bis S.27, Z.2 beschreibt detailliert Verfahren zur Herstellung von Polyisobuten-Derivaten, die dann durch übliche Reaktionen mit Komponenten, die olefinisch ungesättigte Gruppen enthaltend, zu geeigneten Verbindungen d) umgesetzt werden können. Auf diese Beschreibung wird hier in vollem Umfang Bezug genommen. Polyisobuten-Derivate, die zu geeigneten Verbindungen d) umgesetzt werden können, sind beispielsweise die Produkte, die unter den Handelsnamen Glissopal^{®} oder Kerocom^{®} (jeweils BASF) kommerziell erhältlich sind.

In einer besonders bevorzugten Ausführungsform der Erfindung werden als Verbindungen d) 2 bis 10 Gew.-% Octadecylvinylether und/oder Behenylacrylat und/oder Stearylmethacrylat und/oder Lauryl(meth)acrylat verwendet.

In einer weiteren besonders bevorzugten Ausführungsform der Erfindung werden als Verbindungen d) Ester der (Meth)acrylsäure mit Polyethylenglykolmono-C₁₆-C₂₂-alkylethern eingesetzt. Bevorzute Polyethylenglykolmono-C₁₆-C₂₂Alkylether enthalten pro Molekül von 25 bis 80 Einheiten Ethylenoxid.

Beispielsweise können als Verbindungen d) Ester der (Meth)acrylsäure mit Lutensol^{®} AT 25, Lutensol^{®} AT 50 oder Lutenso^{®} AT 80 eingesetzt werden.

Weiterhin geeignet sind Methacrylsäureester von ethoxylierten (beispielsweise mit 25 mol Ethylenoxid) C₁₆ - C₁₈-Fettalkoholgemischen, wie sie beispielsweise als PLEX^{®}O-6877 oder PLEX^{®}O-6954 (Degussa) kommerziell erhältlich sind.

Unter reaktiven Vorprodukten d2) der Komponente d) werden im Rahmen dieser Erfindung solche radikalisch polymerisierbaren Monomere verstanden, die vor oder nach ihrer Einpolymerisation durch eine, gegebenenfalls polymeranaloge, Umsetzung mit wenigstens einer Gruppe ausgewählt aus der Gruppe bestehend aus, gegebenenfalls substituierten, C₅-C₃₀-Alkyl, C₅-C₃₀-Alkenyl, C₅-C₈-Cycloalkyl, Aryl, Arylalkyl und Hetaryl kovalent verknüpft werden können. Als Beispiel seien monoethylenisch ungesättigte Verbindungen, die eine Epoxidgruppe tragen, genannt. Diese Epoxidgruppen können beispielsweise nach der Einpolymerisation in ein Polymer durch Umsetzung mit C₅-C₃₀-Alkoholen mit einer C₅-C₃₀-Alkylkette kovalent verknüpft werden.

Weitere bevorzugte Verbindungen d) sind ausgewählt aus der Gruppe bestehend aus C₁₂C₃₀₋Alkyl(meth)acrylaten und C₁₂-C₃₀-Alkylvinylethern.

Das für die erfindungsgemäße Verwendung geeignete Polymer enthält höchstens 20, bevorzugt höchstens 15 und besonders bevorzugt höchstens 10 und bevorzugt wenigstens 1, besonders bevorzugt wenigstens 2 und insbesondere wenigstens 4 Gew.-% des oder der Verbindungen d) einpolymerisiert.

Insbesondere für die Verwendung der Polymere als Verdicker in hautkosmetischen Zubereitungen ist ein Anteil von wenigstens 2, bevorzugt wenigstens 4 Gew.-% einpolymerisierter Komponente d) vorteilhaft.

### Polyetherhaltige Verbindung f)

Das für die erfindungsgemäße Verwendung geeignete Polymer ist erhältlich durch Polymerisation in Gegenwart von 0 bis 70 Gew.-%, bezogen auf die Menge der Komponenten a) bis e), einer polyetherhaltigen Verbindung f).

Geeignete polyetherhaltige Verbindungen f) sind im Allgemeinen wasserlösliche oder wasserdispergierbare, nichtionische Polymere, die Polyalkylenglycolgruppen aufweisen. Vorzugsweise beträgt der Anteil an Polyalkylenglycolgruppen mindestens 40 Gew.%, bezogen auf das Gesamtgewicht der Verbindung f). Als polyetherhaltige Verbindung f) können beispielsweise Polyalkylenglykole, Polyester auf Basis von Polyalkylenglykolen sowie Polyetherurethane eingesetzt werden.

Bei Komponente f) handelt es sich bevorzugt um Polyether aus der Gruppe der Polyalkylenoxide auf Basis von Ethylenoxid, Propylenoxid und Butylenoxiden, Polytetrahydrofuran sowie Polyglycerin. Je nach Art der zu ihrer Herstellung eingesetzten Monomerbausteine enthalten die polyetherhaltigen Verbindungen f) folgende Struktureinheiten:
-(CH₂)₂-O-, -(CH₂)₃-O, -(CH₂)₄-O-, -CH₂-CH(CH₃)-O-, -CH₂-CH(CH₂-CH₃-O-, -CH₂-CHOR^{a}-CH₂-O,
worin R^{a} für C₁-C₂₄-Alkyl, vorzugsweise C₁-C₄-Alkyl, steht.

Geeignet sind sowohl Homopolymerisate als auch Copolymerisate, wobei die Copolymerisate die Alkylenoxideinheiten statistisch verteilt oder als Blöcke enthalten können.

Die Verbindungen f) können zusätzlich verbrückende Gruppen aufweisen, die beispielsweise ausgewählt sind unter:
-C(=O)-O-, -O-C(=O)-O-, -C(=O)-NR^{b}-, -O-C(=O)-NR^{b}-, -NRC-(C=O)-NR^{b}-,
worin R^{b} und R^{c} unabhängig voneinander für Wasserstoff, C₁-C₃₀-Alkyl, vorzugsweise C₁-C₄-Alkyl, oder Cycloalkyl stehen.

Vorzugsweise haben die Polyether f) ein zahlenmittleres Molekulargewicht Mₙ von mindestens 300.

Vorzugsweise weisen die Polyether f) die allgemeine Formel Va oder Vb 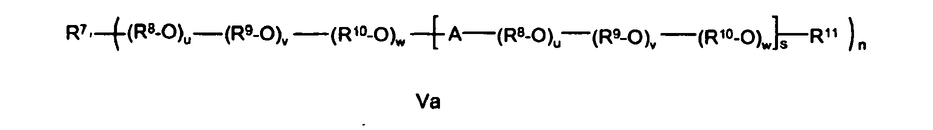 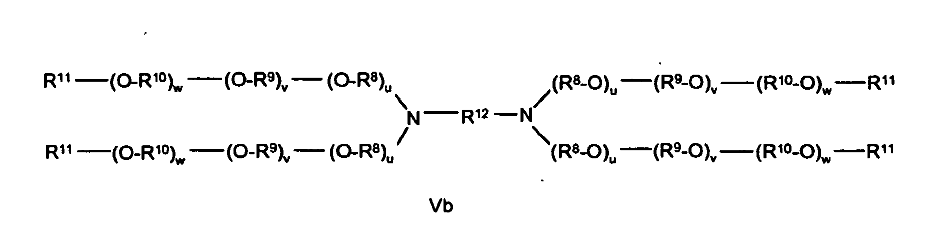 auf, worin:
- R⁷: für Hydroxy, Amino, C₁-C₂₄-Alkoxy, R¹³-COO-, R¹³-NH-COO- oder einen Polyal- koholrest steht,
- R⁸, R⁹ und R¹⁰: unabhängig voneinander für -(CH₂)₂-, -(CH₂)₃-, -(CH₂)₄-, -CH₂CH(CH₃)-. -CH₂-CH(CH₂-CH₃)- oder -CH₂-CHOR¹⁴-CH₂- stehen,
- R¹¹: für Wasserstoff, Amino-C₁-C₆-alkyl, C₁-C₂₄-Alkyl, R¹³-C(=O)- oder R¹³-NH-C(=O)- steht,
- R¹²: für eine C₁-C₂₀-Alkylengruppe steht, deren Kohlenstoffkette durch 1 bis 10 nicht- benachbarte Sauerstoffatome unterbrochen sein kann;
- R¹³: für C₁-C₂₄-Alkyl steht,
- R¹⁴: für Wasserstoff, C₁-C₂₄-Alkyl oder R¹³-CO- steht,
- A: für -C(=O)-O-, -C(=O)-B-C(=O)-O- oder -C(=O)-NH-B-NH-C(=O)-O- steht,
- B: für -(CH₂)r, gewünschtenfalls substituiertes Cycloalkylen, gewünschtenfalls sub- stituiertes Heterocycloalkylen oder gewünschtenfalls substituiertes Arylen steht,
- n: für 1 oder, wenn R⁷ einen Polyalkoholrest bedeutet, 1 bis 8 steht,
- s: für 0 bis 500, bevorzugt 0 bis 100 steht,
- t: für 1 bis 12, bevorzugt 2 bis 6, steht,
- u: unabhängig voneinander jeweils für 1 bis 5000, bevorzugt 1 bis 1000, steht,
- v: unabhängig voneinander jeweils für 0 bis 5000, bevorzugt 1 bis 1000, steht,
- w: unabhängig voneinander jeweils für 0 bis 5000, bevorzugt 1 bis 1000, steht.

Bevorzugte Komponenten f) sind die Polyether der Formel Va.

Die endständigen primären Hydroxylgruppen der auf Basis von Alkylenoxiden, Tetrahydrofuran oder Glycerin hergestellten Polyether sowie die sekundären OH-Gruppen von Polyglycerin können sowohl frei vorliegen als auch mit C₁-C₂₄-Alkoholen verethert, mit C₁-C₂₄-Carbonsäuren verestert oder mit Isocyanaten zu Urethanen umgesetzt sein. Für diesen Zweck geeignete Alkohole sind z.B.: primäre aliphatische Alkohole, wie Methanol, Ethanol, Propanol und Butanol, primäre aromatische Alkohole, wie Phenol, Isopropylphenol, tert.-Butylphenol, Octylphenol, Nonylphenol und Naphthol, sekundäre aliphatische Alkohole, wie Isopropanol, tertiäre aliphatische Alkohole, wie tert.-Butanol und mehrwertige Alkohole, z.B. Diole, wie Ethylenglykol, Diethylenglykol, Propylenglykol, 1,3-Propandiol und Butandiol, und Triole, wie Glycerin und Trimethylolpropan. Die Hydroxylgruppen können jedoch auch durch reduktive Aminierung, z.B. mit Wasserstoff-Ammoniak-Gemischen unter Druck gegen primäre Aminogruppen ausgetauscht oder durch Cyanethylierung mit Acrylinitril und Hydrierung in Aminopropylenendgruppen umgewandelt sein. Die Verschließung der Hydroxylendgruppen kann dabei nicht nur nachträglich durch Umsetzung mit den Alkoholen oder mit Alkalimetallaugen, Aminen und Hydroxylaminen erfolgen, sondern diese Verbindungen können wie LewisSäuren, z.B. Bortrifluorid, auch zu Beginn der Polymerisation als Starter eingesetzt werden. Schließlich können die Hydroxylgruppen auch durch Umsetzung mit Alkylierungsmitteln, wie Dimethylsulfat, verschlossen werden.

Die Alkylreste in den Formeln Va und Vb können verzweigte oder unverzweigte C₁-C₂₄-Alkylreste, wie eingangs definiert sein, wobei C₁-C₁₂Alkylreste bevorzugt und C₁-C₆-Alkylreste besonders bevorzugt sind.

Das mittlere Molekulargewicht Mₙ der Polyether beträgt mindestens 300 und höchstens 100 000. Es beträgt bevorzugt 500 bis 50 000, besonders bevorzugt 2000 bis 35 000 und ganz besonders bevorzugt 2000 bis 10000.

Vorteilhafterweise verwendet man Polytetrahydrofurane, Homo- und Copolymerisate von Ethylenoxid, Propylenoxid, Butylenoxid und Isobutylenoxid, die linear oder verzweigt sein können, als Pfropfgrundlage b). Der Begriff Homopolymerisate soll dabei erfindungsgemäß auch solche Polymerisate umfassen, die außer der polymerisierten Alkylenoxideinheit noch die reaktiven Moleküle enthalten, die zur Initiierung der Polymerisation der cyclischen Ether bzw. zur Endgruppenverschließung des Polymerisats eingesetzt wurden.

Bevorzugte Verbindungen f) sind beispielsweise diejenigen polyetherhaltigen Verbindungen, die unter den Handelsnamen Pluriol™, Pluronic™, LutenSol™, Pluracol™ und Plurafac™ (jeweils BASF), Lupranol™ (Elastogran) oder PolyTHF^{®} (BASF) kommerziell erhältlich sind.

Generell sind als Komponente f) auch Polyol-Makromere verwendbar. Solche Polyol-Makromere sind dem Fachmann bekannt. Insbesondere sei auf die in US 5,093,412 und WO 05/003200 offenbarten Polyol-Makromere verwiesen, auf die hier in vollem Umfang Bezug genommen wird.

In einer bevorzugten Ausführungsform der Erfindung wird die Polymerisation in Gegenwart von polyalkylenoxidhaltigen Silikonen als Verbindungen f) durchgeführt.

Geeignete polyalkylenoxidhaltige Silikone sind beispielsweise in den folgenden Schriften beschrieben, auf deren Offenbarung hiermit Bezug genommen wird:

DE-PS 16 94 366: Sie betrifft Polysiloxan-Polyoxyalkylen-Blockmischpolymerisate, deren Polysiloxanblock in an sich bekannter Weise aufgebaut ist und deren Polyoxyalkylenblock aus 25 bis 70 Gewichtsprozent eines Polyoxyalkylens mit einem durchschnittlichen Molekulargewicht von 1600 bis 4000 und einem Ethylenoxidgehalt von 20 bis 100 Gewichtsprozent, Rest Propylenoxid und gegebenenfalls höhere Alkylenoxide, und 30 bis 75 Gewichtsprozent eines Polyoxyalkylens mit einem durchschnittlichen Molekulargewicht von 400 bis 1200 und einem Ethylenoxidgehalt von 65 bis 100 Gewichtsprozent, Rest Propylenoxid und gegebenenfalls höhere Alkylenoxide, besteht.

DE-OS 25 41 865: Die Polysiloxan-Polyoxyalkylen-Blockmischpolymerisate sind bezüglich ihrer Polyoxyalkylenblöcke so definiert, daß der eine Polyoxyalkylenblock ein mittleres Molgewicht von 900 bis 1300 hat und zu 30 bis 55 Gew.-% aus Ethylenoxid, Rest Propylenoxid, und der andere Polyoxyalkylenblock ein mittleres Molgewicht von 3800 bis 5000 hat und zu 30 bis 50 Gew.-% aus Ethylenoxid, Rest Propylenoxid besteht.

EP-A 0 275 563: Das beschriebene Blockmischpolymerisat umfaßt drei verschiedene Polyoxyalkylenblöcke, nämlich einen Block, welcher 20 bis 60 Gew.-% Oxyethyleneinheiten enthält, bei einem Molgewicht von 3000 bis 5500, einen weiteren Block mit 20 bis 60 Gew.-% Oxyethyleneinheiten und einem Molgewicht von 800 bis 2900 und einen dritten Block nur aus Polyoxypropyleneinheiten und einem Molgewicht von 130 bis 1200.

Bevorzugte polyalkylenoxid-haltige Silikone werden von der EP-A 0 670 342 beschrieben. EP-A 0 670 342 beschreibt auf S.3, Z.22 bis S.4, Z.56 Polysiloxane mit 1) mindestens zwei Polyetherresten A und B, wobei der Polyoxyalkylenrest A mit einem mittlerem Molgewicht von 600 bis 5500 aus 20 bis 100 Gew.-% Oxyethyleneinheiten und 80 bis 0 Gew.-% Oxypropyleneinheiten besteht, und der Polyoxyalkylenrest B mit einem mittleren Molgewicht von 700 bis 5000 aus 0 bis < 20 Gew.-% Oxyethyleneinheiten und 100 bis 80 Gew.-% Oxypropyleneinheiten besteht, und 2) an Si gebundenen Kohlenwasserstoffresten mit 6 bis 30 Kohlenstoffatomen.

Besonders geeignete Silikonderivate sind die unter dem INCl-Namen Dimethicone Copolyole oder Silikontenside bekannten Verbindungen wie zum Beispiel die unter den Markennamen Abil^{®} (Goldschmidt), Alkasil^{®} (Rhöne-Poulenc), Silicone Polyol Copolymer^{®} (Genesee), Belsil^{®} (Wacker), Silwet^{®} (Witco) oder Dow Corning^{®} (Dow Coming) erhältlich Diese beinhalten Verbindungen mit den CAS-Nummem 64365-23-7; 68937-54-2; 68938-54-5; 68937-55-3.

Besonders geeignete Silikonderivate sind die in der WO 99/04750 S.10, Z.24 bis S.12, Z.8 und S.13, Z.3 bis Z.34 beschriebenen Verbindungen.

WO 01/013884 S.24, Z.22 bis S.26, Z.41 beschreibt weitere besonders bevorzugte polyalkylenoxidhaltige Silikone.

Auf die vorgenannten Schriften und Zitate aus dem Stand der Technik wird in vollem Umfang Bezug genommen.

Besonders bevorzugt ist die Polymerisation des Gemisches aus den Komponenten a) bis e) in Gegenwart von, bezogen auf die Gesamtmenge der Komponenten a) bis e), 5 bis 25 Gew.-% Polyethylenglykol mit Molekulargewicht Mₙ von wenigstens 2000 bis höchstens 35000, bevorzugt höchstens 10000 und/oder 5 bis 25 Gew.-% Estern der (Meth)acrylsäure mit Polyethylenglykolmono-C₁₆-C₂₂-alkylethern.

Die Polymerisation des Gemisches aus den Komponenten a) bis e) wird bevorzugt in Gegenwart von höchstens 50, besonders bevorzugt höchstens 40 Gew.-% der Komponente f), bezogen auf die Summe der Mengen der Komponenten a) bis e) durchgeführt.

In einer weiteren Ausführungsform der Erfindung wird die Polymerisation des Gemisches aus den Komponenten a) bis e) wird in Gegenwart von 5 bis 70, bevorzugt 10 bis 50, besonders bevorzugt 20 bis 40 Gew.-% der Komponente f) bezogen auf die Summe der Mengen der Komponenten a) bis e) durchgeführt.

### Weitere Monomere e)

Die für die erfindungsgemäße Verwendung geeigneten Polymere können gewünschtenfalls 0 bis 30 Gew.-% weitere, von a) bis d) verschiedene, monoethylenisch ungesättigte Verbindungen einpolymerisiert enthalten.

Vorzugsweise sind diese weiteren Monomere e) ausgewählt unter Estern α,β-ethylenisch ungesättigter Mono- und Dicarbonsäuren mit C₁-C₃₀-Alkanolen, C₂-C₃₀-Alkandiolen und C₂-C₃₀-Aminoalkoholen, Amiden α,β-ethylenisch ungesättigter Mono- und Dicarbonsäuren mit C₂-C₃₀-Diaminen und C₂-C₃₀-Aminoalkoholen, die eine primäre oder sekundäre Aminogruppe aufweisen, Amiden α,β-ethylenisch ungesättig-ter Monocarbonsäuren und deren N-Alkyl- und N,N-Dialkylderivaten, N-Vinylamiden gesättigter Monocarbonsäuren, Estern von Vinylalkohol und Allylalkohol mit C₁-C₃₀-Monocarbonsäuren, Vinylethern, α,β-ethylenisch ungesättigten Mono- und Dicarbonsäuren, Vinylaromaten, Vinylhalogeniden, Vinylidenhalogeniden, C₁-C₈-Monoolefinen, nicht aromatischen Kohlenwasserstoffen mit mindestens zwei konjugier-ten Doppelbindungen und Mischungen davon.

Geeignete zusätzliche Monomere e) sind Methyl(meth)acrylat, Methylethacrylat, Ethyl(meth)acrylat, Ethylethacrylat, tert.-Butyl(meth)acrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tride-cyl(meth)-acrylat, Myristyl(meth)acrylat, Pentadecyl(meth)acrylat, Palmityl(meth)acrylat, Heptadecyl(meth)acrylat,Nonadecyl(meth)acrylat, Arrachinyl(meth)acrylat, Behenyl-(meth)acrylat, Lignocerenyl(meth)acrylat, Cerotinyl(meth)acrylat, Melissi-nyl(meth)-acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen da-von.

Geeignete zusätzliche Monomere e) sind auch die Ester von α,β-ethylenisch ungesättigten Mono- und Dicarbonsäuren mit Aminoalkoholen, vorzugsweise C₂-C₁₂-Aminoalkoholen. Diese können vorzugsweise am Aminstickstoff C₁-C₈-monoalkyliert oder -dialkyliert sein. Als Säurekomponente dieser Ester eignen sich z.B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Ester der Acrylsäure, Ester der Methacrylsäure und deren Gemische eingesetzt.

Geeignet sind weiterhin tert.-Butylaminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat, N,N-Dimethylaminocyclohexyl(meth)acrylat etc.

Geeignete zusätzliche Monomere e) sind auch N-Methyl(meth)acrylamid, N-Ethyl(meth)acrylamid, N-Propyl(meth)acrylamid, N-(n-Butyl)(meth)acrylamid, tert.-Butyl(meth)acrylamid, n-Octyl(meth)acrylamid, 1,1,3,3-Tetramethylbutyl(meth) acrylamid, Ethylhexyl(meth)acrylamid, n-Nonyl(meth)acrylamid, n-Decyl(meth)acrylamid, n-Undecyl(meth)acrylamid, Tride-cyl(meth)acrylamid, Myristyl(meth)acrylamid, Pentadecyl(meth)acrylamid, Palmi-tyl(meth)acrylamid, Heptadecyl(meth)acrylamid, Nonadecyl(meth)acrylamid, Arrachi-nyl(meth)acrylamid, Behenyl(meth)acrylamid, Lignocerenyl(meth)acrylamid, Ceroti-nyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stea-ryl(meth)acrylamid, Lauryl(meth)acrylamid und Mischungen davon.

Geeignete zusätzliche Monomere e) sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat etc.

Geeignete zusätzliche Monomere e) sind weiterhin N-[2-(dimethylamino)ethyl]-acrylamid, N-[2-(dimethylamino)ethyl]methacrylamid, N-[3-(dimethylamino)propyl]-acrylamid, N-[3-(dimethylamino)propyl]methacrylamid, N-[4-(dimethylamino)butyl]-acrylamid, N-[4-(dimethylamino)- butyl]methacrylamid, N-[2-(diethylamino)ethyl]-acrylamid, N-[4-(dimethylamino)cyclohexyl]acrylamid und N-[4-(dimethylamino)cyclohexyl]methacrylamid.

Geeignete zusätzliche Monomere e) sind weiterhin Acrylsäureamid, Methacrylsäureamid, N-Vinylformamid, N-Vinylacetamid, N-Vinylpropionamid und Mischungen davon.

Als zusätzliche Monomere e) eignen sich auch monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Malein-säureanhydrid, Itaconsäure, Citraconsäure, Mesaconsäure, Glutaconsäure, Aconitsäu-re, Fumarsäure, 2-Acrylamido-2-methylpropansulfonsäure, Vinylsulfonsäure, Vinylschwefelsäure, Vinylphosphorsäure, 10-Undecensäure, 4-Pentensäure, Zimtsäure, 3-Butensäure, 5-Hexensäure, 6-Heptensäure, 7-Octensäure, Citraconsäure, Mesaconsäure, Styrolsulfonsäure, Styrolschwefelsäure, Acrylsäure-(3-sulfopropyl)-ester, Itakonsäure-bis-(3-sulfopropyl)-ester, Methacrylsäure-(3-sulfopropyl)-ester, 3-Allyloxy-2-hydroxypropan-1-sulfonsäure, 2-Acrylamido-2-methylethan-sulfonsäure, Acrylsäure-(2-sulfoethyl)ester, ltakonsäure-bis-(2-sulfoethyl)-ester, Methacrylsäure-(2-sulfoethyl)-ester, Methacrylsäure-(3-sulfopropyl)-ester, 3-Allyloxy-2-hydroxypropan-1-sulfonsäure, 3-Allyloxy-2-hydroxyethan-1-sulfonsäure sowie deren Alkali- und Ammoniumsalze, insbesondere deren Natrium- und Kaliumsalze.
Insofern als Monomer e) anionische bzw. anionogene Verbindungen eingesetzt werden, werden diese zu höchstens 10, bevorzugt zu höchstens 5 und besonders bevorzugt zu höchstens 1 Gew.%, bezogen auf die Gesamtmenge der Komponenten a) bis e) einpolymerisiert. In einer ganz besonders bevorzugten Ausführungsform der Erfindung werden höchstens 0,5 Gew.-% von anionischen bzw. anionogenen Verbindungen als Monomere e) eingesetzt.

Geeignete zusätzliche Monomere e) sind weiterhin Ethylen, Propylen, Isobutylen, Butadien, Styrol, α-Methylstyrol, o-Chlorstyrol, Acrylnitril, Methacrylnitril, Vinylchlorid, Vinylidenchlorid, Vinylfluorid, Vinylidenfluorid, Ether aus Vinylalkohol und 1 bis 18 C-Atome aufweisenden Monoalkoholen, wie beispielsweise Methylvinylether, Ester aus Vinylalkohol und 1 bis 18 C-Atome aufweisenden Monocarbonsäuren, wie Vinylacetat, Vinylpropionat, Vinyl-n-butyrat, Vinyllaurat und Vinylstearat und Mischungen davon. Komponente e) ist am meisten bevorzugt ausgewählt aus der Gruppe bestehend aus Methylmethacrylat, Butylacrylat, Methacrylamid und deren Mischungen.

### Polymerisation

Erfindungsgemäß kann das zu polymerisierende Gemisch der Komponenten a) bis e) gegebenenfalls in Gegenwart von f) sowohl mit Hilfe von Radikale bildenden Initiatoren als auch durch Einwirkung energiereicher Strahlung, worunter auch die Einwirkung energiereicher Elektronen verstanden werden soll, polymerisiert werden.
Das erfindungsgemäße Verfahren ist bevorzugt dadurch gekennzeichnet, dass die wasserunlöslichen Initiatoren A und B so gewählt sind, dass ihre jeweiligen Zerfallstemperaturen wenigstens 70°C betragen.

Zur Herstellung der erfindungsgemäßen Copolymere werden wenigstens zwei Radikal-initiatoren eingesetzt, die eine im Wesentlichen unabhängige Initiierung in wenigstens zwei Phasen ermöglichen. Dabei werden Copolymere mit besonders geringen Restmonomergehalten erzielt werden.

Bevorzugt werden zur Copolymerisation wenigstens zwei Radikal-Initiatoren eingesetzt, deren Zerfallstemperaturen um wenigstens 10°C voneinander verschieden sind. Im Rahmen der Erfindung ist die Zerfallstemperatur definiert als die Temperatur, bei der 50% der Moleküle innerhalb von 1 Stunde in freie Radikale zerfallen, also die Halbwertszeit 1 Stunde beträgt.

In einer Ausführungsform der Erfindung erfolgt die Copolymerisation bei dieser Vorgehensweise bis zum Abschluss der Fällung des Copolymers bei einer Temperatur größer oder gleich der niedrigeren Zerfallstemperatur und kleiner der höheren Zerfallstemperatur, und nach der Fällung erfolgt eine weitere Umsetzung bei einer Temperatur größer oder gleich der höheren Zerfallstemperatur.

In einer bevorzugten Ausführungsform umfasst das erfindungsgemäße Verfahren eine erste Polymerisationsphase bei einer ersten Polymerisationstemperatur und eine zweite Polymerisationsphase bei einer zweiten Polymerisationstemperatur oberhalb der ersten Polymerisationstemperatur, wobei zur Polymerisation wenigstens zwei Initiatoren eingesetzt werden, deren Halbwertszeiten bei der ersten Polymerisationstemperatur sich so unterscheiden, dass wenigstens einer dieser Initiatoren während der ersten Polymerisationsphase in Radikale zerfällt und wenigstens einer dieser Initiatoren während der ersten Polymerisationsphase im Wesentlichen nicht in Radikale zerfällt und während der zweiten Polymerisationsphase in Radikale zerfällt. Vorzugsweise beginnt bei dieser Vorgehensweise die zweite Polymerisationsphase im Wesentlichen nach Fällung des Copolymers. Unter "im Wesentlichen" nach Fällung des Copolymers wird verstanden, dass das Copolymer vorzugsweise zu mindestens 80 Gew.-%, vorzugsweise mindestens 90 Gew.-%, insbesondere wenigstens 95 Gew.-%, bezogen auf das Gesamtgewicht des Copolymers, in gefällter Form vorliegt.

Die Halbwertszeit eines Initiators kann nach üblichen, dem Fachmann bekannten Verfahren bestimmt werden, wie z. B. in der Druckschrift "Initiators for high polymers", Akzo Nobel, Nr. 10737, beschrieben. Vorzugsweise liegt die Halbwertszeit des ersten Polymerisationsinitiators bei der ersten Polymerisationstemperatur und des zweiten Polymerisationsinitiators bei der zweiten Polymerisationstemperatur in einem Bereich von etwa 1 Minute bis 3 Stunden, besonders bevorzugt 5 Minuten bis 2,5 Stunden. Gewünschtenfalls können auch kürzere Halbwertszeiten z. B. von 1 Sekunde bis 1 Minute oder längere Halbwertszeiten als 3 Stunden zum Einsatz kommen, solange sichergestellt ist, dass der/die bei der höheren Temperatur zerfallende(n) Initiator(en) im Wesentlichen während der zweiten Polymerisationsphase in Radikale zerfällt.

Zusätzlich zu der ersten und zweiten Polymerisationsphase können weitere Polymerisationsphasen bei davon verschiedenen Polymerisationstemperaturen angewandt werden. So ist es z. B. möglich, eine erste Polymerisationsphase bei einer ersten Polymerisationstemperatur durchzuführen, die so gewählt ist, dass eine kontrollierte Polymerisation (d. h. z. B. unter Vermeidung eines unerwünschten Temperaturanstiegs durch die Reaktionswärme, einer zu hohen Reaktionsgeschwindigkeit, etc.) erfolgt. Anschließend kann sich z. B. eine Nachpolymerisation bei einer Temperatur anschließen, die oberhalb der ersten und unterhalb der zweiten Polymerisationstemperatur liegt und die so gewählt ist, dass der/die bei der höheren Temperatur zerfallende(n) Initiator(en) im Wesentlichen nicht in Radikale zerfallen. Nach Abschluss dieser Nachpolymerisation, zu der gewünschtenfalls nochmals der bei der niedrigeren Temperatur zerfallende Initiator und/oder ein anderer unter den Bedingungen der Nachpolymerisation zerfallender Initiator zugegeben werden kann, kann sich dann die zweite Polymerisationsphase anschließen.

Insbesondere ist das Verfahren dadurch gekennzeichnet, dass sich die Zerfallstemperaturen der Initiatoren A und B um wenigstens 10°C, bevorzugt um wenigstens 15, weiter bevorzugt um wenigstens 20 und insbesondere um wenigstens 25°C unterscheiden.

Wasserunlöslich bedeutet im Rahmen der vorliegenden Erfindung, dass weniger als 10, bevorzugt weniger als 1g des Initiators in 1 Liter Wasser bei Standardbedingungen für das menschliche Auge klar löslich sind.

Die wasserunlöslichen Initiatoren A und B sind bevorzugt ausgewählt aus wasserunlöslichen Diazo- und Peroxidverbindungen.
Für das erfindungsgemäße Verfahren geeignete Initiatoren A und B sind beispielsweise ausgewählt aus Dibenzoylperoxid, Succinylperoxid, Di-tert.-butylperoxid, tert.-Butylperbenzoat, tert.-Butylperpivalat, tert.-Butylperoctoat, tert.-Butylperoxy-2-ethylhexanoat, tert.-Butylpermaleinat, Benzoylperoxid, tert.-Amylperoxipivalat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Ditert.-Amylperoxid, tert.-Butylhydroperoxid, Azobisisobutyronitril, Dimethyl 2,2'-azobis(2-methylpropionat) oder 2,2'-Azobis(2-methyl-butyronitril).
Bevorzugt sind die Radikal-Initiatoren ausgewählt aus der Gruppe bestehend aus Benzoylperoxid, tert.-Amylperoxipivalat, Dimethyl 2,2'-azobis(2-methylpropionat), 2,2'-Azobis(2-methylbutyro-nitril), 2,2'-Azobis(2-methylpropionamid) und tert-Butyl peroxy-2-ethylhexanoat.
Geeignete Initiatoren sind weiterhin 2,2'-Azobis(4-methoxy-2,4-dimethylvaleronitril), 2,2'-Azobis(2,4-dimethyl valeronitril), Dimethyl 2,2'-azobis(2-methylpropionat), 2,2'-Azobis(2-methylbutyronitril), 1,1'-Azobis(cyclohexan-1-carbonitril), 2,2'-Azobis[N-(2-propenyl)-2-methylpropionamid], 1-[(cyano-1-methylethyl)azo] formamid, 2,2'-Azobis(N-butyl-2-methylpropionamid), 2,2'-Azobis(N-cyclohexyl-2-methylpropionamid). Die vorgenannten Initiatoren sind beispielsweise als Wako^{®}- bzw. Trigonox^{®}-Marken kommerziell erhältlich.

In einer Ausführungsform der Erfindung ist es bevorzugt, für die Polymerisation bis zu einem Zeitpunkt, an dem wenigstens 80, bevorzugt wenigstens 90, besonders bevorzugt wenigstens 95 und insbesondere wenigstens 99 Gew.-% der eingesetzten Monomere a) bis e) polymerisiert sind, als Initiator A denjenigen Initiator zu wählen, dessen Zerfallstemperatur um wenigstens 10°C geringer ist als die des Initiators B.

Der Initiator B wird bevorzugt für die sogenannte Nachpolymerisation eingesetzt. Die Nachpolymerisation wird durchgeführt; nachdem wenigstens 80, bevorzugt wenigstens 90, besonders bevorzugt wenigstens 95 und insbesondere wenigstens 99 Gew.-% der eingesetzten Monomere a) bis e) polymerisiert sind.

Die Nachpolymerisation wird bevorzugt bei einer Temperatur durchgeführt, die größer ist als die Temperatur, bei der die Hauptpolymerisation durchgeführt worden ist.
In einer Ausführungsform ist die Temperatur, bei der die Nachpolymerisation durchgeführt wird, wenigstens 5°C, bevorzugt wenigstens 10°C, besonders bevorzugt wenigstens 15 und insbesondere wenigstens 20°C höher als die Temperatur der Hauptpolymerisation.

Die Menge des wenigstens einen für die Hauptpolymerisation (erste Reaktionsphase) bevorzugt eingesetzten Initiators A beträgt vorzugsweise von 0,001 bis 2,0, weiter bevorzugt 0,01 bis 1,5, besonders bevorzugt 0,05 bis 1,0 und insbesondere 0,1 bis 0,5 Gew.-%, bezogen auf die Gesamtmenge der eingesetzten Monomere a) bis e).

Die Menge des wenigstens einen für die Nachpolymerisation (zweite Reaktionsphase) bevorzugt eingesetzten Initiators B beträgt vorzugsweise von 0,001 bis 2,5, weiter bevorzugt 0,01 bis 2,0, besonders bevorzugt 0,1 bis 1,5 und insbesondere 0,3 bis 1,0 Gew.-%, bezogen auf die Gesamtmenge der eingesetzten Monomere a) bis e).

In einer bevorzugten Ausführungsform der Erfindung ist die Gewichtsmenge von Initiator B größer als die Menge von Initiator A. Besonders bevorzugt ist es, wenn die Gewichtsmenge von B wenigstens das 1,5-fache, weiter bevorzugt wenigstens das 2-fache, besonders bevorzugt wenigstens das 3-fache der Gewichtsmenge von Initiator A beträgt.

Bei einer bevorzugten Initiatorkombination handelt es sich bei dem bei der niedrigeren Temperatur zerfallenden Initiator um tert-Butyl peroxy-2-ethylhexanoat (CAS-Nr. 3006-82-4; Trigonox^{®}21S) und ist der bei der höheren Temperatur zerfallende Initiator ausgewählt unter tert.-Butylperoxypivalat (z. B. Luperox^{®}11 M75 der Fa. Atochem), tert.-Butylperoctoat, Lauroylperoxid (LPO, CAS-Nr. 105-74-8) oder 2,5 Dimethyl 2,5 bis(t-butylperoxy)hexan (Trigonox^{®}101).

Die Hauptpolymerisation (erste Reaktionsphase) erfolgt im Temperaturbereich von 40 bis 200°C, bevorzugt im Bereich von 50 bis 140°C, besonders bevorzugt im Bereich von 70 bis 120°C. Sie wird üblicherweise unter atmosphärischem Druck durchgeführt, kann jedoch auch unter vermindertem oder erhöhtem Druck, vorzugsweise von 1 bis 5 bar, ablaufen.

Die Nachpolymerisation (zweite Reaktionsphase) erfolgt im Temperaturbereich von 50 bis 220°C, bevorzugt im Bereich von 60 bis 150°C, besonders bevorzugt im Bereich von 80 bis 130°C. Sie wird üblicherweise unter atmosphärischem Druck durchgeführt, kann jedoch auch unter vermindertem oder erhöhtem Druck, vorzugsweise von 1 bis 5 bar, ablaufen.

Das erfindungsgemäße Verfahren kann halbkontinuierlich durchgeführt werden, indem man zunächst einen Teil, z.B. etwa 10 % des zu polymerisierenden Gemisches aus der Komponente f), Lösungsmittel, den Monomeren a) bis e) und Initiator A vorlegt, das Gemisch auf Polymerisationstemperatur erhitzt und nach dem Anspringen der Polymerisation den Rest der zu polymerisierenden Mischung nach Fortschritt der Polymerisation zugibt.
Die Polymere können auch dadurch erhalten werden, daß man die Komponente f) vor legt, auf Polymerisationstemperatur erwärmt und das Gemisch der Monomeren a) bis e) und Initiator A entweder auf einmal, absatzweise oder vorzugsweise kontinuierlich zufügt und polymerisiert.

Geeignete Lösemittel für das erfindungsgemäße Verfahren sind beispielsweise Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin, Dioxan, Butylacetat, Ethylacetat und Toluol, wobei Ethylacetat, Butylacetat und deren Mischungen besonders bevorzugt sind.

Besonders bevorzugt wird die Polymerisation als Fällungspolymerisation durchgeführt.

Beim erfindungsgemäßen Verfahren handelt es sich bevorzugt um eine Fällungspolymerisation. Für diese Polymerisation werden Lösungsmittel verwendet, in denen die Ausgangsstoffe für die Polymerisation löslich und das entstehende Polymer unlöslich sind. Geeignete Lösungsmittel sind beispielsweise aromatische Kohlenwasserstoffe wie Toluol, Xylole, Benzol oder aliphatische Kohlenwasserstoffe wie n-Alkane, Cyclohexan, Ester der Essigsäure wie Ethylacetat, Butylacetat, Ether wie z.B. Diethylether, Dipropylether, Dibutylether, Methyl tert.-butylether, Diethylenglykoldimethylether, Ketone wie Aceton, Methylethylketon und Mischungen dieser Lösungsmittel. Mischungen von z.B. Ethylacetat und Butylacetat sind besonders geeignet, da in diesem Lösungsmittelgemisch die Polymere in leicht abtrennbarer Form anfallen (Sedimentation ist beschleunigt) und außerdem die Reaktionstemperatur in Mischungen von Butylacetat und Ethylacetat oberhalb der Siedetemperatur von Ethylacetat gewählt werden kann bei gleichzeitiger Siedekühlung durch siedendes Ethylacetat.

Die Polymerisation wird bevorzugt in Gegenwart von weniger als als 69 Gew.-% Cyclohexan und weniger als 12 Gew.-% Wasser, bezogen auf die Gesamtmenge aller während der Polymerisation gegenwärtigen Komponenten, durchgeführt. Bevorzugt wird die Polymerisation in Gegenwart von weniger als 50, besonders bevorzugt weniger als 40 und insbesondere im Bereich von 30 bis 0 Gew.-% Cyclohexan durchgeführt. Bevorzugt wird die Polymerisation in Gegenwart von weniger als 10, besonders bevorzugt weniger als 8 und insbesondere im Bereich von 5 bis 0 Gew.-% Wasser durchgeführt.

In einer bevorzugten Ausführungsform besteht das für die Polymerisation verwendete Lösungsmittel zu wenigstens 30, bevorzugt zu wenigstens 50 und insbesondere zu wenigstens 70 Gew.-% aus Ethylacetat oder n-Butylacetat oder deren Mischungen.
In einer Ausführungsform der Erfindung besteht das Lösungsmittel zu 80 bis 100, bevorzugt zu 90 bis 100 Gew.-% aus Ethylacetat und/oder n-Butylacetat.

Die Fällungspolymerisation wird üblicherweise bei Temperaturen von 20 bis 150°C, bevorzugt 40 bis 120°C, insbesondere 60 bis 100°C durchgeführt.

Die Fällungspolymerisation wird üblicherweise bei Drücken von 1 bis 15 bar, insbesondere 1 bis 6 bar durchgeführt.

Das Lösungsmittel bzw. Lösungsmittelgemisch bestimmt durch die entsprechenden Siedetemperaturen die maximale Reaktionstemperatur sofern unter Atmosphärendruck polymerisiert wird. Polymerisationen unter Druck sind aber ebenfalls möglich.

Generell kann die Fällungspolymerisation bei Feststoffgehalten bis ca. 40 Gew.-% durchgeführt werden. Bevorzugt wird ein Bereich zwischen 25 und 40 Gew.%. Besonders bei hohen Feststoffgehalten ist es ratsam, die Polymerisation in Gegenwart eines Schutzkolloid-Polymers durchzuführen. Geeignete Schutzkolloid-Polymere sind solche, die sich gut in den verwendeten Lösungsmitteln lösen und nicht mit den Monomeren reagieren. Als Schutzkolloide geeignete Polymere sind z.b. Copolymere der Maleinsäure mit Vinylalkylethern und/oder Olefinen mit 8 bis 20 C-Atomen oder entsprechende Copolymere der Maleinsäurehalbester mit C10-C20-Alkoholen oder auch Mono- und Diamide der Maleinsäure mit C10 -C20-Alkylaminen sowie Polyvinylalkoholether mit Alkylgruppen, die 1 bis 20 C-Atome tragen oder auch Polyvinylmethyl, -ethyl, -isobutyl oder -octadecylether. Die Menge an verwendetem Schutzkolloid-Polymer ist normalerweise im Bereich von 0,05 bis 4 Gew-% (bezogen auf Monomere), vorzugsweise von 0,1 bis 2 Gew-%. Es ist oft vorteilhaft, Mischungen mehrerer Schutzkolloid-Polymere zu verwenden.

Die Polymerisation wird durchgeführt, in dem man Lösungsmittel, Komponente f), Schutzkolloidpolymer und eventuell Vernetzer c) vorlegt, aufheizt, und die Polymerisation durch Zugabe von Inititator und Monomeren a), b), d) und e) (eventuell gelöst im gleichen Lösungsmittel oder Lösungsmittelgemisch) durchführt. Man kann aber auch Teilmengen der Monomere und des Inititators A vorlegen (z.B. 10%), dieses Gemisch auf Polymerisationstemperatur erhitzen und nach Beginn der Reaktion den Rest der zu polymerisierenden Mischung nach Fortschritt der Polymeristion zugegen. Ebenso ist es möglich, den verwendeten Vernetzer in Teilmengen vorzulegen und den Rest zusammen mit den restlichen Komponenten zuzugeben. Bei niedrigeren Feststoffgehalten ist es auch denkbar, sämtliche Einsatzstoffe in einer Batchreaktion vorzulegen.

In einer bevorzugten Ausführungsform wird die Polymerisation zur Herstellung der für die erfindungsgemäße Verwendung geeigneten Polymerisate in Zulauffahrweise durchgeführt. Dabei werden einzelne oder alle Reaktionsteilnehmer ganz oder teilweise, absatzweise oder kontinuierlich, gemeinsam oder in getrennten Zuläufen zu einer Reaktionsmischung gegeben.

Getrennte Zuläufe sind beispielsweise dann vorteilhaft, wenn sich die Löslichkeiten der Komponenten a) bis e) in bestimmten Lösungsmitteln deutlich unterscheiden. Beispielsweise ist bei der Copolymerisation Vinylimidazol als a) und Methacrylamid als b) sowie den weiteren Komponenten c) bis e) ein Verfahren mit getrennten Zuläufen vorteilhaft, da sich Vinylimidazol und Methacrylamid in ihren Löslichkeiten deutlich unterscheiden.

Monomere und Initiator werden im allgemeinen in einer Zeit von 1 bis 10 Stunden zudosiert, bevorzugt von 2 bis 5 Stunden.

Bei der Herstellung der Polymere können gegebenenfalls auch andere Polymere, wie zum Beispiel Polyamide, Polyurethane, Polyester, Homo- und Copolymere von ethylenisch ungesättigten Monomeren, zugegen sein. Beispiele für solche zum Teil auch in der Kosmetik eingesetzten Polymeren sind die unter den Handelsnamen bekannten Polymere Amerhold™, Ultrahold™, Ultrahold Strong™, Luviflex™ VBM, Luvimer™, Acronal™, Acudyne™, Stepanhold™, Lovocryl™, Versatyl™, Amphomer™ oder Eastma AQ™, Luviset™-Marken, Sokalan™-Marken, Luviquat™-Marken.

Das ausgefallene Polymer wird nach dem Nachpolymerisationsschritt aus der Reaktionsmischung isoliert, wofür jede übliche Methode zur Isolierung der Polymere in der konventionellen Fällungspolymerisation verwendet werden kann. Solche Methoden sind Filtration, Zentrifugation, Eindampfen des Lösungsmittels oder Kombinationen dieser Methoden. Zur weiteren Reinigung des Polymers von nichtpolymerisierten Bestandteilen kann das Polymer gewaschen werden. Dafür kann man prinzipiell die gleichen Lösungsmittel verwenden wie sie für die Polymerisation geeignet sind. Aufgrund des vorteilhaften erfindungsgemäßen Verfahrens ist die Menge der Restmonomere im Vergleich zu bekannten Verfahren sehr gering.

Soll das Polymer getrocknet werden, so empfiehlt es sich, nach der Polymerisation bzw. nach der Alkylierung einen Lösungsmitteltausch vorzunehmen und für die Trocknung niedrig siedende Lösungsmittel wie z.B. Aceton zu verwenden.

Bei der Polymerisation können auch Substanzen verwendet werden, mit deren Hilfe das Molekulargewicht der Polymere gesteuert werden kann und die üblicherweise als Regler bezeichnet werden.

### Regler

Die radikalische Polymerisation des Monomergemischs kann in Gegenwart mindestens eines Reglers erfolgen. Regler werden vorzugsweise in einer Einsatzmenge von 0,0005 bis 5 Gew.%, besonders bevorzugt von 0,001 bis 2,5 Gew.-% und insbesondere von 0,01 bis 1,5 Gew.%, bezogen auf das Gesamtgewicht der Komponente a) bis e), eingesetzt.

Als Regler (Polymerisationsregler) werden allgemein Verbindungen mit hohen Übertragungskonstanten bezeichnet. Regler beschleunigen Kettenübertragungsreaktionen und bewirken damit eine Herabsetzung des Polymerisationsgrades der resultierenden Polymeren, ohne die Bruttoreaktions-Geschwindigkeit zu beeinflussen.

Bei den Reglern kann man zwischen mono-, bi- oder polyfunktionalen Regler unterscheiden je nach Anzahl der funktionellen Gruppen im Molekül, die zu einen oder mehreren Kettenübertragungsreaktionen führen können. Geeignete Regler werden beispielsweise ausführlich beschrieben von K.C. Berger und G. Brandrup in J. Brandrup, E.H. lmmergut, Polymer Handbook, 3. Aufl., John Wiley & Sons, New York, 1989, S. II/81 - II/141.

Als Regler eignen sich beispielsweise Aldehyde wie Formaldehyd, Acetaldehyd, Propionaldehyd, n-Butyraldehyd, Isobutyraldehyd.

Ferner können auch als Regler eingesetzt werden: Ameisensäure, ihre Salze oder Ester, wie Ammoniumformiat, 2,5-Diphenyl-1-hexen, Hydroxylammoniumsulfat, und Hydroxylammoniumphosphat.

Weitere geeignete Regler sind Halogenverbindungen, z.B. Alkylhalogenide, wie Tetrachlormethan, Chloroform, Bromtrichlormethan, Bromoform, Allylbromid, und Benzylverbindungen, wie Benzylchlorid oder Benzylbromid.

Weitere geeignete Regler sind Allylverbindungen, wie z.B. Allylalkohol, funktionalisierte Allylether, wie Allylethoxylate, Alkylallylether, oder Glycerinmonoallylether.

Bevorzugt werden als Regler Verbindungen eingesetzt, die Schwefel in gebundener Form enthalten.

Verbindungen dieser Art sind beispielsweise anorganische Hydrogensulfite, Disulfite und Dithionite oder organische Sulfide, Disulfide, Polysulfide, Sulfoxide und Sulfone. Dazu zählen Di-n-butylsulfid, Di-n-octylsulfid, Diphenylsulfid, Thiodiglykol, Ethylthioethanol, Diisopropyldisulfid, Di-n-butyldisulfid, Di-n-hexyldisulfid, Diacetyldisulfid, Diethanolsulfid, Di-t-butyltrisulfid, Dimethylsulfoxid, Dialkylsulfid, Dialkyldisulfid und/oder Diarylsulfid.

Besonders bevorzugt sind organische Verbindungen, die Schwefel in gebundener Form enthalten.

Bevorzugt als Polymerisationsregler eingesetzte Verbindungen sind Thiole (Verbindungen, die Schwefel in Form von SH-Gruppen erhalten, auch als Mercaptane bezeichnet). Bevorzugt sind als Regler mono-, bi- und polyfunktionale Mercaptane, Mercaptoalkohole und/oder Mercaptocarbonsäuren.

Beispiele für diese Verbindungen sind Allylthioglykolate, Ethylthioglykolat, Cystein, 2-Mercaptoethanol, 1,3-Mercaptopropanol, 3-Mercaptopropan-1,2-diol, 1,4-Mercaptobutanol, Mercaptoessigsäure, 3-Mercaptopropionsäure, Mercaptobernsteinsäure, Thioglycerin, Thioessigsäure, Thioharnstoff und Alkylmercaptane wie n-Butylmercaptan, n-Hexylmercaptan oder n-Dodecylmercaptan.

Besonders bevorzugte Thiole sind Cystein, 2-Mercaptoethanol, 1,3-Mercaptopropanol, 3-Mercaptopropan-1,2-diol, Thioglycerin, Thioharnstoff.

Beispiele für bifunktionale Regler, die zwei Schwefel in gebundener Form enthalten sind bifunktionale Thiole wie z.B. Dimercaptopropansulfonsäure (Natrium Salz), Dimercaptobemsteinsäure, Dimercapto-1-propanol, Dimercaptoethan, Dimercaptopropan, Dimercaptobutan, Dimercaptopentan, Dimercaptohexan, Ethylenglykol-bisthioglykolate und Butandiol-bis-thioglykolat.

Beispiele für polyfunktionale Regler sind Verbindungen, die mehr als zwei Schwefel in gebundener Form enthalten. Beispiele hierfür sind trifunktionale und/oder tetrafunktionale Mercaptane.

Bevorzugte trifunktionale Regler sind trifunktionale Mercaptane, wie z.B. Trimethylolpropan-tris(2-mercaptoethanat, Trimethylolpropan-tris(3-mercaptopropionat), Trimethylolpropan-tris(4-mercaptobutanat), Trimethylolpropan-tris(5-mercaptopentanat), Trimethylolpropan-tris(6-mercaptohexanat), Trimethylolpropan-tris(2-mercaptoacetat), Glycerylthioglycolat, Glycerylthiopropionat, Glycerylthioethylat, Glycerylthiobutanat, 1,1,1-Propanetriyl-tris-(mercaptoacetat), 1,1,1-Propanetriyl-tris-(mercaptoethanat), 1,1,1-Propanetriyl-tris-(mercaptoproprionat), 1,1,1-Propanetriyl-tris-(mercaptobutanat), 2-hydroxmethyl-2-methyl-1,3-propandiol-tris-(mercaptoacetat), 2-hydroxmethyl-2-methyl-1,3-propandiol-tris-(mercaptoethanat), 2-hydroxmethyl-2-methyl-1,3-propandiol-tris-(mercaptopropionat), 2-hydroxmethyl-2-methyl-1,3-propandiol-tris-(mercaptobutanat).

Besonders bevorzugte trifunktionale Regler sind Glycerylthioglycolat, Trimethylolpropan-tris(2-mercaptoacetat), 2-hydroxmethyl-2-methyl-1,3-propandiol tris-(mercaptoacetat).

Bevorzugte tetrafunktionale Mercaptane sind Pentaerythrit-tetrakis-(2-mercaptoacetat), Pentaerythrit-tetrakis-(2-mercaptoethanat), Pentaerythrit-tetrakis(3-mercaptopropionat), Pentaerythrit-tetrakis-(4-mercaptobutanat), Pentaerythrit-tetrakis(5-mercaptopentanat), Pentaerythrit-tetrakis-(6-mercaptohexanat).

Als weitere polyfunktionale Regler eignen sich Si-Verbindungen, die durch Umsetzung von Verbindungen der Formel (IVa) entstehen. Weiterhin eignen sich als polyfunktionale Regler Si-Verbindungen der Formel 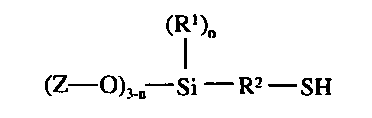 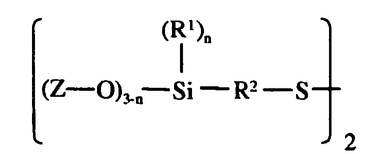 in der
- n: ein Wert von 0 bis 2 ist,
- R¹: eine C₁-C₁₆-Alkylgruppe oder Phenylgruppe bedeutet,
- R²: eine C₁-C₁₈-Alkylgruppe, die Cyclohexyl- oder Phenylgruppe bezeichnet,
- Z: für eine C₁-C₁₈ Alkylgruppe, C₂-C₁₈-Alkylengruppe oder C₂-C₁₈-Alkinylgruppe steht, deren Kohlenstoffatome durch nicht benachbarte Sauerstoff- oder Halo- genatome ersetzt sein können, oder für eine der Gruppen 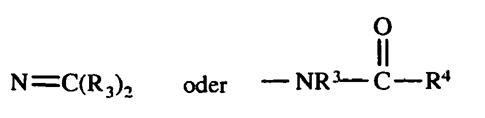
in denen
- R₃: eine C₁-C₁₂-Alkylgruppe bedeutet und
- R₄: eine C₁-C₁₈-Alkylgruppe bezeichnet.

Alle genannten Regler können einzeln oder in Kombination miteinander eingesetzt werden.

In einer Ausführungsform der Erfindung wird kein Regler eingesetzt.

Bevorzugt sind Polymere, die durch erfindungsgemäße Polymerisation von
a) 99,99 bis 10 Gew.-% Komponente a), insbesondere N-Vinylimidazol,
b) 0 bis 90 Gew.-% Komponente b), insbesondere N-Vinylpyrrolidon,
c) 0,01 bis 5 Gew.-% eines Vernetzers c), insbesondere Pentaerythrittriallylether,
d) 0 bis 15 Gew.-% einer Komponente d), insbesondere Octadecylvinylether und/oder Stearylmethacrylat
e) 0 bis 30 Gew.-% einer Komponente e), insbesondere Methytmethacrylat in Gegenwart von
f) 0 bis 70 Gew.-%, bezogen auf die Summe der Komponenten a) bis e) einer polyetherhaltigen Verbindung f), insbesondere Polyethylenglykol,
dadurch gekennzeichnet,
dass im Verlauf des Verfahrens wenigstens zwei unterschiedliche wasserunlösliche Initiatoren A und B eingesetzt werden.

Weiter bevorzugt sind Polymere, die erhältlich sind durch radikalische Pfropfcopolymerisation von
a) 97,95 bis 40 Gew.-% Komponente a), insbesondere N-Vinylimidazol,
b) 1 bis 60 Gew.-% Komponente b), insbesondere N-Vinylpyrrolidon,
c) 0,05- bis 2 Gew.-% eines Vernetzers c), insbesondere Pentaerythrittriallylether,
d) 1 bis 15 Gew.-% einer Komponente d) insbesondere Octadecylvinylether und/oder Stearylmethacrylat
e) 0 bis 20 Gew.-% einer Komponente e), insbesondere Methylmethacrylat in Gegenwart von
f) 0 bis 50 Gew.-% bezogen auf die Summe der Komponenten a) bis e) einer polyetherhaltigen Verbindung f), insbesondere Polyethylenglykol,
dadurch gekennzeichnet,
dass im Verlauf des Verfahrens wenigstens zwei unterschiedliche wasserunlösliche Initiatoren A und B eingesetzt werden.

Ganz besonders bevorzugt sind Polymere, die erhältlich sind durch radikalische Pfropfcopolymerisation von
a) 96,9 bis 60 Gew.-% Komponente a), insbesondere N-Vinylimidazol,
b) 1 bis 40 Gew.-% Komponente b), insbesondere N-Vinylpyrrolidon und/oder Methacrylamid
c) 0,1 bis 1 Gew.-% eines Vernetzers c), insbesondere Pentaerythrittriallylether,
d) 2 bis 10 Gew.-% einer Komponente d) insbesondere Octadecylvinylether und/oder Stearylmethacrylat und/oder Behenylacrylat und/oder Ester der (Meth)acrylsäure mit Polyethylenglykolmono-C₁₆-C₂₂-alkylethern und/oder Laurylacrylat,
e) 0 bis 10 Gew.-% einer Komponente e) insbesondere Methylmethacrylat in Gegenwart von
f) 0 bis 35 Gew.-% bezogen auf die Summe der Komponenten a) bis e) einer polyetherhaltigen Verbindung f), insbesondere Polyethylenglykol und/oder Polyethylenglykolmono-C₁₆-C₂₂-alkylether und/oder Polytetrahydrofuran, mit der Maßgabe, dass sich die Mengen der Komponenten a) bis e) zu 100 Gew.-% addieren, dadurch gekennzeichnet,
   dass im Verlauf des Verfahrens wenigstens zwei unterschiedliche wasserunlösliche Initiatoren A und B eingesetzt werden.

### Neutralisation

Vor der Verwendung als Rheologiemodifier in wässrigen und/oder alkoholischen Zubereitung kann das Polymer nach der Polymerisation und vor oder nach der Filtration neutralisiert werden. Je nach der Wahl der Monomere a) bis e) können zur Neutralisation Säuren oder Basen notwendig sein. Als Neutralisationsmittel für basische Gruppen tragende Monomere werden organische oder anorganische Säuren eingesetzt.
Als mögliche organische Säuren seien ein- und mehrwertige, gegebenenfalls substituierte aliphatische und aromatische Carbonsäuren, ein- und mehrwertige, gegebenenfälls substituierte aliphatische und aromatische Sulfonsäuren oder ein- und mehrwertige, gegebenenfalls substituierte aliphatische und aromatische Phosphonsäuren, säuregruppen tragende Polymere oder Ascorbinsäure genannt.
Bevorzugte organische Säuren sind Hydroxycarbonsäuren, d.h. Derivate der Carbonsäuren, bei denen ein oder mehrere H-Atome durch Hyroxylgruppen ersetzt sind.
Als Beispiele für Hydroxycarbonsäuren seien Glykolsäure, Milchsäure, Weinsäure und Citronensäure genannt. Als bevorzugte anorganische Säuren seien Phosphorsäure, phosphorige Säure, Schwefelsäure, schweflige Säure und Salzsäure genannt.

Als Neutralisationsmittel für Säuregruppen tragende Monomere können zum Beispiel Mineralbasen wie Natriumcarbonat, Alkalihydroxide wie NaOH oder bevorzugt KOH sowie Ammoniak, organische Basen wie Aminoalkohole speziell 2-Amino-2-Methyl-1-Propanol, Monoethanolamin Diethanolamin, Triethanolamin, Triisopropanolamin, Tri[(2-hydroxy)1-Propyl]amin, 2-Amino-2-Methyl-1,3-Propandiol, 2-Amino-2-hydroxymethyl-1,3-Propandiol sowie Diamine, wie zum Beispiel Lysin, verwendet werden. Weiterhin ist es auch möglich, als Neutralisationsmittel Wasserglas einzusetzen, wie in der DE-A 103 54 015, [0008] bis [0010] beschrieben. Auch ist es möglich, die in der WO 03/99253, S.2, Zeile 5 bis S. 3, Zeile 6 beschriebenen Amine als Neutralisierungsmittel vorteilhaft einzusetzen.

### Modifizierung der rheologischen Eigenschaften

Unter Modifizierung der rheologischen Eigenschaften wird ganz allgemein die Änderung des Verformungs- und Fließverhaltens von Materie verstanden. Die bedeutendsten rheologischen Eigenschaften sind Viskosität, Thixotropie, Strukturviskosität, Rheopexie und Dilatanz. Diese Begriffe sind dem Fachmann bekannt.

Unter Viskosität versteht man üblicherweise die "Zähigkeit" einer Flüssigkeit. Sie resultiert aus den zwischenmolekularen Kräften in einer Flüssigkeit, ist also abhängig von Kohäsion (intramolekular) und Adhäsion (intermolekular). Die Viskosität charakterisiert das Fließverhalten einer Flüssigkeit. Hohe Viskosität bedeutet Dickflüssigkeit, eine niedrige dagegen Dünnflüssigkeit.

Unter Thixotropie versteht man üblicherweise die Eigenschaft eines Fluids, nach einer Scherung eine niedrigere Viskosität zu zeigen und die ursprüngliche Viskosität bei Stillstand wieder aufzubauen.

Unter Rheopexie versteht man üblicherweise die Eigenschaft eines Fluids, nach einer Scherung eine höhere Viskosität zu zeigen. Dieses Verhalten ist eng verwandt mit der Dilatanz, bei der die Viskosität nur während der Scherung höher ist.

Unter Modifizierung der Rheologie wird im Rahmen dieser Erfindung insbesondere die Erhöhung der Viskosität von Flüssigkeiten verstanden, üblicherweise auch als "Verdickung" bezeichnet. Diese Viskositätserhöhung kann bis zur Entstehung von Gelen oder Festkörpern reichen.

### Wässrige, alkoholische oder wässrig/alkoholische Zusammensetzungen

Ein weiterer Gegenstand der Erfindung sind kosmetische, dermatologische oder pharmazeutische Zubereitungen und Mittel enthaltend wenigstens ein durch das erfindungsgemäße Verfahren erhältliches Polymer.
Bevorzugt sind als Mittel wässrige, alkoholische oder wässrig/alkoholische Zusammensetzungen, die das wenigstens eine Polymer in einer Menge im Bereich von 0,01 bis 20, besonders bevorzugt von 0,05 bis 10, ganz besonders bevorzugt von 0,1 bis 7 Gew.-% eingesetzt enthalten.
Unter wässrigen Zusammensetzungen werden Zusammensetzungen verstanden, die wenigstens 40, bevorzugt wenigstens 50 und insbesondere wenigstens 60 Gew.-% Wasser und gleichzeitig weniger als 20 Gew.-% Alkohol umfassen.
Unter alkoholischen Zusammensetzungen werden Zusammensetzungen verstanden, die wenigstens 40, bevorzugt 50 Gew.-% ind insbesondere wenigstens 60 Gew.-% eines oder mehrerer Alkohole und gleichzeitig weniger als 20 Gew-% Wasser umfassen.
Unter wässrig/alkoholischen Zusammensetzungen werden Zusammensetzungen, die wenigstens 20 Gew.-% Wasser und gleichzeitig wenigstens 20 Gew.-% Alkohol enthalten.

Eine bevorzugte Ausführungsform der Erfindung sind wässrig/alkoholische Zusammensetzungen mit vorzugsweise wenigstens 50 Gew.-% Wasser und vorzugsweise höchstens 40 Gew.-% Alkohol.

Die durch das erfindungsgemäße Verfahren erhältlichen Polymere zeichnen sich dadurch aus, dass sie als Verdickungsmittel für Zubereitungen verwendet werden können, deren flüssige Phase im wesentlichen OH-Gruppen enthaltende Verbindungen umfasst. Diese OH-Gruppen enthaltende Verbindungen sind im wesentlichen Wasser und Alkohole.

Die durch das erfindungsgemäße Verfahren erhältlichen Polymere eignen sich zur Rheologiemodifizierung von alkoholischen Zubereitungen. Geeignete Alkohole für diese Zubereitungen sind generell alle unter Normalbedingungen flüssig vorliegenden Alkohole. Dies sind beispielsweise Methanol, Ethanol, n-Propanol, Isopropanol, n-Butanol, sek.-Butanol, tert.-Butanol, 3-Methyl-1-butanol (Isoamylalkohol), n-Hexanol, Cyclohexanol oder Glykole wie Ethylenglykol, Propylenglykol und Butylenglykol, mehrwertige Alkohole wie Glycerin , Diethylenglykol, Triethylenglykol, Polyalkylenglykole wie Polyethylenglykol, Alkylether dieser mehrwertigen Alkohole mit zahlenmittleren Molekulargewichten bis etwa 3000.

Bevorzugt sind kosmetisch akzeptable Alkohole, insbesondere ist oder umfasst der Alkohol Ethanol und/oder Isopropanol, insbesondere Ethanol.

Die durch das erfindungsgemäße Verfahren erhältlichen Polymere können sowohl in alkoholischen und im wesentlichen wasserfreien, wässrigen und im wesentlichen alkoholfreien und wässrig/ alkoholischen Zubereitungen als Verdickungs- und gleichzeitig als Konditionier- und Festigungsmittel wirken.

Die Viskosität der erfindungsgemäßen Zubereitungen beträgt bevorzugt wenigstens 1 000, besonders bevorzugt wenigstens 5 000 und insbesondere wenigstens 10 000 und bevorzugt höchstens 100 000, besonders bevorzugt höchstens 50 000 und insbesondere höchstens 30 000 mPa*s, gemessen als dynamische Viskositätsmessung mit einem Viskosimeter Brookfield DV-II+ Pro, Spindel 6, 20 Umdrehungen pro Minute (Upm) bei 25°C in Abhängigkeit von der Konzentration der Polymeren, die ausgewählt ist aus dem Bereich von 0,2 bis 5,0 Gew.-%.

Eine Ausführungsform der Erfindung sind kosmetische Zubereitungen, insbesondere Haargele auf wässriger, im wesentlichen alkoholfreier Basis mit einem Gehalt an einer Kombination aus den durch das erfindungsgemäße Verfahren erhältlichen Polymeren und gegebenenfalls weiteren Inhaltsstoffen wie beispielsweise wenigstens einem filmbildenden und haarfestigenden Polymer.

Eine weitere Ausführungsform der Erfindung sind kosmetische Zubereitungen, insbesondere Haargele auf alkoholischer, im wesentlichen wasserfreier Basis mit einem Gehalt an einer Kombination aus den durch das erfindungsgemäße Verfahren erhältlichen Polymeren, wenigstens 30 Gew.-% C₁-C₄-Alkoholen und ggf. einem alkohollöslichen, filmbildenden und haarfestigenden Polymer.

(Haar)Gele auf Basis von C₁-C₄-Alkoholen können im Vergleich zu wässrigen oder wässrig/alkoholischen Gelen andere/komplementäre Anforderungen an Haargele erfüllen. Soll beispielsweise ein Festigergel hergestellt werden, so können dann auch alkohollösliche Festigerpolymere eingesetzt werden.

Die durch das erfindungsgemäße Verfahren erhältlichen Polymere werden vorzugsweise in einer Menge von 0,01 bis 20, besonders bevorzugt von 0,05 bis 10, ganz besonders bevorzugt von 0,1 bis 7 Gew.-%, bezogen auf die Zusammensetzung eingesetzt.
Wenn ein Haarfestigerpolymer eingesetzt wird, dann vorzugsweise in einer Menge von 0,1 bis 20, besonders bevorzugt von 0,5 bis 15, ganz besonders bevorzugt von 1 bis 10 Gew.-%. Der Alkohol wird vorzugsweise in einer Menge von 50 bis 99, besonders bevorzugt von 70 bis 98 Gew.-% eingesetzt. Die Gew.-% sind jeweils auf das Gesamtgewicht der Zubereitung bezogen.

Unter alkohollöslichen Polymeren werden in diesem Fall solche Polymere verstanden, welche bei 25°C zu mindestens 5 Gew.-% in mindestens einem Alkohol mit 1 bis 4 C-Atomen löslich sind.Für die Haargele auf alkoholischer, im wesentlichen wasserfreier Basis geeignete flüssige Alkohole sind ein- oder mehrwertige Alkohole, welche bei Raumtemperatur (20°C) flüssig sind und 1 bis 4 C-Atome aufweisen. Dies sind insbesondere die für kosmetische Zwecke üblicherweise verwendeten niederen Alkohole wie z.B. Ethanol, Isopropanol, Glycerin, Ethylenglykol oder Propylenglykol. Besonders bevorzugt sind einwertige Alkohole mit 2 bis 4 Atomen, insbesondere Ethanol und Isopropanol. Das Haargel ist vorzugsweise im wesentlichen wasserfrei, es kann aber zur Verbesserung der Löslichkeit weiterer Inhaltsstoffe geringe Wassermengen enthalten, wobei der Alkoholgehalt aber den Wassergehalt deutlich übersteigt. Im wesentlichen wasserfrei bedeutet, dass der Wassergehalt nicht größer als 10 Gew.-%, vorzugsweise nicht größer als 5 Gew.-% ist. Die erfindungsgemäßen alkoholischen Gele zeichnen sich bei Anwesenheit eines Festigerpolymers durch gute konditionierende Eigenschaften, hohen Festigungsgrad, schnelle Trocknung und angenehme Kühlwirkung aus.

Die erfindungsgemäßen Zubereitungen können auf feuchtem oder trockenem Haar angewendet werden. Die Produkte sind sowohl für glattes, lockiges und krauses Haar geeignet.

Die zuvor beschriebenen Polymere eignen sich auch hervorragend zur Herstellung weiterer kosmetischer und pharmazeutischer Mittel. Sie dienen dabei z.B. als polymere Filmbildner in Zubereitungen für die Körperpflege, was die Anwendung kosmetischer Zubereitungen auf keratinösen Oberflächen wie Haut, Haar, Nägel sowie auch Mundpflegepräparate beinhaltet. Sie sind universell in den verschiedensten kosmetischen Zubereitungen einsetzbar und einformulierbar und mit den üblichen Komponenten verträglich. In den kosmetischen Zubereitungen können die für die erfindungsgemäßen Verwendungen geeigneten Polymere besondere Wirkungen entfalten. Die Polymere können unter anderem zur Feuchthaltung und Konditionierung der Haut und zur Verbesserung des Hautgefühls beitragen.

Die Polymere wirken in den Formulierungen insbesondere sowohl als Verdicker als auch als Konditioniermittel. Die Polymere sind insbesondere dazu in der Lage, als neben Wasser alleiniger Inhaltsstoff einer wässrigen Zusammensetzung in einer Konzentration von 0,5 Gew.-% eine Erhöhung der Viskosität dieser Zusammensetzung auf wenigstens 10 000 mPa*s (Brookfield-Viskosität) und eine Erhöhung der Nasskämmbarkeit des Haares, im Vergleich zu mit reinem Wasser behandeltem Haar um wenigstens 10 % zu bewirken.

Ein besonderer Vorteil der Erfindung ist, dass es gelingt, unter alleiniger Verwendung der vorgenannten Polymere in wässrigen, alkoholischen oder wässrig/alkoholischen Zusammensetzungen ein als Konditioniermittel geeignetes Haargel (Conditioner-Gel) bereitzustellen. Der für Haargele notwendige verdickende Effekt und die für Conditioner notwendige konditionierende Wirkung können somit von einem einzigen Inhaltsstoffe bereitgestellt werden.

Die erfindungsgemäßen Mittel weisen neben den für die erfindungsgemäßen Verwendungen geeigneten Polymeren bevorzugt wenigstens einen kosmetisch oder pharmazeutisch akzeptablen Träger B) auf, der ausgewählt ist unter
i) Wasser,
ii) wassermischbaren organischen Lösungsmitteln, vorzugsweise C₁-C₄-Alkanolen,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen und
viii) Mischungen davon.

Die Mittel weisen z.B. eine Öl- bzw. Fettkomponente B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestem; Vaselin; Estern, bevorzugt Estern von Fettsäuren, wie z.B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpälmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie C₁-C₁₀-Salicylaten, z.B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle B) sind z.B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z.B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkemöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl bzw. Fettkomponenten B) sind in Kad-Heinz Schrader, Grundlagen und Rezepturen der Kosmetika, 2. Auflage, Verlag Hüthig, Heidelberg, S. 319 - 355 beschrieben, worauf hier Bezug genommen wird.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglykol, Glycerin, Sorbit, etc.

Bei den kosmetischen Mitteln kann es sich um hautkosmetische oder haarkosmetische Mittel handeln.

Vorzugsweise liegen die Mittel in Form eines Sprays, Gels, Schaums, einer Salbe, Creme, Emulsion, Suspension, Lotion; Milch oder Paste vor. Gewünschtenfalls können auch Liposomen oder Mikrosphären eingesetzt werden.

Die kosmetischen, dermatologischen oder pharmazeutischen Mittel können zusätzlich kosmetisch, dermatologisch oder pharmazeutisch aktive Wirkstoffe sowie Hilfsstoffe enthalten.

Vorzugsweise enthalten die Mittel wenigstens ein wie vorstehend definiertes, für die erfindungsgemäße Verwendung geeignetes Polymer, wenigstens einen wie vorstehend definierten Träger B) und wenigstens einen davon verschiedenen Bestandteil, der ausgewählt ist unter kosmetisch aktiven Wirkstoffen, Emulgatoren, Tensiden, Konservierungsmitteln, Parfümölen, weiteren Verdickern, Haarpolymeren, Haar- und Hautconditionem, Pfropfpolymeren, wasserlösliche oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebem, Feuchthaltemitteln, Rückfettern, Collagen, Eiweisshydrolysaten, Lipiden, Antioxidantien, Entschäumern, Antistatika, Emollentien und Weichmachern.

### Weitere Verdickungsmittel/Gelbildner

Die kosmetischen, dermatologischen oder pharmazeutischen Mittel können zusätzlich zu den durch das erfindungsgemäße Verfahren erhältlichen Polymeren noch weitere Verdickungsmittel/Gelbildner enthalten. Bevorzugt ist es allerdings, keine weiteren Verdickungsmittel zu verwenden.

Weitere Verdickungsmittel/Gelbildner liegen vorzugsweise in einer derartigen Menge vor, dass die Gesamtmenge an Verdickungsmitteln/Gelbildnem im Bereich von 0,01 bis 10 Gew.-%, insbesondere von 0,1 bis 5 Gew.-% oder von 0,5 bis 3 Gew.-% liegt.

Bei den zusätzlichen polymeren Gelbildnem kann es sich um synthetische Homo- oder Copolymere handeln, wobei mindestens eines der Monomere mindestens eine Säuregruppe, vorzugsweise eine Carbonsäure-, Sulfonsäure- oder Phosphorsäuregruppe trägt.

Es kann sich aber auch um Polymere auf natürlicher Basis handeln, insbesondere um Polysaccharide, wobei mindestens eine Saccharidart enthalten ist, die mindestens eine Säuregruppe aufweist, z.B. Glucuronsäure.

Geeignete zusätzliche synthetische Gelbildner sind z.B. aus mindestens einer Monomerart aufgebaut, die ausgewählt ist aus Acrylsäure, Methacrylsäure, Itaconsäuremonoestem, Acrylamidoalkylsulfonsäuren und/oder Methacrylamidoalkylsulfonsäuren.

Synthetische Gelbildner können z.B, sein: vernetzte oder unvernetzte Homopolymere der Acrylsäure (Carbomere) mit einem Molekulargewicht von z.B. von 2.000.000 bis 6.000.000 (entsprechende Gelbildner sind unter der Handelsbezeichnung Carbopol^{®} im Handel erhältlich), Copolymere aus Acrylsäure und Acrylamid, z.B. mit einem Molekulargwicht von 2.000.000 bis 6.000.000, AcrylateSteareth-20 Methacrylate Copolymer, Copolymere von Acryl- oder Methacrylsäure mit Acrylsäure- oder Methacrylsäureestem (Acrylatcopolymere), Acrylates/C10-30 Alkylacrylate Crosspolymer, Acrylat-Vinylalkohol Copolymere, Polystyrolsulfonsäure und deren Mischungen.

Gelbildner auf natürlicher Basis können natürliche oder modifizierte natürliche Polymere sein, z.B.: Alginsäure, Carrageenan, Carboxymethylcellulose, Carboxymethylhydroxyethylcellulose, Carboxymethyldextran, Carboxymethylhydroxypropylguar, Cellulosesulfat, Dextransulfat, Karaya Gum, Xanthan Gum und deren Mischungen.

Geeignete Gelbildner sind insbesondere Homo- oder Copolymere, gebildet aus mindestens einem ethylenisch ungesättigten Monomeren der allgemeinen Formel (I) CH₂=CR¹R², wobei R¹ ausgewählt ist aus A-(CH₂CH₂O),-R³ und COOH, A ausgewählt ist aus C(=O)O, C(=O)NH und CH₂0, x eine Zahl von 1 bis 100, vorzugsweise von 10 bis 50 ist, R³ einen C₁- bis C₃₀-Alkylrest, vorzugsweise einen C₈- bis C₃₀-Alkylrest bedeutet, R² ausgewählt ist aus H, C₁-C₃₀-Alkyl und CH₂-R¹, unter der Maßgabe, dass mindestens einer der Reste R¹ und R² die Gruppe A-(CH₂CH₂0)-R³ enthält. Geeignete Copolymere sind z.B. gebildet aus mindestens einem ethylenisch ungesättigten Monomeren der allgemeinen Formel (I) sowie mindestens einem ethylenisch ungesättigten Monomeren der allgemeinen Formel (11) CH₂=C(R⁴)COOR⁵ wobei R⁴ und R⁵ unabhängig voneinander ausgewählt sind aus H und einer Alkylgruppe mit 1 bis 30, vorzugsweise mit 1 bis 12, insbesondere bevorzugt mit 1 bis 4 C-Atomen. Bevorzugt ist, dass A ausgewählt ist aus C(=O)O und CH₂0, dass R² ausgewählt ist aus H und Methyl oder dass es sich bei dem Monomer der Formel (I) um ein Itaconsäurederivat handelt. Ebenfalls bevorzugt ist, daß es sich bei dem Monomer der Formel (11) um Acrylsäure; Methacrylsäure oder einen ihrer C1- bis C4-Alkylester handelt. Geeignete Copolymere sind beispielsweise Acryl- oder Methacrylsäure/Acryl- oder Methacrylsäurepolyethoxyalkylester Copolymere (INCI-Bezeichnungen: Acrylates/Steareth-20 Methacrylate Copolymer, Acrylates/Palmeth-25 Acrylate Copolymer), wie sie z.B. unter den Bezeichungen Acrysol^{®}-22, Acrysol^{®}ICs, Aculyn^{®}-22 oder Synthaten^{®} W-2000 vertrieben werden oder Acryl- oder Methacrylsäure/Polyethoxyallylether Copolymere (INCI-Bezeichnung: Steareth-10 AllylEtherAcrylates Copolymer), wie sie z.B. unter der Bezeich- nung Salcare^{®}SC 90 vertrieben werden.

Geeignete Gelbildner sind z.B. Copolymere, aufgebaut aus Itaconsäuremonoestern der allgemeinen Formel CH₂=C(COOR¹)CH₂COOR² wobei einer der Substituenten R¹ und R² für Wasserstoff und der andere für die Gruppe -(CH₂CH₂0),-R³ steht; x eine Zahl zwischen 1 und 100 vorzugsweise zwischen 10 und 40, besonders bevorzugt 20 ist; R³ eine Alkylgruppe mit 8 bis 30, vorzugsweise 12 bis 20 C-Atomen, besonders bevorzugt Cetyl oder Stearyl ist sowie mindestens einer zweiten Monomerart, ausgewählt aus Acrylat-Monomeren. Die Acrylat-Monomeren sind vorzugsweise ausgewählt aus Acrylsäure, Methacrylsäure und deren einfachen Estern, insbesondere den Acrylsäurealkylestern und Methacrylsäurealkylestern mit 1 bis 10, vorzugsweise 1 bis 4 C-Atomen in der Alkylgruppe. Geeignete Copolymere sind z.B. Acryl- oder Methacrylsäure/ Itaconsäurepolyethoxyalkylester Copolymere (INCI-Bezeichnungen: Acrylates/ Steareth-20 Itaconate Copolymer und Acrylates/Ceteth-20 Itaconate Copolymer, Acrylates/Aminoacrylates/C10-30 Alkyl PEG-20 Itaconate Copolymer), wie sie z.B. unter den Bezeichnungen Structure^{®}2001, Structure^{®}3001 und Structure^{®}Plus vertrieben werden.

Geeignete Gelbildner sind auch Homo- oder Copolymere, aufgebaut aus mindestens einer Monomerart ausgewählt aus Acryl- oder Methacrylamidoalkylsulfonsäure. Das Polymer ist vorzugsweise aufgebaut aus Monomeren der allgemeinen Formel H₂C=CH-C(=O)-NH-A-S0₃H, wobei A für eine divalente C₂- bis C₆-, vorzugsweise für eine C₃- oder C₄-Kohlenwasserstoffgruppe steht, besonders bevorzugt ist die Gruppe - C(CH,),-CH,-. Dieses Monomer ist vorzugsweise copolymerisiert mit mindestens einem nicht ionischen, ra- dikalisch copolymerisierbarem Monomer, insbesondere einem Vinyllactam, besonders bevorzugt Vinylpyrrolidon. Ein solcher Gelbildner hat z.B. die INCI-Bezeichnung Ammonium Acryloyldimethyltaurate/VP Copolymer. Ein geeignetes Handelsprodukt ist Aristoflex^{®}AVC. [0019] Zusätzlich können in dem erfindungsmgemäßen Mittel als Co-Verdicker nicht-polymere und/oder nich- tionische polymere Verdicker wie z.B. Hydroxyethylcellulose enthalten sein.

### Kosmetisch und/oder dermatologisch aktive Wirkstoffe

Geeignete kosmetisch und/oder dermatologisch aktive Wirkstoffe sind z.B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z.B. Dihydroxyaceton, Alloxan und Wainussschalenextrakt. Geeignete Keratin-härtende Stoffe sind in der Regel Wirkstoffe, wie sie auch in Antitranspirantien eingesetzt werden, wie z.B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z.B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z.B. Zinkricinoleat, Triclosan, Undecylensäurealkylolamide, Citronensäuretriethylester, Chlorhexidin etc.

Geeignete Lichtfilterwirkstoffe sind Stoffe, die UV-Strahlen im UV-B- und/oder UV-A-Bereich absorbieren. Geeignete UV-Filter sind z.B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid.

Zur Verwendung in den Wasser enthaltenden Zusammensetzungen geeignete Lichtschutzmittel sind alle die in der EP-A 1 084 696 in den Absätzen [0036] bis [0053] genannten Verbindungen, auf die hier vollumfänglich Bezug genommen wird.

Die Aufzählung der genannten UV-Lichtschutz-Filter, die in den erfindungsgemäßen Zubereitungen verwendet werden können, soll selbstverständlich nicht limitierend sein.

### Keimhemmende Mittel

Weiterhin können in den Wasser enthaltenden Zusammensetzungen auch keimhemmende Mittel eingesetzt werden. Dazu gehören generell alle geeigneten Konservierungsmittel mit spezifischer Wirkung gegen grampositive Bakterien, z.B. Triclosan (2,4,4'-Trichlor-2'-hydroxydiphenylether), Chlorhexidin (1,1'-Hexamethylenbis[5-(4-chlorphenyl)-biguanid) sowie TTC (3,4,4'-Trichlorcarbanilid). Kommerziell erhältliche Produkte sind Phenonip^{®}, Euxyl^{®}400, Euxyl^{®}100 oder Euxyl^{®}500.

Quartäre Ammonium-Verbindungen sind prinzipiell ebenfalls geeignet, werden jedoch bevorzugt für desinfizierende Seifen und Waschlotionen verwendet.

Auch zahlreiche Riechstoffe haben antimikrobielle Eigenschaften. Spezielle Kombinationen mit besonderer Wirksamkeit gegenüber grampositiven Bakterien werden für die Komposition sog. Deoparfums eingesetzt.

Auch eine große Anzahl etherischer Öle bzw. deren charakteristische Inhaltsstoffe wie z.B. Nelkenöl (Eugenol), Minzöl (Menthol) oder Thymianöl (Thymol), zeigen eine ausgeprägte antimikrobielle Wirksamkeit.

Die antibakteriell wirksamen Stoffe werden in der Regel in Konzentrationen von ca. 0,1 bis 0,3 Gew.-% eingesetzt.

Geeignete Repellentwirkstoffe sind Verbindungen, die in der Lage sind, bestimmte Tiere, insbesondere Insekten, vom Menschen abzuhalten oder zu vertreiben. Dazu gehört z.B. 2-Ethyl-1,3-hexandiol, N,N-Diethyl-m-toluamid etc.

Geeignete hyperemisierend wirkende Stoffe, welche die Durchblutung der Haut anregen, sind z.B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc.

Geeignete keratolytisch und keratoplastisch wirkende Stoffe sind z.B. Salicylsäure, Kalziumthioglykolat, Thioglykolsäure und ihre Salze, Schwefel, etc.

Geeignete Antischuppen-Wirkstoffe sind z.B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z.B. Allantoin, Bisabolol, Dragosantol, Kamillenextrakt, Panthenol, etc.

Die kosmetischen, dermatologischen oder pharmazeutischen Mittel können als kosmetischen und/oder pharmazeutischen Wirkstoff (wie auch gegebenenfalls als Hilfsstoff) wenigstens ein weiteres kosmetisch oder pharmazeutisch akzeptables Polymer enthalten.

### Weitere pharmazeutisch aktive Wirkstoffe

Neben den bereits genannten dermatologischen Wirkstoffen können Wirkstoffe aus folgenden Anwendungsgebieten eingesetzt werden: Antibiotika, beispielsweise Sulfonamide, Antihistaminika, Antimykotika, Antiphlogistika, Antirheumatika, durchblutungsfördernde Mittel, Steroide wie Corticoide, Sexualhormone beispielsweise Gestagene, Wundheilungsmittel wie Dexpanthenol.

Insbesondere können als nichtstereoidale, entzündungshemmende Wirkstoffe eingesetzt werden: Ibuprofen, Ketoprofen, Indomethacin, Diclofenac, Methylsalicylat, Hydroxyethylsalicylat, Etofenamat.

### Weitere Polymere

Bevorzugt sind Mittel, die zusätzlich wenigstens ein nichtionisches, ein anionisches, ein kationisches oder ein ampholytisches Polymer enthalten.

Beispiele für geeignete zusätzliche anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Bevorzugt sind weiterhin Mittel, die zusätzlich ein Polyurethan als anionisches Polymer enthalten.

In einer bevorzugten Ausführungsform enthalten die erfindungsgemäßen Zubereitungen/Mittel wenigstens eines der nachfolgend beschriebenen, üblichen Festigerpolymere.

Besonders geeignet als zusätzliche Polymere sind die in der DE 4225045 A1 beschriebenen wasserlöslichen oder wasserdispergierbaren Polyurethane, auf die hier in vollem Umfang Bezug genommen wird. Insbesondere geeignet ist Luviset^{®}P.U.R. (BASF).

Weiterhin besonders bevorzugt sind silikonhaltige Polyurethane, wie sie in der DE 19807908 A1 beschrieben sind, auf die hier in vollem Umfang Bezug genommen wird. Insbesondere geeignet ist Luviset^{®}Si-P.U.R. (BASF).

Besonders geeignete Polymere sind Copolymere aus (Meth)acrylsäure und Polyetheracrylaten, wobei die Polyetherkette mit einem C₈-C₃₀-Alkylrest terminiert ist. Dazu zählen z.B. AcrylatlBeheneth-25-methacrylat-Copolymere, die unter der Bezeichnung Aculyn^{®} von der Firma Rohm und Haas erhältlich sind. Besonders geeignete Polymere sind weiterhin Copolymere aus t-Butylacrylat, Ethylacrylat, Methacrylsäure (z.B. Luvimer^{®}100P), Copolymere aus Ethylacrylat und Methacrylsäure (z.B. Luviumer^{®} MAE), Copolymere aus N-tert.-Butylacrylamid, Ethylacrylat, Acrylsäure (Ultrahold^{®}8, strong), Copolymere aus Vinylacetat, Crotonsäure und gegebenenfalls weitere Vinylester (z.B. Luviset^{®} Marken), Maleinsäureanhydridcopolymere, gegebenenfalls mit Alkohol umgesetzt, anionische Polysiloxane, z.B. carboxyfunktionelle, t-Butylacrylat, Methacrylsäure (z.B. Luviskol^{®}VBM), Copolymere von Acrylsäure und Methacrylsäure mit hydrophoben Monomeren, wie z.B. C₄-C₃₀-Alkylester der Meth(acrylsäure), C₄-C₃₀-Alkylvinylester, C₄-C₃₀-Alkylvinylether und Hyaluronsäure. Beispiele für anionische Polymere sind weiterhin Vinylacetat/Crotonsäure-Copolymere, wie sie beispielsweise unter den Bezeichnungen Resyn^{®} (National Starch) und Gafset^{®} (GAF) im Handel sind und Vinylpyrrolidon/Vinylacrylat-Copolymere, erhältlich beispielsweise unter dem Warenzeichen Luviflex^{®} (BASF). Weitere geeignete Polymere sind das unter der Bezeichnung Luviflex^{®}VBM-35 (BASF) erhältliche Vinylpyrrolidon/Acrylat-Terpolymer und Natriumsulfonat-haltige Polyamide oder Natriumsulfonat-haltige Polyester.

Weiterhin umfasst die Gruppe der geeigneten anionischen Polymere beispielhaft Balance^{®} CR (National Starch; Acrylate Copolymer), Balance^{®} 0/55 (National Starch; Acrylate Copolymer), Balance^{®} 47 (National Starch; Octylacrylamid/Acrylate/Butylaminoethylmethacrylate-Copolymer), Aquaflex^{®} FX 64 (ISP; Isobutylen/Ethylmaleimid/Hydröxyethylmaleimid-Copolymer), Aquaflex^{®} SF-40 (ISP / National Starch; VP/Vinyl Caprolactam/DMAPA Acrylate Copolymer), Allianz^{®} LT-120 (ISP;Rohm & Haas; Acrylat/C1-2 Succinat/Hydroxyacrylat Copolymer), Aquarez^{®} HS (Eastman; Polyester-1), Diaformer^{®} Z-400 (Clariant; Methacryloylethylbetain/Methacrylat-Copolymer), Diaformer^{®} Z-711 (Clariant; Methacryloylethyl N-oxid/Methacrylat-Copolymer), Diaformer^{®} Z-712 (Clariant; Methacryloylethyl N-oxide/Methacrylat-Copolymer), Omnirez^{®} 2000 (ISP; Monoethylester von Poly(Methylvinylether/Maleinsäure in Ethanol), Amphomer^{®} HC (National Starch; Acrylat/ Octylacrylamid-Copolymer), Amphomer^{®} 28-4910 (National Starch; Octylacrylamid/Acrylat/Butylaminoethylmethacrylat-Copolymer), Advantage^{®} HC 37 (ISP; Terpolymer aus Vinylcaprolactam/Vinylpyrrolidon/Dimethylaminoethylmethacrylat), Advantage^{®} LC55 und LC80 oder LC A und LC E, Advantage^{®} Plus (ISP; VA/Butyl Maleatel/sobornyl Acrylate Copolymer), Aculyne™ 258 (Rohm & Haas; Acrylat/ Hydroxyesteracrylat-Copolymer), Luviset^{®} P.U.R. (BASF, Polyurethane-1), Luviflex^{®} Silk (BASF), Eastman^{®} AQ 48 (Eastman), Styleze^{®} CC-10 (ISP; VP/DMAPA Acrylates Copolymer), Styleze^{®} 2000 (ISP; VP/Acrylates/Lauryl Methacrylate Copolymer), DynamX^{®} (National Starch; Polyurethane-14 AMP-Acrylates Copolymer), Resyn^{®} XP (National Starch; Acrylates/Octylacrylamide Copolymer), Fixomer^{®} A-30 (Ondeo Nalco; Polymethacrylsäure (und) Acrylamidomethylpropahsulfonsäure), Fixate^{®} G-100 (Noveon; AMP-Acrylates/Allyl Methacrylate Copolymer)..

Geeignete zusätzliche Polymere sind auch die in der US 3,405,084 beschriebenen Terpolymere aus Vinylpyrrolidon, C₁-C₁₀-Alkyl- Cycloalkyl- und Aryl(meth)acrylaten und Acrylsäure. Geeignete zusätzliche Polymere sind weiterhin die in der EP-A-0 257 444 und EP-A-0 480 280 beschriebenen Terpolymere aus Vinylpyrrolidon, tert.-Butyl(meth)-acrylat und (Meth)acrylsäure. Geeignete zusätzliche Polymere sind weiterhin die in der DE-A-42 23 066 beschriebenen Copolymere, die wenigstens einen (Meth)acrylsäureester, (Meth)acrylsäure sowie N-Vinylpyrrolidon und/oder N-Vinylcaprolactam einpolymerisiert enthalten. Auf die Offenbarung dieser Dokumente wird hier Bezug genommen.

Geeignete Carbonsäuregruppen-haltige Polymere sind weiterhin Carbonsäuregruppen-haltige Polyurethane.

Die EP-A-636361 offenbart geeignete Blockcopolymere mit Polysiloxanblöcken und Polyurethan-/Polyharnstoffblöcken, die Carbonsäure- und/oder Sulfonsäuregruppen aufweisen. Geeignete siliconhaltige Polyurethane sind auch in der WO 97/25021 und der EP-A-751 162 beschrieben. Geeignete Polyurethane sind auch in der DE-A-42 25 045 beschrieben, worauf hier in vollem Umfang Bezug genommen wird.

Diese Polyurethane sind prinzipiell aufgebaut aus
i) mindestens einer Verbindung, die zwei oder mehrere aktive Wasserstoffatome pro Molekül enthält,
ii) mindestens einem Carbonsäuregruppen enthaltenden Diol oder einem Salz davon und
iii) mindestens einem Polyisocyanat.

Bei der Komponente i) handelt es sich z.B. um Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein.

Brauchbare Diole i) sind z.B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol und/oder Cyclohexandimethylol eingesetzt. Geeignete Aminoalkohole i) sind z.B. 2-Aminoethanol, 2-(N-Methylamino)-ethanol, 3-Aminopropanol, 4-Aminobutanol, 1-Ethylaminobutan-2-ol, 2-Amino-2-methyl-1-propanol, 4-Methyl-4-aminopentan-2-ol etc. Geeignete Diamine i) sind z.B. Ethylendiamin, Propylendiamin, 1,4-Diaminobutan, 1,5-Diaminopentan und 1,6-Diaminohexan sowie α,ω-Diaminopolyether, die durch Aminierung von Polyalkylenoxiden mit Ammoniak herstellbar sind.

Bei der Komponente i) kann es sich auch um ein Polymer mit einem zahlenmittleren Molekulargewicht im Bereich von etwa 300 bis 5000, bevorzugt etwa 400 bis 4000, insbesondere 500 bis 3000. Brauchbare Polymere i) sind z.B. Polyesterdiole, Polyetherole und Mischungen davon. Polyetherole sind vorzugsweise Polyalkylenglykole, z.B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Geeignete Polytetrahydrofurane i) können durch kationische Polymerisation von Tetrahydrofuran in Gegenwart von sauren Katalysatoren, wie z.B. Schwefelsäure oder Fluoroschwefelsäure, hergestellt werden. Derartige Herstellungsverfahren sind dem Fachmann bekannt. Brauchbare Polyesterdiole i) weisen vorzugsweise ein zahlenmittleres Molekulargewicht im Bereich von etwa 400 bis 5000, bevorzugt 500 bis 3000, insbesondere 600 bis 2000, auf. Als Polyesterdiole i) kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, etc.

Geeignete Verbindungen ii), die zwei aktive Wasserstoffatome und mindestens eine Carbonsäuregruppe pro Molekül aufweisen, sind z.B. Dimethylolpropansäure und Mischungen, die Dimethylolpropansäure enthalten.

Bei der Komponente iii) handelt es sich um übliche aliphatische, cycloaliphatische und/oder aromatische Polyisocyanate, wie Tetramethylendiisocyanat, Hexamethylendiisocyanat, Methylendiphenyldiisocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon, insbesondere Isophorondiisocyanat und/oder Dicyclohexylmethandiisocyanat. Gewünschtenfalls können bis zu 3 Mol-% der genannten Verbindungen durch Triisocyanate ersetzt sein.

Geeignete zusätzliche Polymere sind weiterhin kationische Polymere. Dazu zählen z.B. Polymere mit der Bezeichnung Polyquaternium nach INCI, z.B. Copolymere aus Vinyipyrrolidon/N-Vinyiimidazoiiumsalzen(Luviquat™ FC, Luviquat™ HM, Luviquat™ MS, Luviquat™ Care), Copolymere aus N-Vinylpyrrolidon/Dimethylaminoethylmethacrylat, quaternisiert mit Diethylsulfat (Luviquat™ PQ 11), Copolymere aus N-Vinylcaprolactam/N-Vinylpyrrolidon/N-Vinylimidazoliumsalzen (Luviquat™Hold); kationische Cellulosederivate (Polyquatemium-4 und -10), Acrylamidocopolymere (Polyquatemium-7) und Chitosan. Geeignete kationische (quaternisierte) Polymere sind auch Merquat™ (Polymer auf Basis von Dimethyldiallylammoniumchlorid), Gafquat™ (quatemäre Polymere, die durch Reaktion von Polyvinylpyrrolidon mit quaternären Ammoniumverbindungen entstehen), Polymer™ JR (Hydroxyethylcellulose mit kationischen Gruppen) und kationische Polymere auf pflanzlicher Basis, z.B. Guarpolymere, wie die Jaguar™-Marken der Fa. Rhodia.

Geeignete zusätzliche Polymere sind auch amphotere oder zwitterionische Polymere, wie die unter den Bezeichnungen Amphomer™ (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette™ (AMERCHOL) im Handel erhältlich sind, und Copolymere aus Hydroxyethylmethacrylat, Methylmethacrylat, N,N-Dimethylaminoethylmethacrylat und Acrylsäure (Jordapon™).

Als zusätzliche Polymere geeignete neutrale Polymere sind z.B. Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex™ Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z.B. Luviskol™Plus (BASF), oder Polyvinylpyrrolidon und deren Copolymere, insbesondere mit Vinylestern, wie Vinylacetat, z.B. Luviskol™VA 37 (BASF); Polyamide, z.B. auf Basis von Itaconsäure und aliphatischen Diaminen, wie sie z.B. in der DE-A-43 33 238 beschrieben sind.

Geeignete Polymere sind auch nichtionische, siloxanhaltige, wasserlösliche oder -dispergierbare Polymere, z.B. Polyethersiloxane, wie Tegopren™ (Fa. Goldschmidt) oder Belsil™ (Fa. Wacker).

### Mittel für die Hautreinigung- und pflege

Durch Zusatz der durch das erfindungsgemäße Verfahren erhältlichen Polymere kann in bestimmten Formulierungen eine erhebliche Verbesserung der Hautverträglichkeit erreicht werden.

Die durch das erfindungsgemäße Verfahren erhältlichen Polymere können vorteilhaft auch in Hautreinigungsmitteln verwendet werden.

Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Die durch das erfindungsgemäße Verfahren erhältlichen Polymere können auch zur Rheologiemodifizierung von kosmetischen Mittel zur Pflege und zum Schutz der Haut, Nagelpflegemittel oder Zubereitungen für die dekorative Kosmetik verwendet werden. Derartige hautkosmetische Mittel sind z.B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyelinern, Rouges, Puder und Augenbrauenstifte.

Außerdem können die durch das erfindungsgemäße Verfahren erhältlichen Polymere in Nose-Strips zur Porenreinigung, in Antiaknemitteln, Repellents, Rasiermitteln, Haarentfernungsmitteln, Intimpflegemitteln, Fußpflegemitteln sowie in der Babypflege verwendet werden.

Bei den Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel enthalten vorzugsweise 0,05 bis 20 Gew.-%, vorzugsweise 0,1 bis 15 Gew.-%, ganz besonders bevorzugt 0,1 bis 10 Gew.-% der durch das efindungsgemäße Verfahren erhältlichen Polymere, bezogen auf das Gesamtgewicht des Mittels.

Besonders Lichtschutzmittel, zu deren Rheologiemodifizierung die durch das erfindungsgemäße Verfahren erhältlichen Polymere verwendet werden, besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die Mittel in einer zur Hautpflege geeigneten Form, wie z.B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den rheologiemodifizierenden und konditionierenden Polymer und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, andere Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z.B. Paraffine, Siliconöle und aliphatische Kohlenwasserstoffe mit mehr als 8 Kohlenstoffatomen, tierische und pflanzliche Öle, wie z.B. Sonnenblumenöl, Kokosöl, Avocadoöl, Olivenöl, Lanolin, oder Wachse, Fettsäuren, Fettsäureester, wie z.B. Triglyceride von C₆-C₃₀-Fettsäuren, Wachsester, wie z.B. Jojobaöl, Fettalkohole, Vaseline, hydriertes Lanolin und acetyliertes Lanolin sowie Mischungen davon.

Zur Einstellung bestimmter Eigenschaften wie z.B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen weitere konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

### Herstellung der Zubereitungen

Die Herstellung der kosmetischen oder pharmazeutischen oder dermatologischen Zubereitungen erfolgt nach üblichen, dem Fachmann bekannten Verfahren.

In einer bevorzugten Ausführungsform der Erfindung liegen die kosmetischen und dermatologischen Zubereitungen in Form von Gelen vor.

In einer weiteren bevorzugten Ausführungsform der Erfindung liegen die kosmetischen und dermatologischen Zubereitungen in Form von Emulsionen, insbesondere als Wasser-in-Öl-(W/O)- oder Öl-in-Wasser(O/W)-Emulsionen vor.

Es ist aber auch möglich, andere Formulierungsarten zu wählen, beispielsweise Hydrodispersionen, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem durch das erfindungsgemäße Verfahren erhältlichen Polymer in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z.B. für eine Hautcreme etc., enthält im Allgemeinen eine wässrige Phase, die mittels eines geeigneten Emulgatorsystems in einer Öl- oder Fettphase emulgiert ist.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karite-ÖI, Hoplostethus-Öl; mineralische Öle, deren Destillationsbeginn unter Atmosphärendruck bei ca. 250°C und deren Destillationsendpunkt bei 410°C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetyhicinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Weiterhin können auch Wachse verwendet werden, wie z.B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

### Wasch-, Dusch- und Badepräparate

Nach einer weiteren bevorzugten Ausführungsform werden die durch das erfindungsgemäße Verfahren erhältlichen rheologiemodifizierenden Polymere besonders vorteilhaft in Duschgelen, Shampoo-Formulierungen oder Badepräparaten verwendet.

Solche Formulierungen enthalten außerdem üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z.B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z.B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolainide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z.B. quaternäre Ammoniumverbindungen, beispielsweise Cetyltrimethylammoniumchlorid.

Weiterhin können die Duschgel-/Shampoo-Formulierungen weitere Verdicker, wie z.B. Kochsalz, PEG-55, Propyleneglykol-Oleat, PEG-120-Methylglucosedioleat und andere, sowie Konservierungsmittel, weitere Wirk- und Hilfsstoffe und Wasser enthalten.

### Haarbehandlungsmittel

Eine besonders bevorzugte Ausführungsform der Erfindung sind Haarbehandlungsmittel, insbesondere die bereits zuvor beschriebenen verdickten Zubereitungen und Haargele.
Erfindungsgemäße Haarbehandlungsmittel enthalten vorzugsweise wenigstens ein durch das erfindungsgemäße Verfahren erhältliches Polymer in einer Menge im Bereich von etwa 0,01 bis 20, besonders bevorzugt von 0,05 bis 10, ganz besonders bevorzugt von 0,1 bis 7 Gew.-% bezogen auf das Gesamtgewicht des Mittels.
Wie bereits ausfürhlich beschrieben, liegen die erfindungsgemäßen Haarbehandlungsmittel vorzugsweise in Form von Haargelen vor.
Sie können aber auch in Form von Haarmousses, Shampoos, Haarsprays, Haarschäumen, Spitzenfluids, Egatisierungsmitteln für Dauerwellen, Haarfärbe- und - bleichmitteln oder "Hot-Oil-Treatments" vorliegen.
Je nach Anwendungsgebiet können die haarkosmetischen Zubereitungen als Gel, Gelspray, (Aerosol-)Spray, (Aerosol-)Schaum, Creme, Lotion oder Wachs appliziert werden.
Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

In einer Ausführungsform der Erfindung enthalten die efindungsgemäßen Mittel einen Anteil an flüchtigen organischen Komponenten (VOC) von höchstens 80 Gew.-%, besonders bevorzugt höchstens 55 Gew.-%.

Die erfindungsgemäßen haarkosmetischen Zubereitungen/Mittel enthalten in einer Ausführungsform
i. 0,05 bis 10 Gew.-% wenigstens eines durch das erfindungsgemäße Verfahren erhältlichen Polymers,
ii. 20 bis 99,95 Gew.-% Wasser und/oder Alkohol,
iii. 0 bis 50 Gew.-% wenigstens eines Treibgases,
iv. 0 bis 5 Gew.-% wenigstens eines Emulgators,
v. bis zu 25 Gew.-% weitere Bestandteile

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren. Farbstoffe, Farbstoffe, Salze, Feuchthaltemittel, Rückfetter, Komplexbildner und weitere übliche Additive.

Alle vorgenannten, für kosmetische Mittel geeigneten Inhaltsstoffe können gegebenenfalls auch für die haarkosmetischen Mittel verwendet werden. Weiterhin zählen hierzu alle in der Kosmetik bekannten, üblichen und zuvor genannten Styling-, Festiger- und Conditionerpolymere.

Zur Einstellung bestimmter Eigenschaften können die Zubereitungen zusätzlich auch weitere konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Für Aerosolschäume besonders geeignete Treibmittel sind Mischungen aus Dimethylether und, gegebenenfalls halogenierten, Kohlenwasserstoffen wie Propan, Butan, Pentan oder HFC-152 a. Dabei sind je nach weiteren Lösungsmitteln und gewünschter Anwendung die Mengenverhältnisse der Treibmittel zu variieren.

Beispiele für nichtionische Emulgatoren (INCI-Nomenklatur) sind Laurethe, z.B. Laureth-4; Cetethe, z.B. Cetheth-1, Polyethylenglycolcetylether; Cetearethe, z.B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Die Polymere können erfindungsgemäß als Verdickungsmittel auch in Shampoos eingesetzt werden. Bevorzugte Shampooformulierungen enthalten
i. 0,05 bis 10 Gew.-% wenigstens eines für die erfindungsgemäße Verwendung geeigneten Polymers,
ii. 25 bis 94,95 Gew.-% Wasser,
iii. 5 bis 50 Gew.-% Tenside,
iv. 0 bis 5 Gew.-% eines weiteren Konditioniermittels,
v. 0 bis 10 Gew.-% weitere kosmetische Bestandteile.

Besonders Polymere, die Methacrylamid als Komponente b) einpolymerisiert enthalten, sind für die Verwendung als Verdicker für Shampoos und andere tensidhaltige Zusammensetzungen geeignet.

In den Shampooformulierungen können alle in Shampoos üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z.B. quaternäre Ammoniumverbindungen, beispielsweise Cetyltrimethylammoniumchlorid.

In den Shampooformulierungen können zur Erzielung bestimmter Effekte neben den auch alleine als Konditioniermittel geeigneten Polymeren dieser Erfindung weitere übliche Konditioniermittel eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquatemium-4 und -10), Acrylamidcopolymere (Polyquatemium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Eine erfindungsgemäß für Styling-Gele geeignete Zubereitung kann beispielsweise wie folgt zusammengesetzt sein:
i. 0,1 bis 10 Gew.-% wenigstens eines für die erfindungsgemäße Verwendung geeigneten Polymers,
ii. 80 bis 99,9 Gew.-% Wasser und/oder Alkohol,
iii. 0 bis 20 Gew.-% weitere Bestandteile.

Der Einsatz der für die erfindungsgemäße Verwendung geeigneten Polymere als Gelbildner ist insbesondere von Vorteil, wenn spezielle rheologische oder andere anwendungstechnische Eigenschaften der Gele einzustellen sind. Aufgrund der hervorragenden Verträglichkeit der für die erfindungsgemäße Verwendung geeigneten Polymeren mit weiteren kosmetisch üblichen Gelbildnern, können diese Gelbildner auch in Kombination eingesetzt werden.

In einer Ausführungsform der Erfindung wird die erfindungsgemäße verdickte Zubereitung bzw. das kosmetische Gel in einer Phase mit vorzugsweise wenigstens 20, besonders bevorzugt wenigstens 40 und insbesondere wenigstens 50 Gew.-% Wasser und bevorzugt höchstens 40 Gew.-% Alkohol konfektioniert. Als Alkohole können die bereits zuvor genannten, für kosmetische Zwecke üblicherweise verwendeten niederen Monoalkohole mit 1 bis 4 C-Atomen, z.B. Ethanol und Isopropanol enthalten sein.

In einer bevorzugten Ausführungsform enthält das Gel insbesondere zur Glanzverbesserung der behandelten Haare mehrwertige Alkohole, vorzugsweise solche mit 2 bis 6 C-Atomen und mit 2 bis 6 Hydroxygruppen in einer Menge von 0,1 bis 15, bevorzugt von 1 bis 10 Gew.-%. Besonders bevorzugt sind Glycerin, Ethylenglykol und Propylenglykol, insbesondere 1,2-Propylenglykol und Sorbitol. Zur Glanzverbesserung können auch Silikonöle, insbesondere Polydimethylsiloxane (Dimethicone) und arylsubstituierte Polydimethylsiloxane (2.B. Phenyltrimethicone) eingesetzt werden.

### Pigmente

In einer besonderen Ausführungsform ist das erfindungsgemäße Gel zur gleichzeitigen Konditionierung und temporären Haarfärbung geeignet und enthält zusätzlich mindestens ein temporär haarfärbendes Pigment.

Unter temporärer Haarfärbung wird eine Farbänderung von menschlichen Haaren verstanden, die bis zur nächsten Haarwäsche hält und durch Waschen der Haare mit üblichen Shampoos wieder entfernt werden kann. Die Pigmente sind vorzugsweise in einer Menge von 0,01 bis 25 Gew.-%, besonders bevorzugt in einer Menge von 5 bis 15 Gew.-% enthalten. Bei den Pigmenten handelt es sich vorzugsweise nicht um Nano- sondern um Mikropigmente. Die bevorzugte Teilchengröße beträgt 1 bis 200 µm, insbesondere 3 bis 150 µm, besonders bevorzugt 10 bis 100 µm.

Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Bunt-Pigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, - hydroxide und -oxidhydrate, Mischphasen-Pigmente, schwefelhaltige Silicate, Metallsulfide, komplexe Metallcyanide, Metallsulfate, -Chromate und -molybdate sowie die Metalle selbst (Bronzepigmente). Geeignet sind insbesondere Titandioxid (Cl 77891), schwarzes Eisenoxid (Cl 77499), gelbes Eisenoxid (Cl 77492), rotes und braunes Eisenoxid (Cl 77491), Manganviolett (Ci 77742), Ultramarine (Natrium-Aluminiumsulfosilikate, Cl 77007, Pigment Blue 29), Chromoxid-hydrat (C177289), Eisenblau (Ferric Ferrocyanide, Cl7751 0), Carmine (Cochineal).

Besonders bevorzugt sind Pigmente auf Mica- bzw. Glimmerbasis welche mit einem Metalloxid oder einem Metalloxychforid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnung Rona^{®}, Colorona^{®}, Dichrona^{®} und Timiron^{®} (Firma Merck, Deutschland) vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylen- und Perinon-, Metallkomplex-, Alkali blau- und Diketopyrrolopyrrol-Pigmente.

Ein weiterer Gegenstand der Erfindung sind wässrige Zusammensetzungen enthaltend wenigstens ein für die erfindungsgemäßen Verwendungen geeignetes Polymer und wenigstens ein weiteres Polymer, insbesondere eines, das N-Vinyllactam einpolymerisiert enthält.

Bevorzugte einpolymerisierte N-Vinyllactame sind unsubstituierte N-Vinyllactame und N-Vihyllactamderivate, die z.B. einen oder mehrere C₁-C₆-Alkylsubstituenten, wie Methyl, Ethyl, n-Propyl, Isopropyl, n-Butyl, sec.-Butyl, tert.-Butyl etc., aufweisen können. Dazu zählen z.B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. sowie deren Mischungen. Besonders bevorzugt enthalten die wässrigen Zusammensetzungen wenigstens ein für die erfindungsgemäßen Verwendungen geeignetes Polymer und Polyvinylpyrrolidon. Dabei sind Polyvinylpyrrolidone mit einem K-Wert von wenigstens 30, bevorzugt wenigstens 60, besonders bevorzugt wenigstens 90 besonders geeignet. Solche Polyvinylpyrrolidone sind beispielsweise unter dem Handelsnamen Luviskol™ (BASF) im Handel erhältlich. Derartige Zusammensetzungen zeigen bereits bei Gesamt-Konzentrationen von erfindungsgemäßem Polymeren und Polymer,das N-Vinyllactam einpolymerisiert enthält (insbesondere Polyvinylpyrrolidon), im Bereich von ca. 0,5-10 Gew.-%, bevorzugt 1-5 Gew.-% insbesondere als Gele sehr gute Eigenschaften wie hohe Viskosität und Klarheit der wässrigen Zusammensetzung sowie sehr gute Festigung bei Anwendung auf den Haaren.

### Darreichung

Es ist vorteilhaft, wenn die erfindungsgemäßen kosmetischen Zusammensetzungen in entsprechenden Behältnissen wie beispielsweise einer Tube, eines Tiegels, einer Dose einer Flasche oder Quetschflasche aufbewahrt und aus dieser heraus angewendet werden. Demzufolge sind auch Tuben, Tiegel, Dosen, Flaschen oder Quetschflaschen, welche eine erfindungsgemäße Zusammensetzung enthalten, erfindungsgemäß.
Es ist erfindungsgemäß vorteilhaft, wenn die erfindungsgemäße kosmetische Zubereitung in einer Dose, einem Tiegel, einer Flasche, Quetschflasche, Pumpspray-, oder Aerosoldose aufbewahrt und aus dieser heraus angewendet werden. Entsprechend sind auch Flaschen, Quetschflaschen, Pumpspray-, oder Aerosoldosen, welche eine erfindungsgemäße Zubereitung enthalten, erfindungsgemäß.
Die erfindungsgemäßen Zubereitungen werden vorteilhaft zur Pflege des Haars, insbesondere des Kopfhaares, eingesetzt.
Die Verwendung einer erfindungsgemäßen kosmetische Zubereitung als Haarshampoo und oder Haarkonditionierer, d.h. als Mittel zum Konditionieren des Haares, ist ebenfalls erfindungsgemäß.

### Pharmazeutische Zubereitungen

Bevorzugte Polymere für pharmazeutische Zubereitungen enthalten N-Vinyllactame, insbesonder N-Vinylimidazol, welches auch in quaternisierterForm vorliegen kann, wobei die Quaternisierung insbesonder mit einer Methylgruppe erfolgt.

Weiterhin können die Zubereitungen für topische Zubereitungen übliche pharmazeutische Hilfsstoffe in üblichen Mengen enthalten.

Geeignete Hilfsstoffe sind beispielsweise Tenside.Als Tenside eignen sich beispielsweise polyalkoxilierte Sorbitansäurester, polyalkoxilierte Rizinusöle oder polyalkoxilierte hydrierte Rizinusöle, beispielsweise Cremophor®-Typen wie Cremophor RH 40.

Die pharmazeutischen Zubereitungen sind üblicherweise im wesentlichen wässrige Systeme. Weiterhin können die Zubereitungen aber auch organische Lösungsmittel wie Ethanol, Isopropanol, Porpylenglykol, Polypropylenglykole oder Glycerin enthalten.

### Beispiele

Durch die nachfolgenden Beispiele soll die Erfindung detailliert veranschaulicht werden, ohne sie jedoch darauf zu beschränken.

### Herstellung der Polymere nach dem erfindungsgemäßen Verfahren

In einem gerührten Reaktor mit Stickstoffzuführung, Rückflusskühler und Dosiervorrichtung wurde die Vorlage unter Stickstoff auf ca. 100°C erhitzt. Danach wurde Zulauf 1 und 1/3 des Zulaufes 2 in ca. 2 Stunden zugegeben. Danach wurden die verbleibenden 2/3 von Zulauf 2 in ca. 2 Stunden zudosiert. Man ließ die Mischung bei ca. 100°C für ca. weitere 2 Stunden polymerisieren. Anschließend wurde die Mischung auf ca. 120°C aufgeheizt und Zulauf 3 innerhalb von ca. einer Stunde zudosiert. Danach ließ man die Mischung für ca. weitere 6 Stunden nachreagieren, um einen möglichst niedrigen Gehalt an Restmonomeren zu erzielen. Die Mischung wurde auf ca. 60°C abgekühlt und entspannt.
Die erforderliche Menge MeCl wurde langsam zugegeben, die Mischung anschließend wieder auf ca. 100°C erhitzt und für ca. 2 Stunden unter Rühren reagiert. Danach wurde auf Raumtemperatur abgekühlt und die Vorrichtung überdrucklos gemacht. Danach wurde die Vorrichtung mindestens 1 Stunde mit Stickstoffgas gespült. Der Feststoffgehalt der Mischungen betrug in der Regel im Bereich von 25-30 Gew.-%.
Es wurde filtriert, mit Ethylacetat gewaschen und getrocknet unter Vakuum bei ca. 100°C, so dass man jeweils ein feinteiliges weißes Pulver erhielt.

Die folgende Tabelle zeigt Polymere, die nach dem erfindungsgemäßen Verfahren hergestellt wurden und die die an sie gestellten Anforderungen erfüllen.

Soweit nicht anders vermerkt, sind die Mengenangaben in Gramm [g], die Mengenverhältnisse sind Gewichtsverhältnisse (w/w).

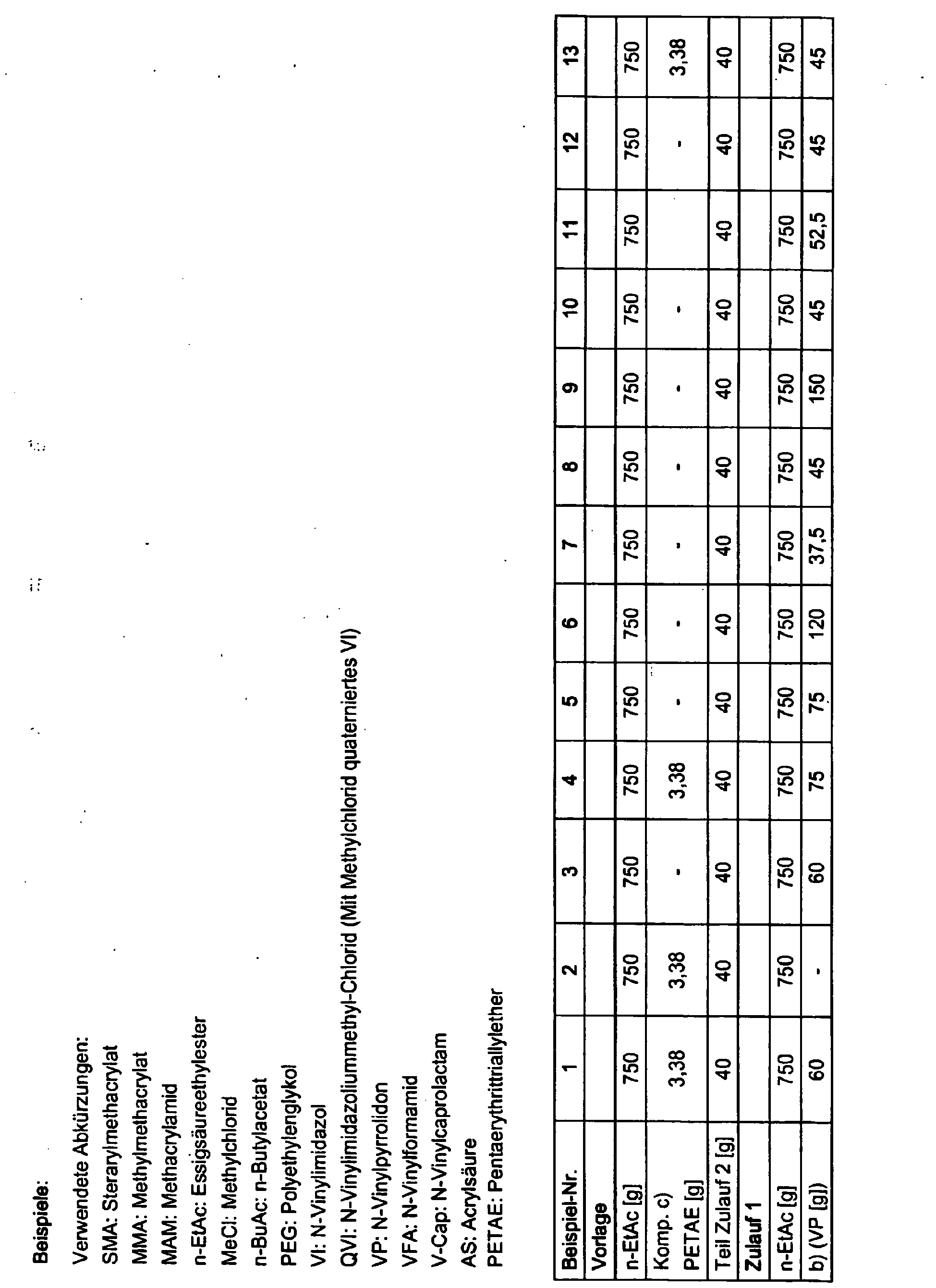

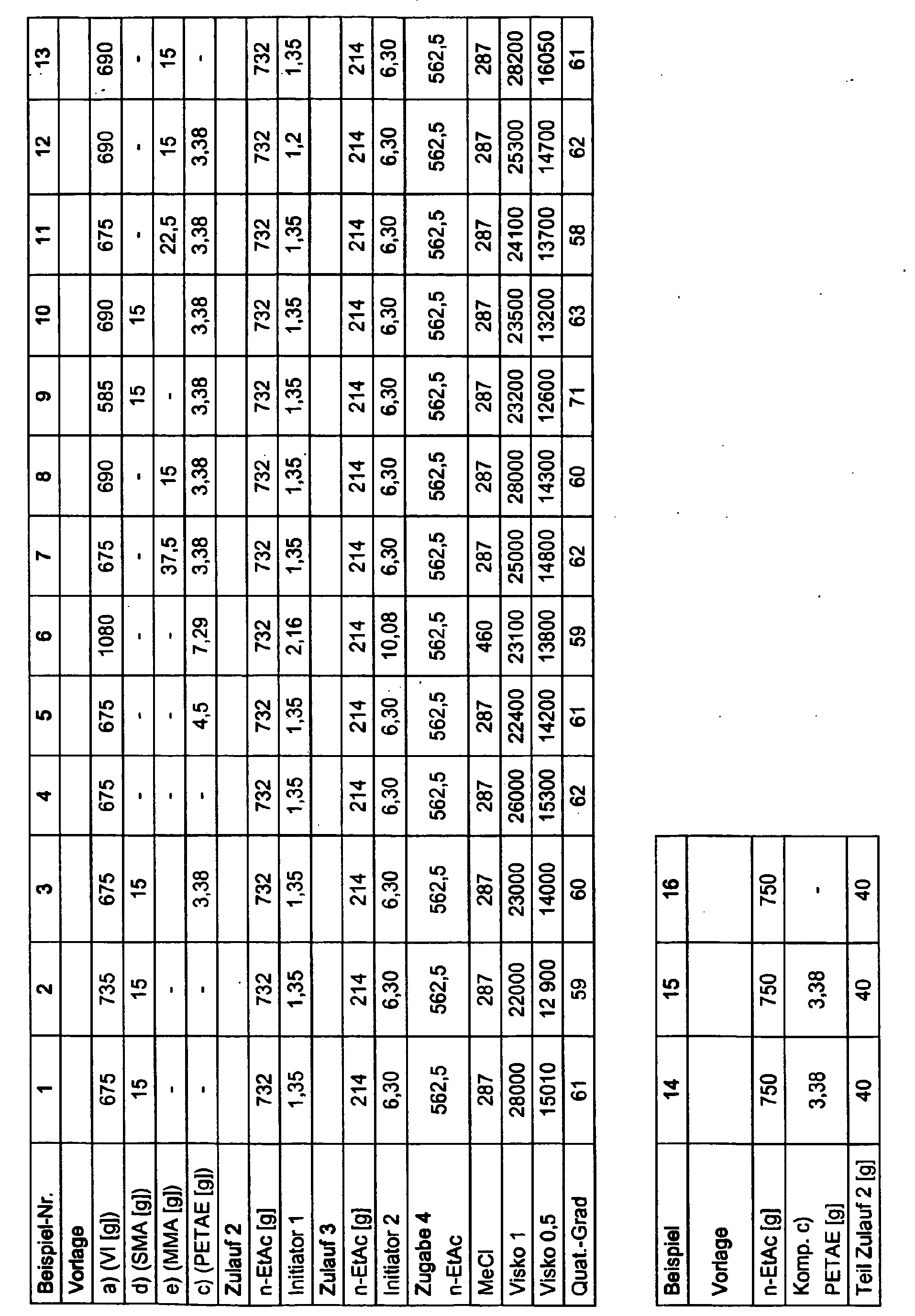

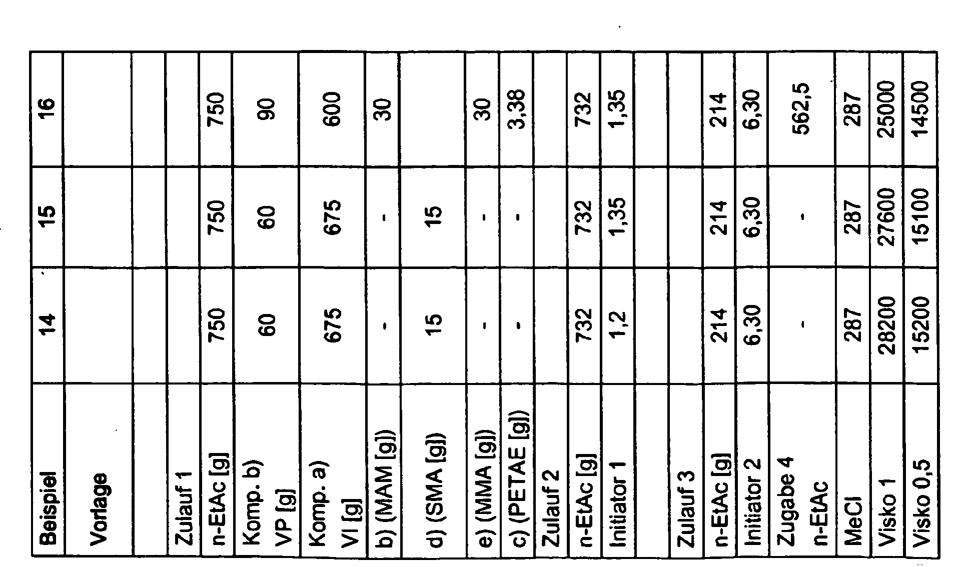

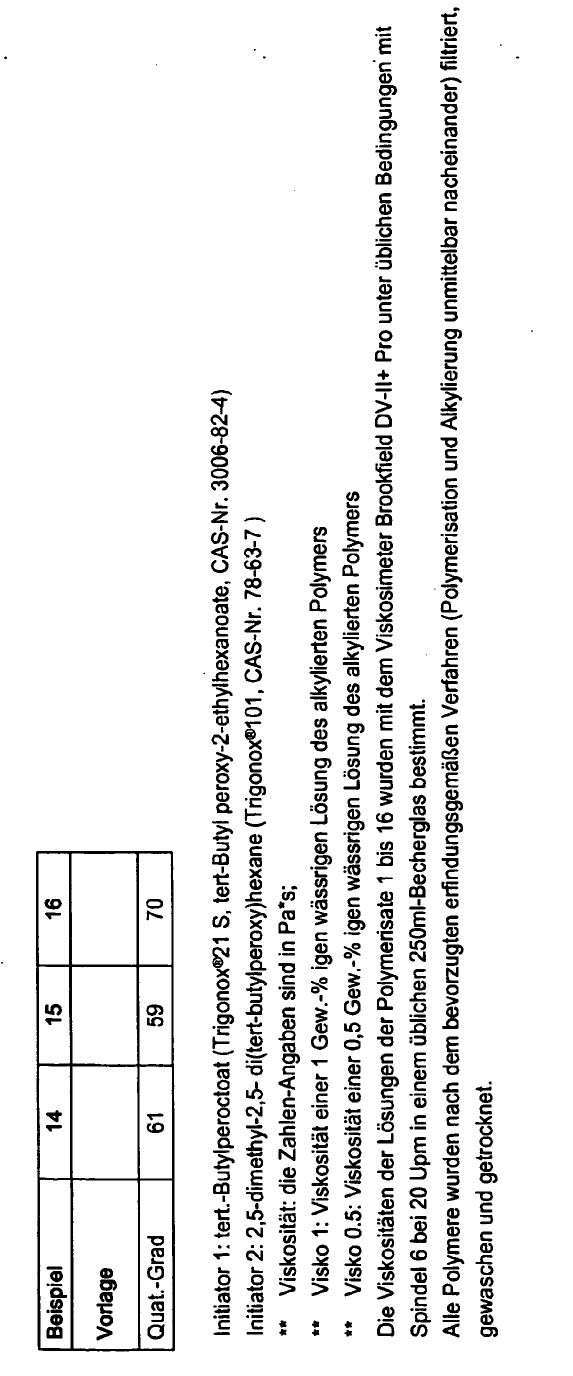

### Anwendungstechnische Prüfung

Die zuvor beschriebenen Polymere wurde folgenden anwendungstechnischen Prüfungen unterzogen:

### Viskosität:

Die Viskositäten der Gele wurden mit dem Viskosimeter Brookfield DV-II+ Pro unter üblichen Bedingungen mit Spindel 6 bei 20 Upm in einem üblichen 250ml-Becherglas bestimmt.

### Gelstruktur:

Mit einem Flachspatel wurde ein 1 mm dicker Film des Gels auf einer Glasplatte ausgestrichen. Die Oberflächenbeschaffenheit des Gelfilms wurde anschließend gemäß folgender Notenskala beurteilt: 1 = völlig glatt und homogen, 2 = nahezu glatt und homogen, geringe Oberflächenstruktur erkennbar, 3 = narbiger Film, deutliche Oberflächenstruktur erkennbar, 4 = griesig, stark strukturiert

### Klarheit:

Ein 250ml Glas mit einem Durchmesser von ca. 8 cm wurde blasenfrei mit Gel gefüllt. Vor einem schwarzen Hintergrund wurde die Klarheit in der Durchsicht gegen ein Referenzgel enthaltend 0,2 Gew.-% Carbopol®Ultrez 21 und 3 Gew.-% Luviset®Clear vergleichend beurteilt.

### Festigung (Handtest):

Eine Haarsträhne (kaukasisches Humanhaar, ungebleicht, Durchmesser 5 mm) wurde mit dem Haargel behandelt und gleichmäßig rund geformt (Auftragsmenge des Gels: 1g). Nach 24 Stunden Trocknen bei 20°C und 65% relativer Luftfeuchte wurde die Festigungswirkung des Haargels durch Biegen der Haarsträhne zwischen Daumen und Zeige- und Mittelfinger beurteilt.

### Auswaschbarkeit:

Eine analog zur Bestimmung der Festigung mit Polymer behandelte Haarsträhne wurde in einer ca. 37°C warmen Texapon^{®}NSO- Lösung (6ml Texapon^{®}NSO (28 Gew.-%)) in 1 Liter warmes Wasser) ca. 15 Sekunden durch 5maliges Eintauchen und Ausdrücken gewaschen. Anschließend wurde die Haarsträhne klargespült und nochmals in der gleichen Art behandelt. Danach wurde die Haarsträhne auf Filterpapier gut ausgedrückt und über Nacht trocknen gelassen. Die trockene Haarsträhne wurde eingedreht und visuell und sensorisch (Handtest) auf Rückstände untersucht.

Haargele enthaltend erfindungsgemäße Verdicker und 3% Luviskol^{®}K90:

| Beispiel | Konzentration Polymer Viskosität [mPas] / pH | Klarheit | Struktur | Auswaschbarkeit / Griff | Festigung |
|---|---|---|---|---|---|
| 1 | 0,5 Gew.-% 21800 / 7,4 | leicht angetrübt | 2 | noch gut/ sehr zart | gut |
| 2 | 0,5 Gew.-% 23050 / 7,1 | fast klar | 2 | noch gut/ sehr zart | gut |
| 3 | 0,5 Gew.-% 18750/7,4 | leicht angetrübt | 2 | noch gut/ sehr zart | gut |
| 4 | 0,5 Gew.-% 2 2900 / 7,3 | fast klar | 2 - 3 | noch gut/ sehr zart | gut |
| 5 | 0,5 Gew.-% 16700 / 7,2 | fast klar | 2 | noch gut/ sehr zart | sehr gut |
| 6 | 0,5 Gew.-% 23000 / 7,4 | leicht angetrübt | 2 | noch gut/ sehr zart | gut |
| 7 | 0,5 Gew.-% 17060 / 7,4 | klar | 2 | noch gut/ sehr zart | gut |
| 8 | 0,5 Gew.-% 18660 / 7,2 | klar | 2 | noch gut/ sehr zart | gut |
| 9 | 0,5 Gew.-% 17620 / 7,2 | trüb | 1 - 2 | noch gut/ sehr zart | gut |
| 10 | 0,5 Gew.-% 15100/ 6,94 | klar, gelblich | 2 - 3 | noch gut/ sehr zart | sehr gut |
| 11 | 0,5 Gew.-% 13080/ 7,29 | klar, gelblich | 2 - 3 | noch gut | sehr gut |
| 12 | 0,5 Gew.-% 17900/7,0 | fast klar | 2 - 3 | noch gut noch gut | gut |
| 13 | 0,5 Gew.-% 18700/7,2 | fast klar | 3 | noch gut | gut |
| 14 | 0,5 Gew.-% 15120/7,0 | fast klar | 1 - 2 | noch gut | sehr gut |
| 15 | 0,5 Gew.-% 21100/7,4 | fast klar | 2 - 3 | noch gut/ sehr zart | gut |
| 16 | 0,5 Gew.-% 19600/7,2 | fast klar | 2 - 3 | noch gut/ sehr zart | noch gut |

## Patentansprüche

1. Verfahren zur Herstellung von Polymeren durch radikalische Polymerisation von
a) 99,99 bis 10 Gew.-% wenigstens einer α,β-ethylenisch ungesättigten Verbindung mit wenigstens einer kationogenen und/oder kationischen Gruppe pro Molekül,
b) 0 bis weniger als 25 Gew.-% wenigstens einer von a) verschiedenen monoethylenisch ungesättigten amidgruppenhaltigen Verbindung,
c) 0,01 bis 5 Gew.-% eines Vernetzers,
d) 0 bis 15 Gew.-% wenigstens einer monoethylenisch ungesättigten Verbindung d1) enthaltend wenigstens eine Gruppe ausgewählt aus der Gruppe bestehend aus, gegebenenfalls substituierten, C₅-C₃₀-Alkyl, C₅-C₃₀-Alkenyl, C₅-C₈-Cycloalkyl, Aryl, Arylalkyl und Hetaryl und/oder eines reaktiven Vorproduktes d2) der Komponente d),
e) 0 bis 30 Gew.-% weiterer, von a) bis d) verschiedener, monoethylenisch ungesättigter Verbindungen,
mit der Maßgabe, dass sich die Mengen der Komponenten a) bis e) zu 100 Gew.-% addieren,
in Gegenwart von
f) 0 bis 70 Gew.-%, bezogen auf die Summe der Komponenten a) bis e) einer polyetherhaltigen Verbindung,
**dadurch gekennzeichnet,**
- **dass** im Verlauf des Verfahrens wenigstens zwei unterschiedliche wasserunlösliche Initiatoren A und B eingesetzt werden und
- **dass** die Polymerisation eine Fällungspolymerisation ist.

2. Verfahren nach Anspruch 1, **dadurch gekennzeichnet, dass** a) ausgewählt ist aus
ai) Estern α,β-ethylenisch ungesättigter Mono- und Dicarbonsäuren mit Aminoalkoholen, welche am Aminstickstoff mono- oder dialkyliert sein können,
aii) Amiden α,β-ethylenisch ungesättigter Mono- und Dicarbonsäuren mit Diaminen, welche mindestens eine primäre oder sekundäre Aminogruppe auf weisen,
aiii) N,N-Diallylaminen,
aiv) vinyl- und allylsubstituierten Stickstoffheterocyclen,
av) vinyl- und allylsubstituierten heteroaromatischen Verbindungen und
avi) Mischungen davon.

3. Verfahren nach Anspruch 2, **dadurch gekennzeichnet, dass** a) ausgewählt ist aus aiv), insbesondere Vinylimidazolen der allgemeinen Formel (III), worin R¹ bis R³ für Wasserstoff, C₁-C₄-Alkyl oder Phenyl steht.

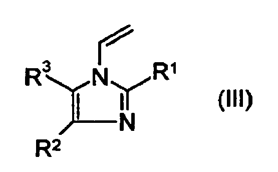

4. Verfahren nach Anspruch 3, **dadurch gekennzeichnet, dass** R¹ bis R³ Wasserstoff bedeuten.

5. Verfahren nach einem der Ansprüche 1 bis 4, **dadurch gekennzeichnet, dass** wenigstens eine weitere Komponente b) ausgewählt ist aus α,β-ethylenisch ungesättigten amidgruppenhaltigen Verbindungen der allgemeinen Formel IV 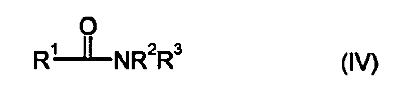 wobei
R¹ für eine Gruppe der Formel CH₂=CR⁴- mit R⁴ = H oder C₁-C₄-Alkyl steht und R² und R³ unabhängig voneinander für H, Alkyl, Cycloalkyl, Heterocycloalkyl, Aryl oder Hetaryl stehen oder R² und R³ gemeinsam mit dem Stickstoffatom, an das sie gebunden sind, für einen fünf- bis achtgliedrigen Stickstoff-Heterocyclus stehen oder
R² für eine Gruppe der Formel CH₂=CR⁴- steht und R¹ und R³ unabhängig voneinander für H, Alkyl, Cycloalkyl, Heterocycloalkyl, Aryl oder Hetaryl stehen oder R¹ und R³ gemeinsam mit der Amidgruppe, an die sie gebunden sind für ein Lactam mit 5 bis 8 Ringatomen stehen.

6. Verfahren nach Anspruch 5, **dadurch gekennzeichnet, dass** in Formel IV R² für CH₂=C_{H}- und R¹ und R³ gemeinsam mit der Amidgruppe, an die sie gebunden sind für ein Lactam mit 5 Ringatomen stehen.

7. Verfahren nach einem der Ansprüche 1 bis 6, **dadurch gekennzeichnet, dass** als polyetherhaltige Komponente f) Verbindungen der allgemeinen Formeln Va oder Vb eingesetzt werden 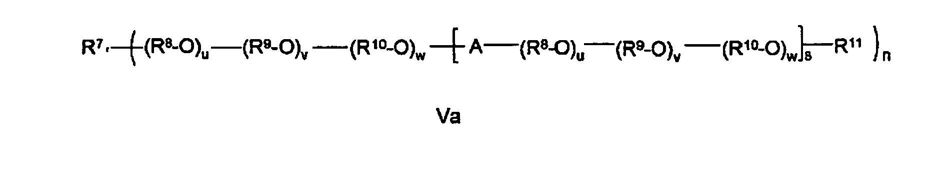 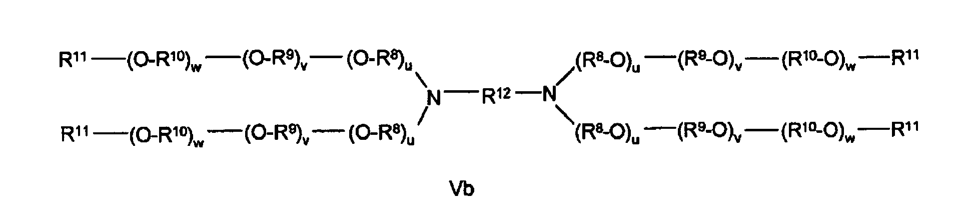 worin:
R⁷ für Hydroxy, Amino, C₁-C₂₄-Alkoxy, R¹³-COO-, R¹³-NH-COO- oder einen Polyalkoholrest steht,
R⁸, R⁹ und R¹⁰ unabhängig voneinander für -(CH₂)₂-, -(CH₂)₃-, -(CH₂)₄-, -CH₂- CH(CH₃)-, -CH₂CH(CH₂-CH₃)- oder -CH₂-CHOR¹⁴-CH₂- stehen,
R¹¹ für Wasserstoff, Amino-C₁-C₆,-alkyl, C₁-C₂₄-Alkyl, R¹³-C(=O)- oder R¹³-NH- C(=O)- steht,
R¹² für eine C₁-C₂₀-Alkylengruppe steht, deren Kohlenstoffkette durch 1 bis 10 nichtbenachbarte Sauerstoffatome unterbrochen sein kann;
R¹³ für C₁-C₂₄-Alkyl steht,
R¹⁴ für Wasserstoff, C₁-C₂₄-Alkyl oder R¹³-CO- steht,
A für -C(=O)-O-, -C(=O)-B-C(=O)-O- oder -C(=O)-NH-B-NH-C(=O)-O- steht,
B für -(CH₂)ₜ-, gewünschtenfalls substituiertes Cycloalkylen, gewünschten- falls substituiertes Heterocycloalkylen oder gewünschtenfalls substituiertes Arylen steht,
n für 1 oder, wenn R⁷ einen Polyalkoholrest bedeutet, 1 bis 8 steht,
s für 0 bis 500, bevorzugt 0 bis 100 steht,
u unabhängig voneinander jeweils für 1 bis 5000, bevorzugt 1 bis 1000, steht,
v unabhängig voneinander jeweils für 0 bis 5000, bevorzugt 1 bis 1000, steht,
w unabhängig voneinander jeweils für 0 bis 5000, bevorzugt 1 bis 1000, steht.

8. Verfahren nach Anspruch 7, **dadurch gekennzeichnet, dass** als polyetherhaltige Komponente Homo- und Copolymerisate von Ethylenoxid und/oder Propylenoxid mit einem gewichtsmittleren Molekulargewicht M_{w} im Bereich von 1000 bis 100000, bevorzugt von 5000 bis 50000 g/mol eingesetzt werden, die einseitig oder beidseitig endgruppenverschlossen sein können.

9. Verfahren nach einem der Ansprüche 1 bis 8, **dadurch gekennzeichnet, dass** die polyetherhaltige Komponente f) bei der Polymerisation in einer Menge im Bereich von 5 bis 70, bevorzugt von 10 bis 50 Gew.-%, bezogen auf die Gesamtmenge der Komponenten a) bis e), gegenwärtig ist.

10. Verfahren nach einem der Ansprüche 1 bis 9, **dadurch gekennzeichnet, dass** der Vernetzer c) ausgewählt ist aus der Gruppe bestehend aus Pentaerythrittriallylether, Methylenbisacrylamid, N,N'-Divinylethylenharnstoff, Triallylamin und Triallylmonoalkylammoniumsalze, Acrylsäureester von Ethylenglykol, Butandiol, Trimethylolpropan oder Glycerin oder Acrylsäureester von mit Ethylenoxid und/oder Epichlorhydrin umgesetztem Glykol, Butandiol, Trimethylolpropan oder Glycerin.

11. Verfahren nach einem der Ansprüche 1 bis 10, **dadurch gekennzeichnet, dass** das zu polymerisierende Gemisch 0,05 bis 2 Gew.-% des Vernetzers c), bezogen auf die Gesamtmenge der Monomere a) bis e) enthält.

12. Verfahren nach einem der Ansprüche 1 bis 11, **dadurch gekennzeichnet, dass** Verbindung d) ausgewählt ist aus der Gruppe bestehend aus C₁₂-C₃₀-Alkyl(meth)acrylaten und C₁₂-C₃₀-Alkylvinylethern.

13. Verfahren nach einem der Ansprüche 1 bis 12, **dadurch gekennzeichnet, dass** Verbindung d) ausgewählt ist aus der Gruppe bestehend aus Methylmethacrylat, Butylacrylat, Methacrylamid und deren Mischungen.

14. Verfahren nach einem der Ansprüche 1 bis 13, **dadurch gekennzeichnet, dass** das Polymer nach oder während der Polymerisation teilweise oder vollständig protoniert oder im Bereich von 20 bis 99 Mol-% quaternisiert wird, sofern als Komponente a) ein nicht oder nur teilweise protoniertes oder quaternisiertes Monomer zur Polymerisation eingesetzt wird.

15. Verfahren nach einem der Ansprüche 1 bis 14, **dadurch gekennzeichnet, dass**, die Initiatoren A und B so gewählt sind, dass die jeweiligen Zerfallstemperaturen wenigstens 70°C betragen.

16. Verfahren nach Anspruch 15, **dadurch gekennzeichnet, dass** sich die jeweiligen Zerfallstemperaturen um wenigstens 10°C unterscheiden.

17. Verfahren nach einem der Ansprüche 1 bis 16, **dadurch gekennzeichnet, dass** die Quaternisierung des Polymers im Wesentlichen unmittelbar nach der Polymerisation in der Polymerisationslösung durchgeführt wird.

18. Polymer erhältlich nach einem Verfahren nach einem der Ansprüche 1 bis 17.

19. Verwendung wenigstens eines Polymer gemäß Anspruch 18 zur Modifizierung der Rheologie wässriger, alkoholischer oder wässriglalkoholischer Zusammensetzungen.

20. Kosmetisches oder pharmazeutisches Mittel enthaltend wenigstens ein Polymer gemäß Anspruch 18.

21. Mittel nach Anspruch 20, enthaltend wenigstens ein Polymer gemäß Anspruch 18 in einer Menge im Bereich von 0,01 bis 20 Gew.-%.

22. Mittel nach einem der Ansprüche 20 oder 21, das weiterhin wenigstens ein Polymer, insbesondere eines, das N-Vinyllactam einpolymerisiert enthält, umfasst.

23. Mittel nach einem der Ansprüche 20 bis 22, wobei es sich bei dem Mittel um ein Gel, insbesondere um ein Haargel handelt.

24. Mittel nach einem der Ansprüche 20 bis 21, wobei es sich bei dem Mittel um ein Gel oder eine Creme für topische pharmazeutische Anwendungen handelt.

25. Mittel nach einem der Ansprüche 20 bis 24, auf im Wesentlichen wässriger Basis.

26. Mittel nach einem der Ansprüche 20 bis 24, auf im Wesentlichen alkoholischer Basis.

## Claims

1. A method for producing polymers by radically polymerizing
a) 99.99 to 10% by weight of at least one α,β-ethylenically unsaturated compound with at least one cationogenic and/or cationic group per molecule,
b) 0 to less than 25% by weight of at least one monoethylenically unsaturated amide-group-containing compound different from a),
c) 0.01 to 5% by weight of a crosslinker,
d) 0 to 15% by weight of at least one monoethylenically unsaturated compound d1) comprising at least one group selected from the group consisting of optionally substituted C₅-C₃₀-alkyl, C₅-C₃₀-alkenyl, C₅-C₈-cycloalkyl, aryl, arylalkyl and hetaryl and/or a reactive precursor d2) of component d),
e) 0 to 30% by weight of further monoethylenically unsaturated compounds different from a) to d), with the proviso that the amounts of components a) to e) add up to 100% by weight,
in the presence of
f) 0 to 70% by weight, based on the sum of components a) to e), of a polyether-containing compound,
wherein
- in the course of the process at least two different water-insoluble initiators A and B are used and
- the polymerization is a precipitation polymerization.

2. The method according to claim 1, wherein a) is selected from
ai) esters of α,β-ethylenically unsaturated mono- and dicarboxylic acids with amino alcohols which may be mono- or dialkylated on the amine nitrogen,
aii) amides of α,β-ethylenically unsaturated mono- and dicarboxylic acids with diamines which have at least one primary or secondary amino group,
aiii) N,N-diallylamines,
aiv) vinyl- and allyl-substituted nitrogen heterocycles,
av) vinyl- and allyl-substituted heteroaromatic compounds and
avi) mixtures thereof.

3. The method according to claim 2, wherein a) is selected from aiv), in particular vinylimidazoles of the general formula (III), in which R¹ to R³ are hydrogen, C₁-C₄-alkyl or phenyl.

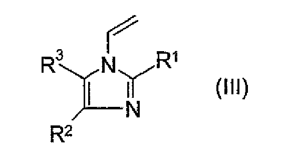

4. The method according to claim 3, wherein R¹ to R³ are hydrogen.

5. The method according to any one of claims 1 to 4, wherein at least one further component b) is selected from α,β-ethylenically unsaturated amide-group-containing compounds of the general formula IV 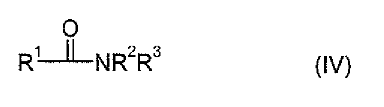 where
R¹ is a group of the formula CH₂=CR ⁴- where R⁴ = H or C₁-C₄-alkyl and R² and R³ independently of one another, are H, alkyl, cycloalkyl, heterocycloalkyl, aryl or hetaryl, or R² and R³, together with the nitrogen atom to which they are bonded, are a five-to eight-membered nitrogen heterocycle or
R² is a group of the formula CH₂=CR⁴- and R¹ and R³, independently of one another, are H, alkyl, cycloalkyl, heterocycloalkyl, aryl or hetaryl or R¹ and R³, together with the amide group to which they are bonded, are a lactam with 5 to 8 ring atoms.

6. The method according to claim 5, wherein, in formula IV, R² is CH₂=CH- and R¹ and R³, together with the amide group to which they are bonded, are a lactam with 5 ring atoms.

7. The method according to any one of claims 1 to 6, wherein the polyether-containing components f) used are compounds of the general formulae Va or Vb 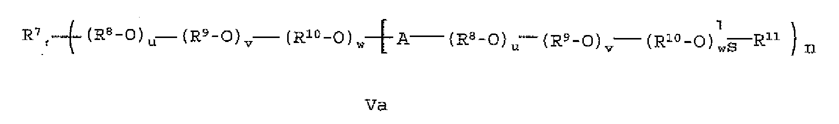 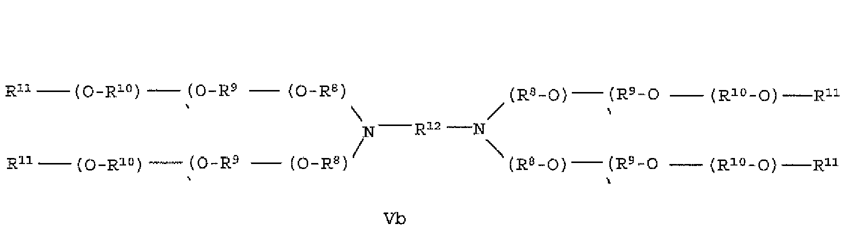 in which:
R⁷ is hydroxy, amino, C₁-C₂₄-alkoxy, R¹³-COO-, R¹³- NH-COO- or a polyalcohol radical,
R⁸, R⁹ and R¹⁰, independently of one another, are -(CH₂)₂-, (CH₂)₃-, -(CH₂)₄-, CH₂-CH(CH₃)-, -CH₂- CH(CH₂-CH₃)- or -CH₂CHOR¹⁴-CH₂-,
R¹¹ is hydrogen, amino-C₁-C₆-alkyl, C₁-C₂₄-alkyl, R¹³- C(=O)- or R¹³-NH-C(=O)-,
R¹² is a C₁-C₂₀-alkylene group whose carbon chain can be interrupted by 1 to 10 nonadjacent oxygen atoms;
R¹³ is C₁-C₂₄-alkyl,
R¹⁴ is hydrogen, C₁-C₂₄-alkyl or R¹³-CO-,
A is -C(=O)-O-, -C(=O)-B-C(=O)-O- or -C(=O)-NH-B- NH-C(=O)-O-,
B is -(CH₂)ₜ-, if desired substituted cycloalkylene, if desired substituted heterocycloalkylene or if desired substituted arylene,
n is 1 or, when R⁷ is a polyalcohol radical, 1 to 8,
s is 0 to 500, preferably 0 to 100,
u independently of the others is in each case 1 to 5000, preferably 1 to 1000,
v independently of the others is in each case 0 to 5000, preferably 1 to 1000,
w independently of the others is in each case 0 to 5000, preferably 1 to 1000.

8. The method according to claim 7, wherein the polyether-containing components f) used are homopolymers and copolymers of ethylene oxide and/or propylene oxide with a weight-average molecular weight M_{w} in the range from 1000 to 100 000, preferably from 5000 to 50 000 g/mol, which may be terminally capped at one or both ends.

9. The method according to any one of claims 1 to 8, wherein the polyether-containing component f) is present during the polymerization in an amount in the range from 5 to 70% by weight, preferably from 10 to 50% by weight, based on the total amount of components a) to e).

10. The method according to any one of claims 1 to 9, wherein the crosslinker c) is selected from the group consisting of pentaerythritol triallyl ether, methylenebisacrylamide, N,N'-divinylethyleneurea, triallylamine and triallylmonoalkylammonium salts, acrylic acid esters of ethylene glycol, butanediol, trimethylolpropane or glycerol or acrylic acid esters of glycol, butanediol, trimethylolpropane or glycerol reacted with ethylene oxide and/or epichlorohydrin.

11. The method according to any one of claims 1 to 10, wherein the mixture to be polymerized comprises 0.05 to 2% by weight of the crosslinker c), based on the total amount of monomers a) to e).

12. The method according to any one of claims 1 to 11, wherein compound d) is selected from the group consisting of C₁₂-C₃₀-alkyl(meth)acrylates and C₁₂-C₃₀-alkyl vinyl ethers.

13. The method according to any one of claims 1 to 12, wherein compound d) is selected from the group consisting of methyl methacrylate, butyl acrylate, methacrylamide and mixtures thereof.

14. The method according to any one of claims 1 to 13, wherein the polymer is partially or completely protonated or quaternized in the range from 20 to 99 mol% after or during the polymerization if a non- or only partially protonated or quaternized monomer is used for the polymerization as component a).

15. The method according to any one of claims 1 to 14, wherein the initiators A and B are selected such that the respective decomposition temperatures are at least 70°C.

16. The method according to claim 15, wherein the respective decomposition temperatures differ by at least 10°C.

17. The method according to any one of claims 1 to 16, wherein the quaternization of the polymer is carried out essentially directly after the polymerization in the polymerization solution.

18. A polymer obtainable by a method according to any one of claims 1 to 17.

19. The use of at least one polymer according to claim 18 for modifying the rheology of aqueous, alcoholic or aqueous/alcoholic compositions.

20. A cosmetic or pharmaceutical composition comprising at least one polymer according to claim 18.

21. The composition according to claim 20, comprising at least one polymer according to claim 18 in an amount in the range from 0.01 to 20% by weight.

22. The composition according to any one of claims 20 or 21, which further comprises at least one polymer, in particular one which comprises N-vinyllactam in copolymerized form.

23. The composition according to any one of claims 20 to 22, where the composition is a gel, in particular a hair gel.

24. The composition according to any one of claims 20 to 21, where the composition is a gel or a cream for topical pharmaceutical applications.

25. The composition according to any one of claims 20 to 24, on an essentially aqueous basis.

26. The composition according to any one of claims 20 to 24, on an essentially alcoholic basis.

## Revendications

1. Procédé pour la préparation de polymères par polymérisation radicalaire de
a) 99,99 à 10 % en poids d'au moins un composé à insaturation α,β-éthylénique, comportant au moins un groupe cationique et/ou cationogène par molécule,
b) 0 à moins de 25 % en poids d'au moins un composé à insaturation monoéthylénique, contenant des groupes amido, différent de a),
c) 0,01 à 5 % en poids d'un agent de réticulation,
d) 0 à 15 % en poids d'au moins un composé à insaturation monoéthylénique d1) contenant au moins un groupe choisi dans l'ensemble constitué par des groupes alkyle en C₅-C₃₀, alcényle en C₅-C₃₀, cycloalkyle en C₅-C₈, aryle, arylalkyle et hétéroaryle, éventuellement substitués, et/ou un précurseur réactif d2) du composant d),
e) 0 à 30 % en poids d'autres composés à insaturation monoéthylénique, différents de a) à d),
étant entendu que la somme des quantités des composants a) à e) est égale à 100 % en poids,
en présence de
f) 0 à 70 % en poids, par rapport à la somme des composants a) à e), d'un composé contenant un polyéther,
**caractérisé**
- **en ce qu'**on utilise au cours du processus au moins deux amorceurs différents A et B insolubles dans l'eau et
- **en ce que** la polymérisation est une polymérisation par précipitation.

2. Procédé selon la revendication 1, **caractérisé en ce que** a) est choisi parmi
ai) des esters d'acides mono- et dicarboxyliques à insaturation α,β-éthylénique avec des aminoalcools qui peuvent être mono- ou dialkylés sur l'atome d'azote en fonction amine,
aii) des amides d'acides mono- et dicarboxyliques à insaturation α,β-éthylénique avec des diamines qui comportent au moins un groupe amino primaire ou secondaire,
aiii) des N,N-diallylamines,
aiv) des hétérocycles azotés à substitution vinylique et allylique,
av) des composés hétéroaromatiques à substitution vinylique et allylique,
avi) des mélanges de ceux-ci.

3. Procédé selon la revendication 2, **caractérisé en ce que** a) est choisi parmi des composés aiv), en particulier des vinylimidazoles de formule générale (III), dans laquelle R¹ à R³ représentent un atome d'hydrogène, un groupe alkyle en C₁-C₄ ou phényle.

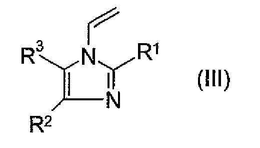

4. Procédé selon la revendication 3, **caractérisé en ce que** R¹ à R³ représentent des atomes d'hydrogène.

5. Procédé selon l'une quelconque des revendications 1 à 4, **caractérisé en ce qu'**au moins un autre composant b) est choisi parmi des composés à insaturation α,β-éthylénique, contenant des groupes amido, de formule générale IV 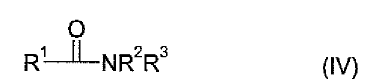 dans laquelle
R¹ représente un groupe de formule CH₂=CR⁴- où R⁴ = H ou un groupe alkyle en C₁-C₄ et R² et R³ représentent, indépendamment l'un de l'autre, H, un groupe alkyle, cycloalkyle, hétérocycloalkyle, aryle ou hétéroaryle, ou R² et R³ représentent ensemble, avec l'atome d'azote auquel ils sont liés, un hétérocycle azoté à cinq à huit chaînons ou
R² représente un groupe de formule CH₂=CR- et R¹ et R³ représentant, indépendamment l'un de l'autre, H, un groupe alkyle, cycloalkyle, hétérocycloalkyle, aryle ou hétéroaryle, ou R¹ et R³ représentent ensemble, avec le groupe amido auquel ils sont liés, un lactame à 5 ou 8 atomes formant le cycle.

6. Procédé selon la revendication 5, **caractérisé en ce que** dans la formule IV R² représente CH₂=CH- et R¹ et R³ représentent ensemble, avec le groupe amido auquel ils sont liés, un lactame à 5 atomes formant le cycle.

7. Procédé selon l'une quelconque des revendications 1 à 6, **caractérisé en ce qu'**on utilise comme composant f) contenant un polyéther des composés de formules générales Va ou Vb : 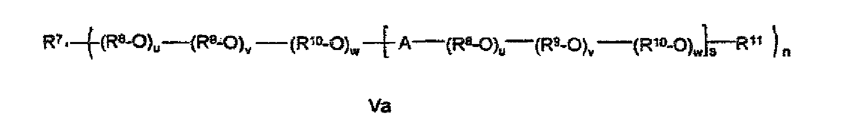 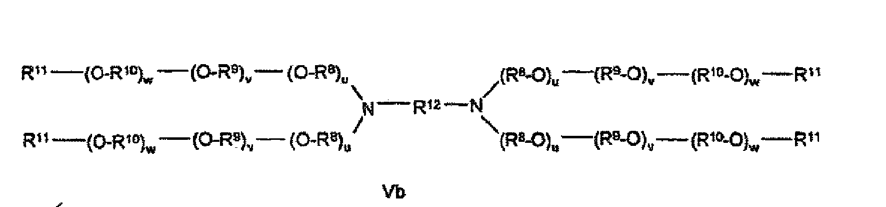 dans lesquelles :
R⁷ représente un groupe hydroxy, amino, alcoxy en C₁-C₂₄, R¹³-COO-, R¹³-NH-COO- ou un reste de polyalcool,
R⁸, R⁹ et R¹⁰ représentent chacun indépendamment - (CH₂)₂-, -(CH₂)₃-, -(CH₂)₄-, -CH₂-CH(CH₃)-, -CH₂-CH(CH₂-CH₃)- ou -CH₂-CHOR¹⁴-CH₂-,
R¹¹ représente un atome d'hydrogène, un groupe amino- alkyle (C₁-C₆), alkyle en C₁-C₂₄, R¹³-C(=O)- ou R¹³-NH-C(=O)-,
R¹² représente un groupe alkylène en C₁-C₂₀, dont la chaîne carbonée peut être interrompue par 1 à 10 atomes d'oxygène non contigus,
R¹³ représente un groupe alkyle en C₁-C₂₄,
R¹⁴ représente un atome d'hydrogène, un groupe alkyle en C₁-C₂₄ ou R¹³-CO-,
A représente -C(=O)-O-, -C(=O)-B-C(=O)-O- ou -C(=O)-NH-B-NH-C(=O)-O-,
B représente un groupe -(CH₂)ₜ-, cycloalkylène éventuellement substitué, hétérocycloalkylène éventuellement substitué ou arylène éventuellement substitué,
n représente 1 ou, lorsque R⁷ représente un reste de polyalcool, 1 à 8,
s représente 0 à 500, de préférence 0 à 100,
u représentent chacun indépendamment 1 à 5000, de préférence 1 à 1000,
v représentent chacun indépendamment 0 à 5000, de préférence 1 à 1000,
w représentent chacun indépendamment 0 à 5000, de préférence 1 à 1000.

8. Procédé selon la revendication 7, **caractérisé en ce qu'**on utilise comme composant f) contenant un polyéther des homo- et copolymérisats d'oxyde d'éthylène et/ou d'oxyde de propylène ayant une masse moléculaire moyenne en poids M_{w} dans la plage de 1 000 à 100 000, de préférence de 5 000 à 50 000 g/mole, qui peuvent être coiffés d'un côté ou des deux côtés par des groupes terminaux.

9. Procédé selon l'une quelconque des revendications 1 à 8, **caractérisé en ce que** le composant f) contenant un polyéther est présent lors de la polymérisation en une quantité dans la plage de 5 à 70, de préférence de 10 à 50 % en poids, par rapport à la quantité totale des composants a) à e).

10. Procédé selon l'une quelconque des revendications 1 à 9, **caractérisé en ce que** l'agent de réticulation c) est choisi dans le groupe constitué par l'éther triallylique de pentaérythritol, le méthylènebisacrylamide, la N,N'-divinyléthylène-urée, la triallylamine et des sels de triallylmonoalkylammonium, des esters d'acide acrylique avec l'éthylèneglycol, le butanediol, le triméthylolpropane ou le glycérol ou des esters d'acide acrylique avec un glycol, butanediol, triméthylolpropane ou glycérol ayant réagi avec l'oxyde d'éthylène et/ou l'épichlorhydrine.

11. Procédé selon l'une quelconque des revendications 1 à 10, **caractérisé en ce que** le mélange à polymériser contient de 0,05 à 2 % en poids de l'agent de réticulation c), par rapport à la quantité totale des monomètres a) à e).

12. Procédé selon l'une quelconque des revendications 1 à 11, **caractérisé en ce que** le composé d) est choisi dans le groupe constitué par des (méth)acrylates d'alkyle en C₁₂-C₃₀ et des éthers alkyl(C₁₂-C₃₀) vinyliques .

13. Procédé selon l'une quelconque des revendications 1 à 12, **caractérisé en ce que** le composé d) est choisi dans le groupe constitué par le méthacrylate de méthyle, l'acrylate de butyle, le méthacrylamide et des mélanges de ceux-ci.

14. Procédé selon l'une quelconque des revendications 1 à 13, **caractérisé en ce qu'**après ou pendant la polymérisation le polymère est partiellement ou totalement protoné ou rendu quaternaire dans la plage de 20 à 99 % en moles, lorsque dans la polymérisation on utilise comme composant a) un monomère non ou seulement partiellement protoné ou rendu quaternaire.

15. Procédé selon l'une quelconque des revendications 1 à 14, **caractérisé en ce qu'**on choisit les amorceurs A et B de manière que les températures de décomposition respectives soient d'au moins 70 °C.

16. Procédé selon la revendication 15, **caractérisé en ce que** les températures de décomposition respectives diffèrent d'au moins 10 °C.

17. Procédé selon l'une quelconque des revendications 1 à 16, **caractérisé en ce que** la quaternisation du polymère est effectuée dans la solution de polymérisation, pratiquement immédiatement après la polymérisation.

18. Polymère pouvant être obtenu conformément à un procédé selon l'une quelconque des revendications 1 à

19. Utilisation d'au moins un polymère selon la revendication 18, pour la modification des propriétés rhéologiques de compositions aqueuses, alcooliques ou aqueuses-alcoolique.

20. Produit cosmétique ou pharmaceutique contenant au moins un polymère selon la revendication 18.

21. Produit selon la revendication 20, contenant au moins un polymère selon la revendication 19, en une quantité dans la plage de 0,01 à 20 % en poids.

22. Produit selon la revendication 20 ou 21, qui en outre contient au moins un polymère, en particulier un polymère qui contient du N-vinyl-lactame incorporé par polymérisation.

23. Produit selon l'une quelconque des revendications 20 à 22, le produit consistant en un gel, en particulier en un gel capillaire.

24. Produit selon la revendication 20 ou 21, le produit consistant en un gel ou une crème pour applications pharmaceutiques topiques.

25. Produit selon l'une quelconque des revendications 20 à 24, essentiellement à base aqueuse.

26. Produit selon l'une quelconque des revendications 20 à 24, essentiellement à base alcoolique.
